(19) Europäisches Patentamt
European Patent Office
Office européen des brevets

(11) Numéro de publication : **0 068 968**
**B1**

(12) # FASCICULE DE BREVET EUROPÉEN

(45) Date de publication du fascicule du brevet : **18.09.85**

(21) Numéro de dépôt : **82401084.7**

(22) Date de dépôt : **15.06.82**

(51) Int. Cl.⁴ : **C 07 C143/78**, C 07 C143/80, C 07 D213/71, C 07 D333/34, C 07 D295/18, A 61 K 31/195, A 61 K 31/535, A 61 K 31/20, A 61 K 31/22, A 61 K 31/23, A 61 K 31/63

(54) **Nouveaux médicaments contenant à titre de substance active des composés du type arylbenzènesulfonamide et leurs procédés de préparation.**

(30) Priorité : **16.06.81 FR 8111858**
**16.06.81 FR 8111859**

(43) Date de publication de la demande :
**05.01.83 Bulletin 83/01**

(45) Mention de la délivrance du brevet :
**18.09.85 Bulletin 85/38**

(84) Etats contractants désignés :
**AT BE CH DE GB IT LI LU NL SE**

(56) Documents cités :
**BE-A- 566 666**
**DE-B- 1 101 408**
**FR-A- 1 551 116**
**FR-M- 7 477**
**GB-A- 562 349**
**NL-C- 47 894**
**US-A- 2 142 847**
**US-A- 2 592 105**
**CHEMICAL ABSTRACTS, vol.55, no.3, 6 février 1961, colonne 2573c-d, Columbus Ohio (US)**
**CHEMICAL ABSTRACTS, vol.64, no.7, 28 mars 1966, colonne 9710-d, Columbus, Ohio (US), A. SHOEB et al.: "Possible oral hypoglycemic agents. IV. Synthesis of 1-arylsulfonyl-3-substituted-2-imidazolidinones and 1,2,3-trisubstituted ureas »**

(73) Titulaire : **CHOAY S.A.**
**48, Avenue Théophile-Gautier**
**F-75782 Paris Cedex 16 (FR)**

(72) Inventeur : **Choay, Patrick**
**7, Cours Jasmin**
**F-75016 Paris (FR)**
Inventeur : **Roger, Pierre**
**6, rue Paul Valéry**
**F-78423 Montigny-les-Bretonneux (FR)**
Inventeur : **Olliero, Dominique**
**Résidence des Facultés Avenue de la Justice**
**F-34000 Montpellier (FR)**

(74) Mandataire : **Gutmann, Ernest et al**
**Cabinet Plasseraud 84, rue d'Amsterdam**
**F-75009 Paris (FR)**

**EP 0 068 968 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

CHEMICAL ABSTRACTS, vol.65, no.12, 5 décembre 1966, colonne 19175 th, Columbus, Ohio (US) R.S. BAICHWAL et al.: « Oral hypoglycemic agents »
CHEMISCHES ZENTRALBLATT, no.36, partie II, 4 septembre 1963, Weinheim (DE), Z. BUDESINSKY et al.: « Synthetische Antidiabetica. 2. Mitt. N-Tosylaminosäuren und deren N-Alkylderivate », pages 15761-2
CHEMICAL ABSTRACTS, vol.55, no.23, 13 novembre 1961, colonne 23446a, Columbus, Ohio (US)
JOURNAL OF MEDICINAL CHEMISTRY, vol.15, no.12, 1972, Washington (US), R.F. BORNE et al.: "Antiinflammatory activity of para-substituted N-benzenesulfonyl derivatives of amino acids", pages 1325-1326
CHEMICAL ABSTRACTS, vol.58, no.7, 1er avril 1963, colonnes 6829, 6830, Columbus, Ohio (US), D.F. HAYMAN et al.: "Hypoglycemic agents III"
CHEMICAL ABSTRACTS, vol.59, no.10, 11 novembre 1963, colonne 11932a,b, Columbus, Ohio (US), E. SEIFTER et al.: "Antimicrobial action of some amino acids and their derivatives I. Chemistry"

**Description**

L'invention est relative à de nouveaux médicaments présentant des propriétés normolipémiantes et qui contiennent à titre de substance active des composés présentant une structure de base du type arylsulfonamide ou un sel physiologiquement acceptable de ces composés.

On appellera ci-après « médicament » toute composition pharmaceutique contenant, en association avec un véhicule pharmaceutiquement acceptable, l'un au moins des composés chimiques tels qu'ils sont définis ci-après.

L'invention vise également le procédé de préparation des susdits médicaments ainsi que leurs sels.

Des arylsulfonamides ont déjà été décrits dans :

— le brevet FR-1 551 116 qui concerne un procédé de préparation des composés de formule suivante :

$$A - SO_2\overset{R}{\overset{|}{N}}CH_2\overset{R'}{\overset{|}{C}}HCONH_2$$

dans laquelle

A peut représenter un radical aryle, tel que benzène, naphtalène, anthraquinone, diphényle ou indène, un radical hétérocyclique du type hétéroaromatique, tel que pyridine, quinoléine, furane, benzofurane, thiophène, etc. ; A pouvant être substitué par un ou plusieurs groupes tels que amino, alkyle inférieur, alcoxy inférieur, halogéno, hydroxy, nitro, alkylsulfonyl inférieur, etc. ;

R est l'hydrogène, un résidu aliphatique ou le reste $CH_2CHR'CONH_2$ ;

R' est un atome d'hydrogène ou un reste méthyle ; dans ces composés, la chaîne alcoylée en alpha de $SO_2N$ est une chaîne courte ayant deux atomes de carbone ; il est indiqué que les produits obtenus par le procédé selon l'invention ont des emplois étendus dans de nombreux domaines d'application, sans aucune précision, et sans aucun exemple, on mentionne que ces produits peuvent servir, par exemple, comme intermédiaires dans la préparation des colorants, comme azurants optiques, produits pharmaceutiques, insecticides, fongicides, etc.

— le brevet GB-562 349 qui concerne des composés de formule :

$$H_2N—C_6H_4—SO_2—NH—X—CO—NH_2$$

dans laquelle X peut représenter un radical alkyle sans que le nombre d'atomes de carbone soit précisé ; aucun exemple de composés dans lesquels X représente un radical alcoyle n'est mentionné ; sans aucune précision, ces composés sont présentés comme ayant des propriétés thérapeutiques ;

— le brevet DE-904 895 qui concerne des composés de formule :

$$R—SO_2—NH—X$$

dans laquelle R peut être un noyau hétérocyclique comportant un amino en para, et X peut être le radical d'un acide carboxylique ; il n'y a aucune précision sur le nombre d'atomes de carbone du radical X, et le seul exemple de composé dont la synthèse est indiquée, est relatif à un composé dont le radical X présente deux atomes de carbone ; sans aucune précision, ces composés sont présentés comme ayant des propriétés thérapeutiques ;

— le brevet DE-1 101 408 qui concerne des composés de formule :

$$R—SO_2—NH—CH_2—CO—Y—Z$$

dans laquelle R représente un reste phényle substitué ou non substitué ;

Y représente un atome d'hydrogène ou un groupe NH,

Z représente une chaîne alcoyle linéaire ou ramifiée de 3 à 6 atomes de carbone ; dans ces composés, la chaîne alcoylée en alpha du groupe $—SO_2NH$ ne comprend qu'un seul atome de carbone ; ces composés sont présentés, sans aucune autre indication, comme ayant des propriétés antidiabétiques ;

— le brevet NL-47 894 qui concerne des composés de formule :

$$NH_2—C_6H_4—SO_2—NH—CH_2—COOH$$

dans ces composés, le radical en alpha du groupe $—SO_2NH$ ne présente qu'un seul atome de carbone ; sans aucune précision, ces composés sont présentés comme ayant des propriétés antibactériennes ;

— le brevet US-2 592 105 qui concerne des composés de formule :

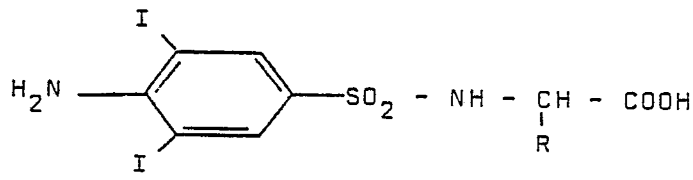

dans laquelle R est un groupe hydrocarboné ayant de 2 à 7 atomes de carbone ; ces composés sont présentés comme étant des agents de contraste dans les examens radiologiques ; dans ces composés, la chaîne latérale alcoylée en alpha du groupe —$SO_2NH$ ne présente qu'un seul atome de carbone ;

— le brevet US-2 142 847 qui concerne des composés de formule :

$$R—SO_2—NH—R'—COOX$$

dans laquelle R est un radical amino aryle,

R' est un radical aliphatique, sans précision du nombre d'atomes de carbone,

et X est un atome d'hydrogène ou une base ;

d'après les exemples, le radical en alpha du $SO_2NH$ représente soit une chaîne alcoylée ayant un atome de carbone, soit un groupe $CHCH_2COOX$, dans ce dernier cas, les composés visés par ce document comprennent deux fonctions COOX ; sans aucune autre précision, ces composés sont présentés comme ayant des propriétés antibactériennes ;

— les Chemical Abstracts, *55*, n° 23 (1961) qui concernent les deux composés suivants : le 3-chloro-4-méthylphényl-sulfonyl-N-butylglycine et le 4-chloro-phénylsulfonyl-N-isobutylglycine ; dans ces composés, la chaîne alcoylés en alpha de $SO_2N$, présente un seul atome de carbone ; ces composés sont présentés comme ayant des propriétés antidiabétiques ;

— les Chemical Abstracts, *55*, n° 3 (1961) qui concernent le N-(p-toluènesulfonyl)-N-butylglycinamide ; dans ces composés, la chaîne en alpha de $SO_2NH$ ne comporte qu'un seul atome de carbone ; ces composés sont présentés comme ayant une activité antidiabétique ;

— les Chemical Abstracts, *58*, n° 7 (1963) qui concernent des composés dérivés du tolbutamide ; dans ces composés, la chaîne alcoylée en alpha de $SO_2N$ ne présente pas plus de deux atomes de carbone ; ces composés sont présentés comme ayant des propriétés pharmacologiques analogues ou tolbutamide ;

— les Chemical Abstracts, *59*, n° 10 (1963) qui concernent des dérivés de sufonamidoaminoacides ; dans ces composés, la chaîne alcoylée en alpha du $SO_2N$ présente un ou deux atomes de carbone ; ces composés sont présentés comme ayant des propriétés antibactériennes ;

— le brevet BE-566 666 qui concerne des composés de formule :

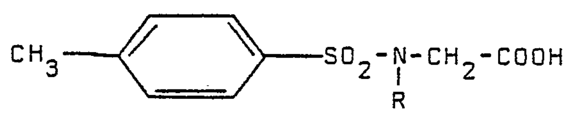

dans laquelle R représente un groupe alcoyle ayant de 2 à 14 atomes de carbone ; dans ces composés, la chaîne alcoylée en alpha du

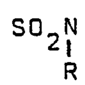

ne présente qu'un seul atome de carbone ; ces composés sont présentés comme ayant une activité hypoglycémiante.

Il y a lieu de remarquer que toutes les indications pharmacologiques ne sont supportées par aucun test pharmacologique ; par conséquent l'enseignement de ces documents antérieurs ne permet pas de déduire les propriétés pharmacologiques et thérapeutiques des arylsulfonamides actifs.

Jusqu'à présent, les seuls arylsulfonamides présentés comme ayant des propriétés pharmacologiques sont décrits dans les documents suivants :

— le Journal of Medicinal Chemistry, 1972, *15*, n° 12 qui concerne des N-benzènesulfonyl de glycine, de sarcosine, de D,L-alanine, de beta-alanine, de L-histidine, de D,L-tryptophane, de L-proline, de L-asparagine et de D,L-phénylalanine, c'est-à-dire des dérivés dans lesquels la chaîne alcoylée en alpha de $SO_2NH$ présente au plus deux atomes de carbone ; le noyau de ces composés est substitué, d'après les exemples, par les atomes d'halogène, ou par les radicaux $OCH_3$ ou $NHCOCH_3$ ; ces composés sont présentés comme ayant des propriétés anti-inflammatoires, mais seuls des composés, dont la chaîne alcoylée en alpha du $SO_2$ présente un atome de carbone, ont été pharmacologiquement testés :

— le brevet de médicament n° 7 477 M qui concerne des composés de formule :

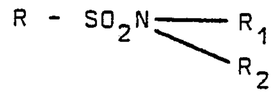

dans laquelle R peut être un radical aminophényle, ortho, méta ou paranitrophényle, phényle, phénylalcoyle, etc.

$R_1$ peut être l'hydrogène, un radical alcoyle, etc.,

$R_2$ est un radical du type $A—SO_3B$ ou $A—CO_2B$, dans lequel B désigne une base et A un radical à chaîne droite ou ramifiée, ou cyclique, substituée ou non ; il n'y a aucune précision quant au nombre d'atomes de carbone du radical A, mais les seuls exemples ne concernent que des composés dans lesquels A présente deux atomes de carbone ; ces composés sont présentés comme ayant des propriétés antidiabétiques, antibactériennes, hépatoprotectrices, diurétiques, mais il s'avère que les résultats d'essais pharmacologiques ne sont donnés que pour les composés dans lesquels $R_2$ est un radical propane sulfonate ;

— L'Indian J. Chem., *3* (1965) qui concerne notamment des composés de formule :

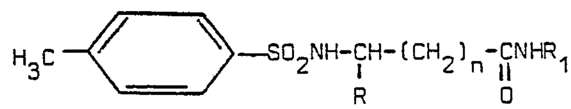

dans lesquels, d'après les exemples, la valeur de n est 0 ou 1 ; bien que ces composés sont présentés avec d'autres composés pour lesquels est indiquée une action hypoglycémiante, il est expressément indiqué qu'ils ne possèdent aucun effet sur le taux de sucre dans le sang ;

— The Indian Journal of Pharmacy, *28*. n° 8 qui concerne des composés de formule :

$$R—C_6H_4—SO_2NH—CH_2—COOH$$

ou de formule :

$$CH_3 \text{ ou } Cl—C_6H_4—SO_2N(H \text{ ou } Br)—CH_2—CONH—R$$

dans ces composés, la chaîne alcoylée en alpha du groupe $—SO_2NH$ ne présente qu'un seul atome de carbone ; mais il est indiqué qu'aucun effet hypoglycémiant efficace et comparable au tolbutamide n'est obtenu avec ces composés ; mais il est indiqué qu'aucun effet hypoglycémiant efficace et comparable au tolbutamide n'est obtenu avec ces composés ;

— le Chemisches Zentralblatt, *2*, n° 36 (1963) qui concerne notamment des composés de formule :

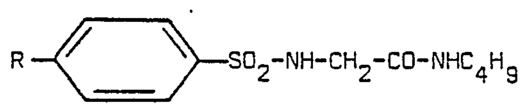

dans laquelle R représente $NH_2$ ou $CH_3$ ; dans ces composés, la chaîne latérale en alpha de $SO_2N$ n'a qu'un seul atome de carbone ; ces composés sont présentés comme ayant été testés pour leurs propriétés antidiabétiques.

L'enseignement de ces documents antérieurs n'indique rien quant à une éventuelle activité pharmacologique des arylsulfonamides dans lesquels la chaîne alcoylée en alpha de $SO_2N$ présente plus de deux atomes de carbone.

Les médicaments selon l'invention sont caractérisés en ce qu'ils répondent à la formule générale :

$$Ar - SO_2 - \underset{R_5}{N} - (CH_2)_n - \underset{R_6}{CH} - (CH_2)_m - \underset{O}{\overset{}{C}} - R_4 \quad (I)$$

Dans cette formule, Ar représente l'un des cycles suivants :

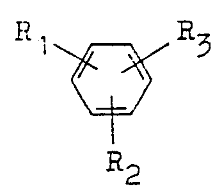

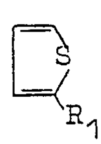

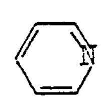

$R_1$, $R_2$, $R_3$ sont identiques ou différents et représentent un atome d'hydrogène, un atome d'halogène, le radical $NO_2$, le radical $NH_2$, le radical $CF_3$, un groupe alkyle ayant de 1 à 6 et de préférence de 1 à 4 atomes de carbone, un groupe alcoxy ayant de 1 à 6 et de préférence de 1 à 4 atomes de carbone, un groupe acide, un groupe ester ayant de 2 à 7 et de préférence de 2 à 5 atomes de carbone ;

la somme n + m + 1 est comprise de 3 à 11 et de préférence égale à 3, 5 ou 10, cette somme pouvant également être égale à 1 ou 2, lorsque Ar contient un noyau benzénique dont l'une au moins des substituants $R_1$, $R_2$ et $R_3$ est un groupe $CF_3$ ;

$R_5$ et $R_6$ représentent, indépendamment l'un de l'autre, un atome d'hydrogène, un radical alcoyle ayant de 1 à 6 atomes de carbone, de préférence de 1 à 4 atomes de carbone, un radical aralcoyle ayant de 7 à 9 atomes de carbone ;

$R_4$ représente :

— soit un groupe hydroxy,

— soit le radical $OR_7$ dans lequel $R_7$ est un groupe alkyle ayant de 1 à 6 atomes de carbone, et de préférence de 1 à 4 atomes de carbone :

— soit le radical

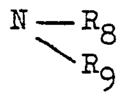

dans lequel $R_8$ et $R_9$, identiques ou différents, représentent un atome d'hydrogène, un groupe alkyle ayant de 1 à 6 et de préférence de 1 à 4 atomes de carbone, ou conjointement forment avec l'azote un hétérocycle azoté à 5 ou 6 chaînons, notamment un groupe pipéridino, morpholino, pyrrolidino, pyrrole ou pyrroline.

Un groupe préféré de médicaments conformes à l'invention est constitué par ceux répondant à la formule générale (I) dans laquelle $R_6$ représente un atome d'hydrogène. Ces médicaments répondent à la formule générale suivante.

$$Ar - SO_2 - \underset{\underset{R_5}{|}}{N} - (CH_2)_{n+m+1} - \underset{\underset{O}{||}}{C} - R_4 \qquad (II)$$

dans laquelle

n + m + 1 est compris de 3 à 11, étant de préférence égal à 3, 5 ou 10 ;

$R_5$ représente un radical alcoyle ayant de 1 à 6 atomes de carbone, de préférence de 1 à 4 atomes de carbone, un radical aralcoyle ayant de 7 à 9 atomes de carbone ;

$R_4$ représente :

— le groupe hydroxy ;

— le radical $OR_7$ dans lequel $R_7$ est un groupe alkyle ayant de 1 à 6 atomes de carbone, de préférence de 1 à 4 atomes de carbone ;

— le radical

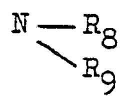

dans lequel $R_8$ et $R_9$, identiques, ou différents, représentent un atome d'hydrogène, un groupe alkyle ayant de 1 à 6 et de préférence de 1 à 4 atomes de carbone, ou conjointement forment avec l'azote un hétérocycle azoté à 5 ou 6 chaînons, notamment un groupe pipéridino, morpholino, pyrrolidino, pyrrole ou pyrroline.

Dans une forme préférée, les médicaments selon l'invention répondent à la formule (I) dans laquelle ;

Ar représente un noyau benzénique substitué de formule :

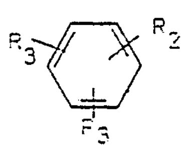

$R_1$, $R_2$ et $R_3$ ayant les significations ci-dessus indiquées.

Dans la suite, on désignera par $A_1$ le groupe formé par l'ensemble des médicaments de formule :

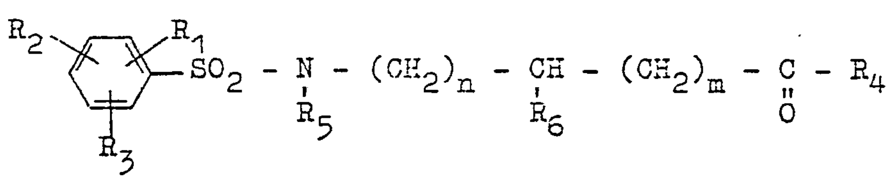

(III)

dans laquelle

$R_1$, $R_2$, $R_3$, $R_4$, $R_5$ et $R_6$ ont les significations ci-dessus indiquées ;

n + m + 1 est compris de 3 à 11, étant de préférence égal à 3, 5 ou 10.

A l'intérieur de ce groupe $A_1$, une classe de composés préférés est constituée par ceux dans lesquels $R_6$ représente un hydrogène et répondent à la formule (IV) suivante :

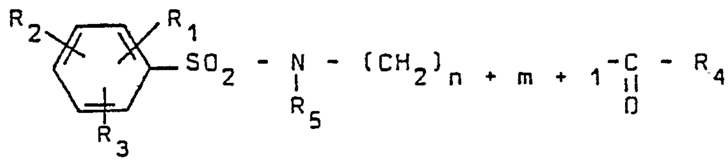

(IV)

Dans une autre forme préférée, les médicaments selon l'invention répondent à la formule (III) dans laquelle $R_1$,$R_2$ et $R_3$ ont la signification ci-dessus indiquée et $R_5$ représente l'hydrogène.

Ces médicaments répondent à la formule (V) suivante :

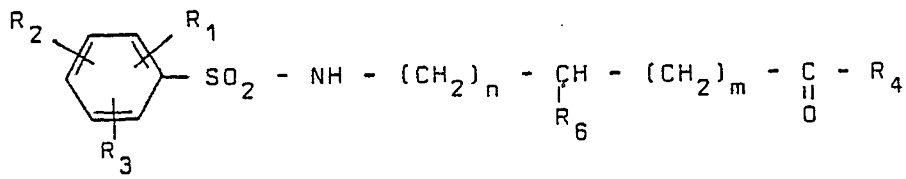

(V)

Dans la suite du texte, on désignera par $A_2$ le groupe formé par l'ensemble des médicaments de formule (V).

A l'intérieur des groupes $A_1$ et $A_2$, une classe de composés préférés est constituée par ceux de formule (VI) suivante :

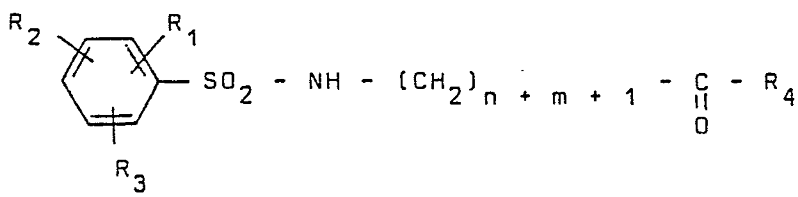

(VI)

Une famille préférée de médicaments selon l'invention est constituée par les médicaments de formule (V) dans laquelle $R_1$, $R_2$ et $R_3$ représentent simultanément un atome d'hydrogène, ou sont simultanément différents de l'hydrogène et son choisis dans le groupe constitué par un atome d'halogène, le radical $NO_2$, le radical $NH_2$, le radical $CF_3$, un groupe alkyle ayant de 1 à 6 et de préférence de 1 à 4 atomes de carbone, un groupe alcoxy ayant de 1 à 6 et de préférence de 1 à 4 atomes de carbone, un groupe acide, un groupe ester ayant de 2 à 7 et de préférence de 2 à 5 atomes de carbone.

Les médicaments qui répondent aux formules (V) et (VI) dans laquelle $R_1$, $R_2$, $R_3$ sont tous les trois différents de l'hydrogène forment un groupe qui sera par la suite désigné par $B_1$.

Parmi ce groupe $B_1$ ou une classe avantageuse de composés est constituée par ceux dans lesquels $R_6$ représente l'hydyrogène.

Les médicaments qui répondent à la formule (V), dans laquelle tous les substituants du noyau aromatique sont des hydrogènes, c'est-à-dire la formule (VII) suivante :

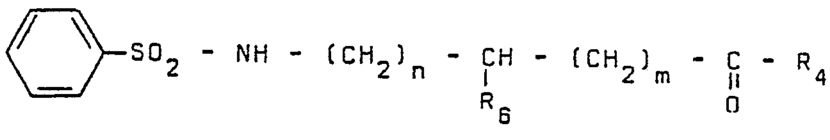

(VII)

forment un groupe qui par la suite sera désigné par $B_2$.

Parmi ce groupe $B_2$, une classe avantageuse de composés est constituée par ceux dans lesquels $R_6$ représente l'hydrogène. Ces composés répondent à la formule (VIII) suivante :

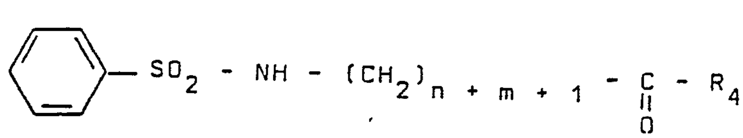

(VIII)

Dans le groupe $B_1$ ci-dessus défini, une classe préférée de médicaments est constituée par les médicaments de formule (V ou VI) dans laquelle le noyau benzénique est trisubstitué par les radicaux choisis dans le groupe comprenant $NO_2$, $NH_2$, $OCH_3$, $CH_3$, $CF_3$, un atome d'halogène et notamment le chlore.

Ce groupe de médicaments sera par la suite désigné par $E_1$.

Une famille préférée qui répond à la formule (V) dans laquelle $R_1$, $R_2$ et $R_3$ sont tous les trois différents de l'hydrogène, est constituée par celle dans laquelle $R_1$, $R_2$ et $R_3$ représentent simultanément un radical méthoxy.

Ce groupe sera dans la suite désigné par $E_2$.

Parmi les médicaments du groupe $A_2$, une classe de médicaments préférés est constituée par les médicaments répondant à la formule (V) dans laquelle $R_1$ représente un atome d'hydrogène, $R_2$ et $R_3$ représentent un atome d'halogène, le radical $NO_2$, le radical $NH_2$, le radical $CF_3$, un groupe alkyle ayant de 1 à 6 et de préférence de 1 à 4 atomes de carbone, un groupe alcoxy ayant de 1 à 6 et de préférence de 1 à 4 atomes de carbone, un groupe acide, un groupe ester ayant de 2 à 7 et de préférence de 2 à 5 atomes de carbone.

Ces médicaments dont le noyau aromatique est disubstitué et qui répondent à la formule (IX) suivante :

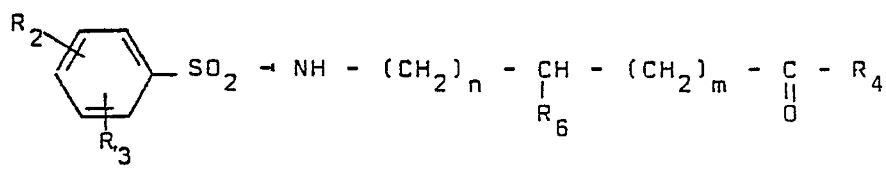

(IX)

dans laquelle $R_2$, $R_3$, $R_4$ et $R_6$ ont la signification ci-dessus indiquée, forment un groupe qui sera par la suite désigné par $G_1$.

Parmi le groupe $G_1$ ci-dessus désigné, une classe préférée de médicaments est constituée par ceux de formule :

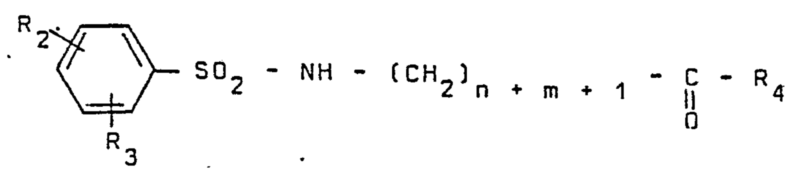

(X)

dans laquelle

$n + m + 1$ est compris de 3 à 11, de préférence de 3 à 6 ;

$R_2$, $R_3$ et $R_4$ ayant la signifcation ci-dessus indiquée.

Parmi le groupe $G_1$ ci-dessus défini, une classe préférée de médicaments est constituée par les médicaments pour lesquels le noyau benzénique est di-substitué par les radicaux choisis dans le groupe constitué par $NO_2$, $CH_3$, $NH_2$, $OCH_3$, $CF_3$, par un halogène notamment le chlore.

Ce groupe de médicaments sera par la suite désigné par $G_2$.

Parmi les médicaments du groupe $A_2$, une famille de médicaments préférés est constituée par les médicaments répondant à la formule (V) dans laquelle $R_1$ et $R_2$ représentent simultanément un atome d'hydrogène, $R_3$ représentant un atome d'halogène, le radical $NO_2$, le radical $NH_2$, le radical $CF_3$, un groupe alkyle ayant de 1 à 6 et de préférence de 1 à 4 atomes de carbone, un groupe alcoxy ayant de 1 à 6 et de préférence de 1 à 4 atomes de carbone, un groupe acide, un groupe ester ayant de 2 à 7 et de préférence de 2 à 5 atomes de carbone.

Ces médicaments dont le noyau aromatique est monosubstitué et qui répondent à la formule (XI) suivante :

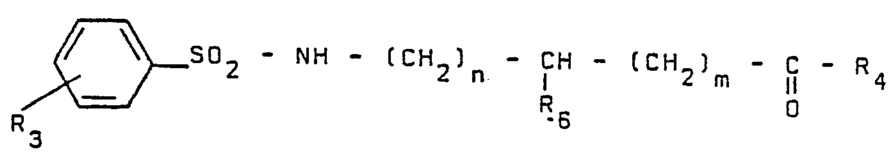

(XI)

$R_3$ ayant la signification ci-dessus indiquée, forment un groupe qui sera par la suite désigné par $H_1$.

Parmi le groupe $H_1$ ci-dessus défini, une classe préférée de composés conformes à l'invention est constituée par ceux dans lesquels $R_6$ représente un atome d'hydrogène. Ces composés répondent à la formule (XII) suivante :

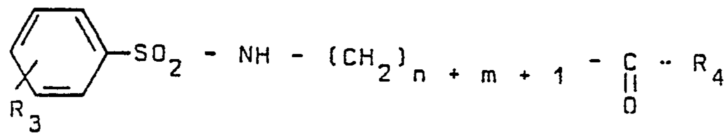 (XII)

Parmi le groupe $H_1$ ci-dessus défini, une classe préférée de médicaments est constituée par les médicaments pour lesquels le noyau benzénique est monosubstitué par le radical choisi dans le groupe constitué par $NO_2$, $NH_2$, $CH_3$, $OCH_3$, $CF_3$, un atome d'halogène, notamment le chlore.

Ce groupe de médicaments sera par la suite désigné par $H_2$.

Une autre famille de médicaments préférés selon l'invention est constituée par les médicaments du groupe $A_2$ dans lequel le noyau benzénique comprend un substituant en para choisi dans le groupe constitué par $NO_2$, $NH_2$, $CH_3$ ou $OCH_3$.

Ce groupe sera désigné par la suite par K.

Selon un autre mode de réalisation préféré de l'invention, une autre famille de médicaments préférés est constituée par les médicaments du groupe $A_2$ ci-dessus défini dans lequel le noyau benzénique comprend un substituant en méta choisi dans le groupe constitué par $NO_2$, $NH_2$, $CF_3$, un atome d'halogène notamment le chlore.

Ce groupe de médicaments sera désigné par la suite par L.

Parmi le groupe $A_2$ ci-dessus défini, une autre classe préférée de médicaments est constituée par ceux pour lesquels le noyau benzénique est substitué en ortho par le radical $OCH_3$.

Ce groupe de médicaments sera par la suite désigné par M.

Dans les groupes $B_1$ et $E_1$ ci-dessus définis, une classe préférée de médicaments selon l'invention est constituée par les médicaments pour lesquels le noyau aromatique est trisubstitué :

— en para par l'un des radicaux choisis dans le groupe constitué par $NO_2$ ou $NH_2$ ;

— en méta (celle des positions méta non comprise entre les positions ortho et para substituées) par un halogène, notamment le chlore ;

— en ortho par le radical $OCH_3$.

Ce groupe de médicaments sera par la suite désigné par N.

Une autre classe de médicaments préférés selon l'invention est constiuée par les médicaments des groupes $G_1$ et $G_2$ pour lesquels le noyau benzénique est disubstitué :

— en para par le radical $NH_2$, $NO_2$, un atome d'halogène, notamment le chlore ;

et

— soit en méta par le radical $NH_2$, $CO_2$, un atome d'halogène, notamment le chlore ;

— soit en ortho par le radical $OCH_3$.

Ce groupe sera par la suite désigné par S.

La classe de médicaments appartenant au groupe S, disubstitués en para et en méta sera par la suite désignée par le groupe $S_1$.

La classe de médicaments appartenant au groupe S, disubstitués en para et en ortho sera par la suite désignée par $S_2$.

Une autre classe de médicaments préférés selon l'invention est constituée par les médicaments des groupes $H_1$ et $H_2$ pour lesquels le noyau benzénique est monosubstitué soit en para par le radical $NO_2$, $NH_2$, $CH_3$, $OCH_3$, un atome d'halogène, notamment le chlore ; soit en méta par le radical $NO_2$ ou par le radical $CF_3$.

Ce groupe de médicaments sera par la suite désigné par T.

Une autre classe de médicaments préférés conformes à l'invention est constituée par les médicaments de formule (III) :

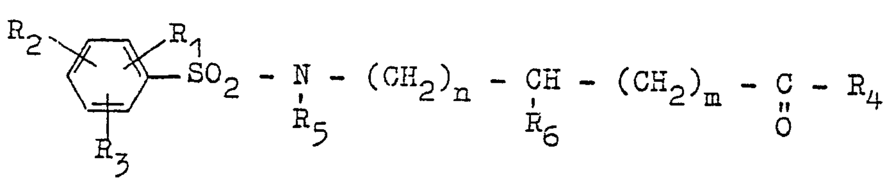 (III)

dans laquelle l'un au moins des substituants $R_1$, $R_2$, $R_3$ représente un radical $CF_3$, $R_4$ représente :

— le groupe hydroxy ;

— le radical $OR_7$ dans lequel $R_7$ est un groupe alkyle ayant de 1 à 6 atomes de carbone, de préférence de 1 à 4 atomes de carbone ;

— le radical

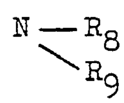

dans lequel $R_8$ et $R_9$, identiques ou différents, représentent un atome d'hydrogène, un groupe alkyle ayant de 1 à 6 et de préférence de 1 à 4 atomes de carbone, ou conjointement forment avec l'azote un hétérocycle azoté à 5 ou 6 chaînons, notamment un groupe pipéridino, morpholino, pyrrolidino, pyrrole ou pyrroline ;

$n + m + 1$ est compris de 1 à 11, étant de préférence égal à 3,5 ou 10.

Cette classe de médicaments sera par la suite désignée par $D_1$.

Parmi cette classe $D_1$, une famille préférée de médicaments conformes à l'invention est constituée par ceux dans lesquels $R_6$ représente l'hydrogène.

Une autre classe de médicaments préférés conformes à l'invention est constituée par les médicaments de formule (V) :

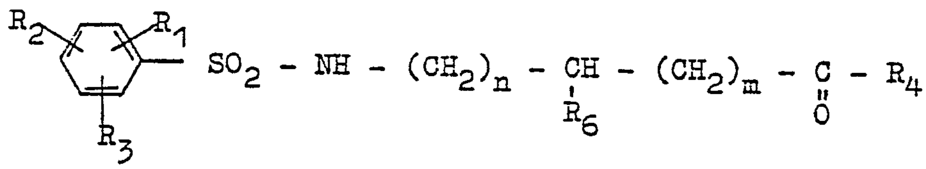

(V)

dans laquelle l'un au moins des substituants $R_1$, $R_2$, $R_3$ représente $CF_3$,

$R_4$ représente :

— le groupe hydroxy ;

— le radical $OR_7$ dans lequel $R_7$ est un groupe alkyle ayant de 1 à 6 atomes de carbone, et de préférence de 1 à 4 atomes de carbone ;

— le radical

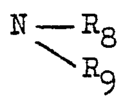

dans lequel $R_8$ et $R_9$, identiques ou différents, représentent un atome d'hydrogène, un groupe alkyle ayant de 1 à 6 atomes de carbone, de préférence de 1 à 4 atomes de carbone ; ou conjointement forment avec l'azote un hétérocycle azoté à 5 ou 6 chaînons, notamment un groupe pipéridino, morpholino, pyrrolidino, pyrrole ou pyrroline ;

$n + m + 1$ est compris de 1 à 11, étant de préférence égal à 3, 5 ou 10.

Ce groupe de médicaments sera par la suite désigné par $D_2$.

Parmi cette classe $D_2$, une famille préférée de médicaments conformes à l'invention est constituée par ceux dans lesquels $R_6$ représente un atome d'hydrogène.

Une autre classe de médicaments préférés conformes à l'invention est constituée par les médicaments de formule (IX) :

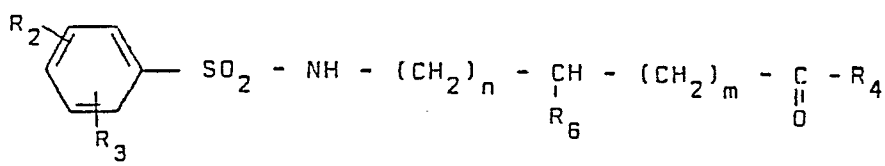

(IX)

dans laquelle l'un au moins des substituants $R_2$ ou $R_3$ représente le radical $CF_3$ ;

$R_4$ représente :

— le groupe hydroxy,

— le radical $OR_7$ dans lequel $R_7$ est un groupe alkyle ayant de 1 à 6 atomes de carbone, de préférence de 1 à 4 atomes de carbone ;

— le radical

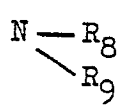

dans lequel $R_8$ et $R_9$ sont identiques ou différents et représentent un atome d'hydrogène, un groupe alkyle ayant de 1 à 6 atomes de carbone, de préférence de à 4 atomes de carbone ; ou conjointement forment avec l'azote un hétérocycle azoté à 5 ou 6 chaînons, notamment un groupe pipéridino, morpholino, pyrrolidino, pyrrole ou pyrroline ;

$n + m + 1$ est compris de 1 à 11, étant de préférence égal à 3, 5 ou 10.

Cette classe de médicaments sera par la suite désignée par $D_3$.

Parmi cette classe $D_3$, une famille préférée de médicaments conformes à l'invention, est constituée par ceux dans lesquels $R_6$ représente un atome d'hydrogène.

Une autre classe de médicaments préférés conformes à l'invention est constituée par les médicaments de formule (XIII) :

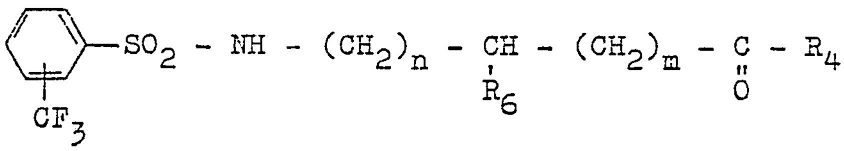
(XIII)

dans laquelle $R_4$ représente :

— le groupe hydroxy,

— le radical $OR_7$ dans lequel $R_7$ est un groupe alkyle ayant de 1 à 6 atomes de carbone, de préférence de 1 à 4 atomes de carbone ;

— le radical

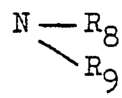

dans lequel $R_8$ et $R_9$ sont identiques ou différents et représentent un atome d'hydrogène, un groupe alkyle ayant de 1 à 6 atomes de carbone et de préférence de 1 à 4 atomes de carbone, ou conjointement forment avec l'azote un hétérocycle azoté à 5 ou 6 chaînons, notamment un groupe pipéridino, morpholino, pyrrolidino, pyrrole ou pyrroline ;
et $n + m + 1$ est compris de 1 à 11, étant de préférence égal à 3, 5 ou 10.

Cette classe de médicaments sera par la suite désignée par $D_4$.

Parmi cette classe $D_4$, une famille préférée de médicaments conformes à l'invention est constituée par ceux dans lesquels $R_6$ représente un atome d'hydrogène.

Une autre classe de médicaments préférés conformes à l'invention est constituée par les médicaments de formule (XIII) :

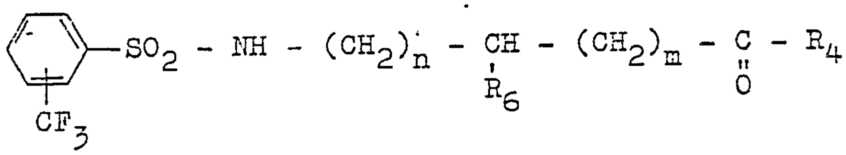
(XIII)

dans laquelle $R_4$ représente OH ou $C_2H_5$ et $n + m + 1$ est compris de 1 à 11, étant de préférence égal à 3, 5 ou 10.

Ce groupe de médicaments sera par la suite désigné par $D_5$.

Parmi cette classe $D_5$, une famille préférée de médicaments conformes à l'invention est constituée par ceux dans lesquels $R_6$ représente un atome d'hydrogène.

Ces médicaments répondent à la formule (XIV) suivante :

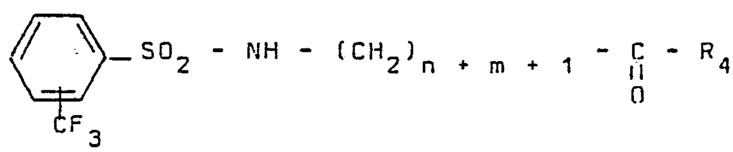
(XIV)

Une autre classe de médicaments préférés conformes à l'invention est constituée par les médicaments de formule (XIII) :

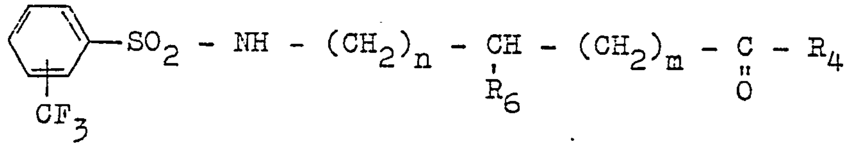
(XIII)

dans laquelle $R_4$ représente :

— le groupe hydroxy,

— le radical $OR_7$ dans lequel $R_7$ est un groupe alkyle ayant de 1 à 6 atomes de carbone, de préférence de 1 à 4 atomes de carbone ;

— le radical

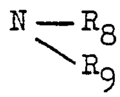

dans lequel $R_8$ et $R_9$ identiques ou différents représentent un atome d'hydrogène, un groupe alkyle ayant de 1 à 6 atomes de carbone, de préférence de 1 à 4 atomes de carbone,
et n + m + 1 vaut 3, 5 ou 10.

Cette classe de médicaments sera par la suite désignée par $D_6$.

Parmi cette classe $D_6$, une famille préférée de médicaments conformes à l'invention est constituée par ceux dans lesquels $R_6$ représente un atome d'hydrogène.

Une autre classe de médicaments préférés conformes à l'invention est constituée par les médicaments de formule (XV) suivante :

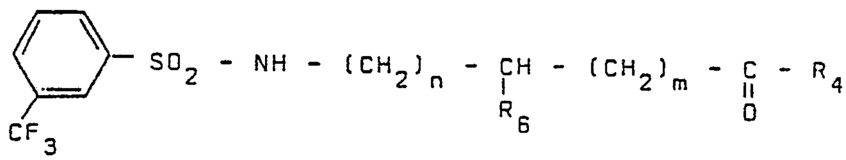 (XV)

dans laquelle $R_4$ représente :

— le groupe hydroxy,

— le radical $OR_7$ dans lequel $R_7$ est un groupe alkyle ayant de 1 à 6 atomes de carbone, et de préférence le groupe $C_2H_5$,

— le radical

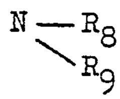

dans lequel $R_8$ et $R_9$ identiques ou différents représentent un atome d'hydrogène, un groupe alkyle ayant de 1 à 6, de préférence de 1 à 4 atomes de carbone, ou conjointement forment avec l'azote un hétérocycle azoté à 5 ou 6 chaînons, notamment un groupe pipéridino, morpholino, pyrrolidino, pyrrole ou pyrroline ;

n + m + 1 est compris de 1 à 11, et prend de préférence les valeurs de 3, 5 ou 10.

Cette classe de médicaments sera par la suite désignée par $D_7$.

Parmi cette classe $D_7$, une famille préférée de médicaments conformes à l'invention est constituée par ceux dans lesquels $R_6$ représente un atome d'hydrogène.

Ces médicaments répondent à la formule (XVI) suivante :

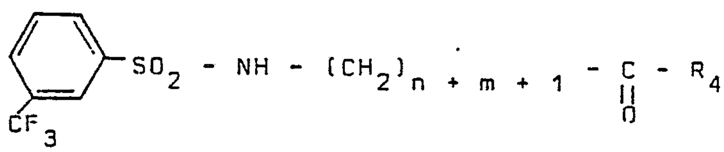 (XVI)

Selon encore un autre mode de réalisation préféré de l'invention, une classe préférée de médicaments répond à la formule (I) dans laquelle :

Ar représente le cycle de formule :

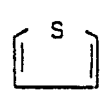

ce cycle pouvant éventuellement être substitué notamment en α du soufre par un halogène, notamment le chlore, ou par un radical méthyle.

Le radical Ar est de préférence rattaché à la chaîne :

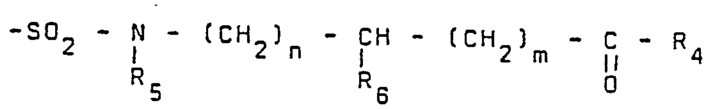

par l'intermédiaire de l'atome de carbone situé en α du soufre.

Ce groupe de médicaments sera par la suite désigné par W.

Selon un autre mode de réalisation, une classe préférée de médicaments répond à la formule (I) dans laquelle Ar représente le cycle de formule :

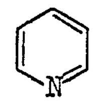

ce cycle pouvant éventuellement être substitué par $CF_3$, un halogène, notamment par le chlore, ou un alkyle ayant de 1 à 6 atomes de carbone, notamment le méthyle.

Dans cette classe de médicaments, le radical Ar est de préférence attaché à la chaîne :

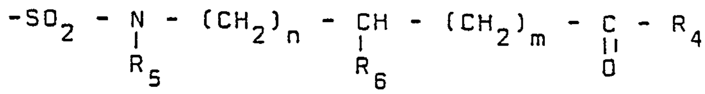

par l'intermédiaire de l'atome de carbone situé en méta de l'azote.

Ce groupe de médicaments sera par la suite désigné par X.

Parmi ce groupe X, une classe préférée de médicaments est constituée par ceux dans lesquels $R_6$ représente un atome d'hydrogène.

Ces médicaments répondent à la formule :

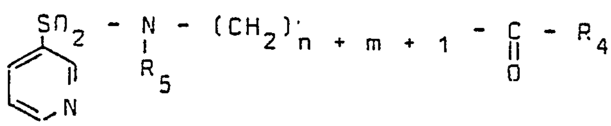

Une autre famille de médicaments est constituée par ceux de formule (III) :

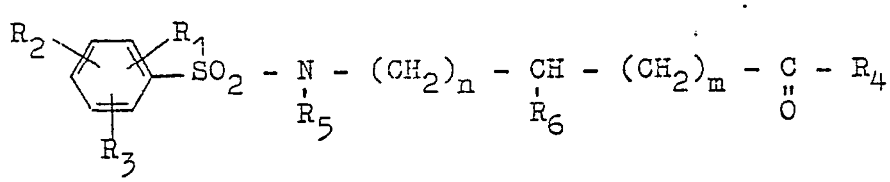

(III)

dans laquelle $R_1$, $R_2$ représentent l'hydrogène et $R_3$ un halogène, notamment le chlore, $NO_2$ et $R_5$ représente un radical alcoyle ayant de 1 à 6 atomes de carbone, notamment le radical méthyle.

Ce groupe de médicaments sera par la suite désigné par Q.

Parmi ce groupe Q, une classe préférée de médicaments est constituée par ceux dans lesquels $R_6$ représente un atome d'hydrogène.

Dans un groupe préféré de médicaments selon l'invention, appartenant à l'un quelconque des groupes $A_1$, $A_2$, W, X ou Q, n prend avantageusement les valeurs 3, 5 ou 10.

Ce groupe de médicaments sera par la suite désigné par Y.

Dans un autre groupe préféré de médicaments, selon l'invention, appartenant à l'un quelconque des groupes A, W, X, Y ou Q, $R_4$ représente avantageusement le radical hydroxy ou le radical $OC_2H_5$.

Ce groupe de médicaments sera par la suite désigné par Z.

L'invention concerne également les médicaments contenant à titre de substances actives les isomères optiques des composés de formule (I), ainsi que les sels physiologiquement acceptables de ces isomères optiques.

Les médicaments particulièrement préférés selon l'invention sont ceux de formule :

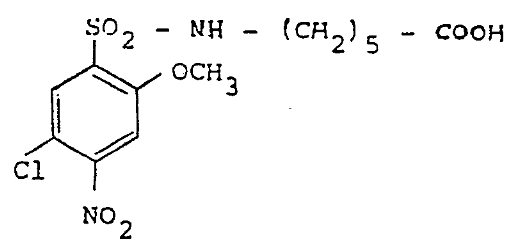

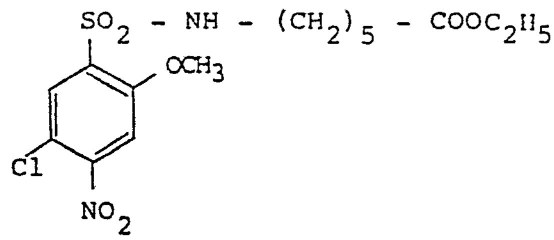

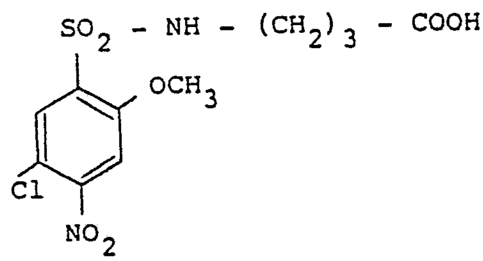

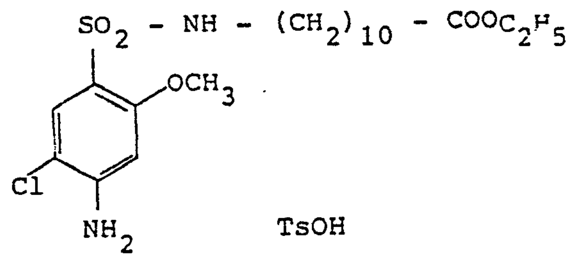

TsOH

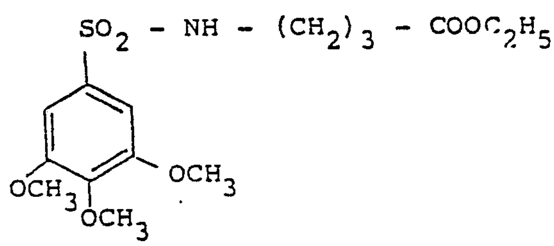

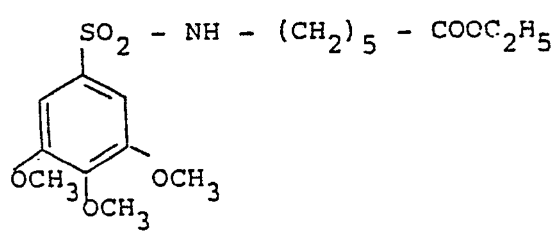

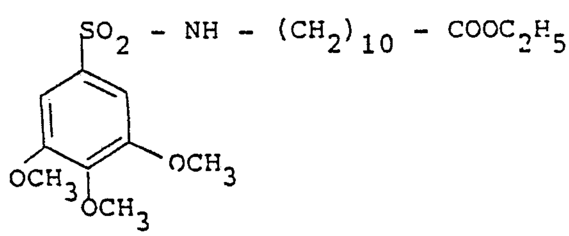

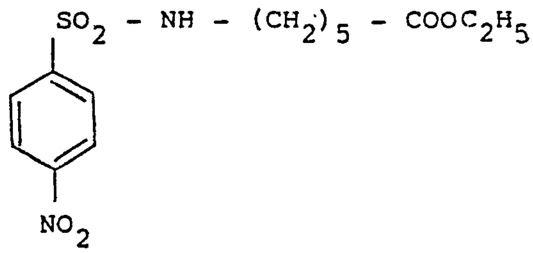

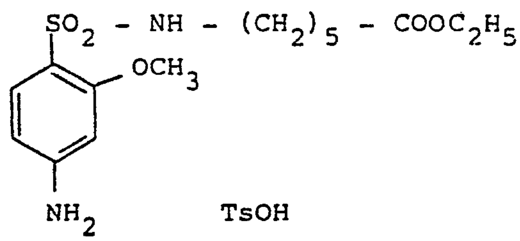

TsOH

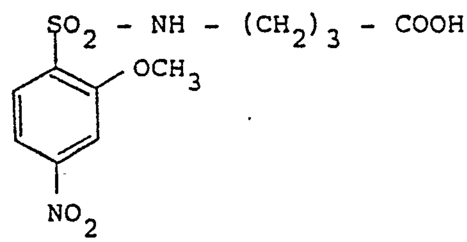

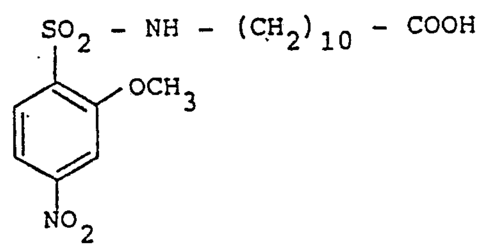

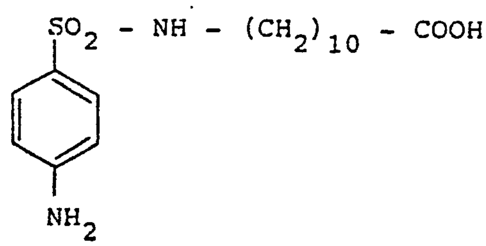

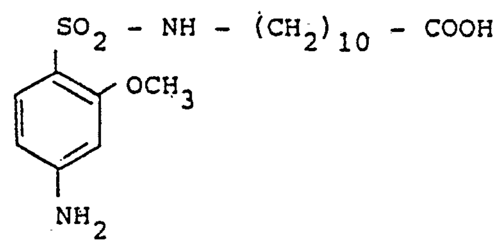

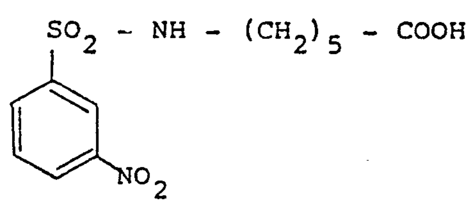

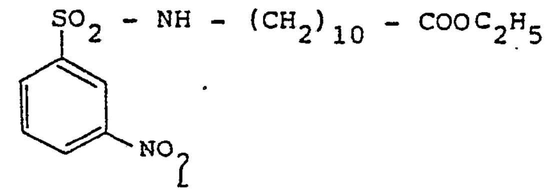

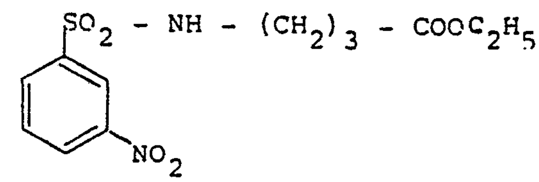

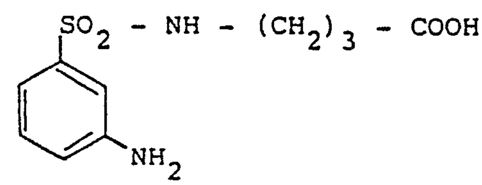

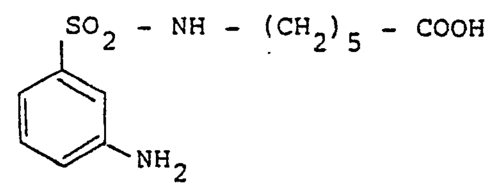

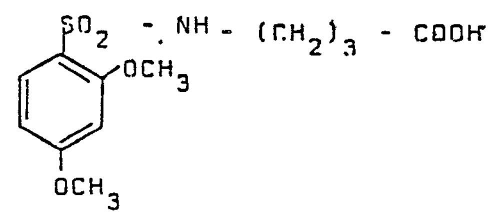

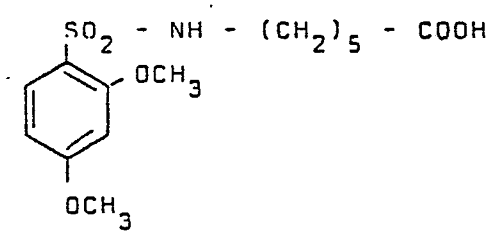

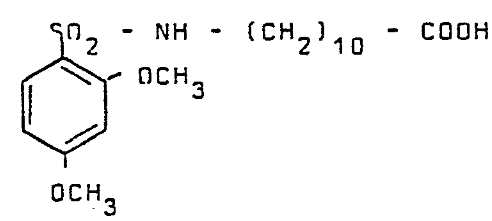

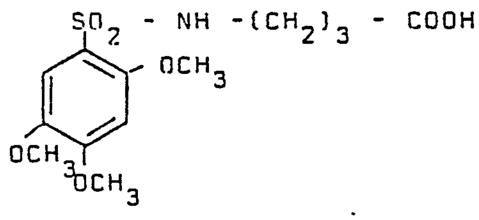

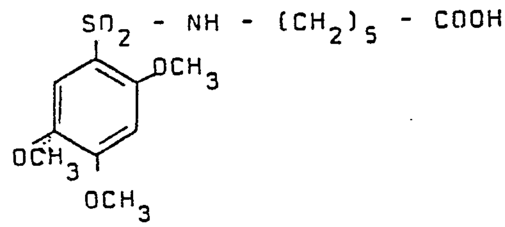

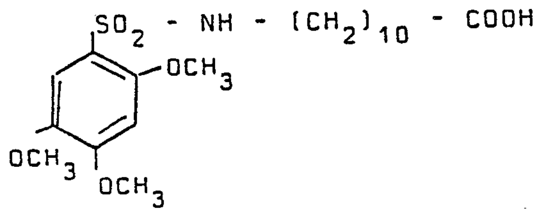

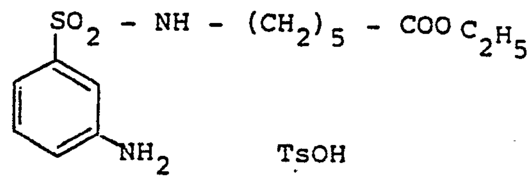

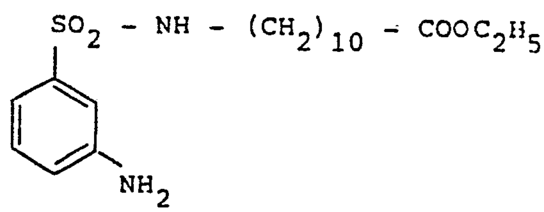

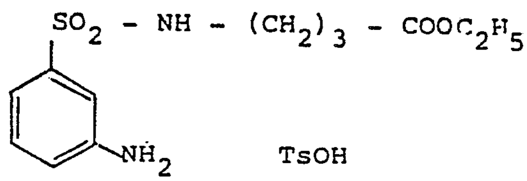

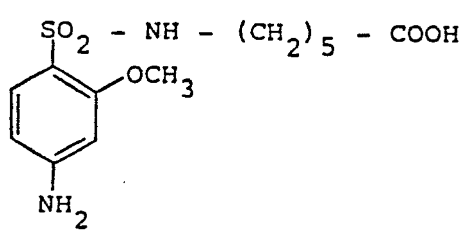

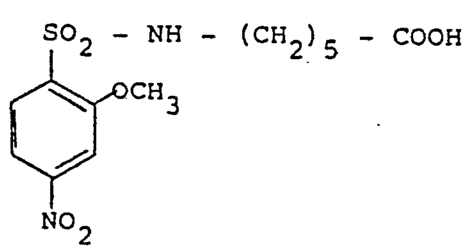

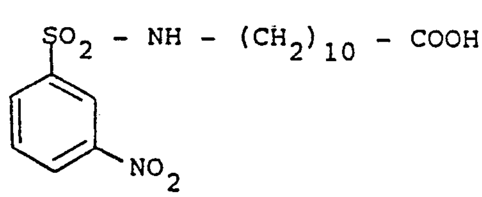

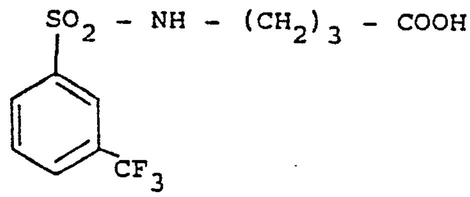

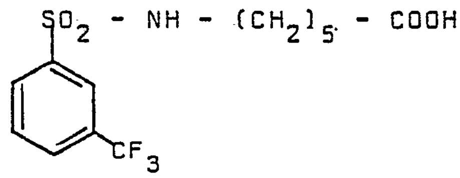

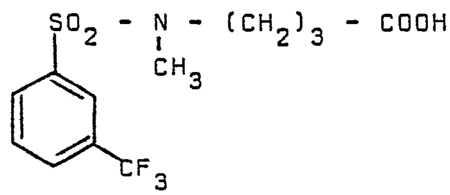

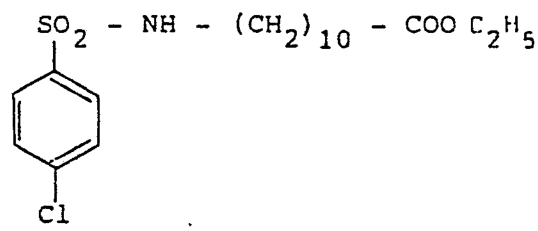

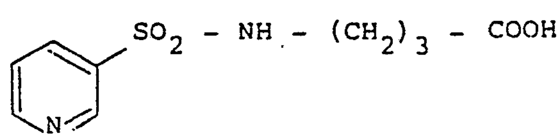

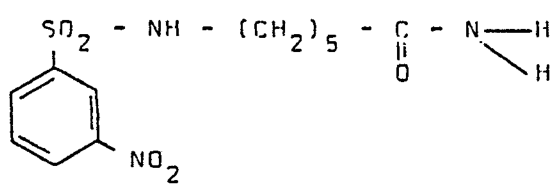

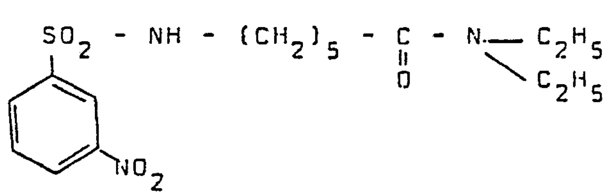

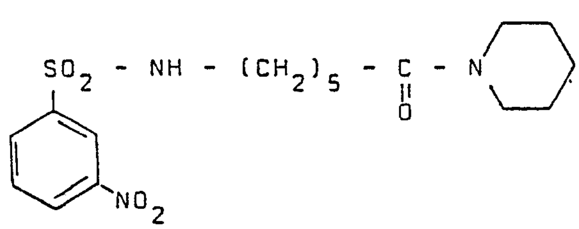

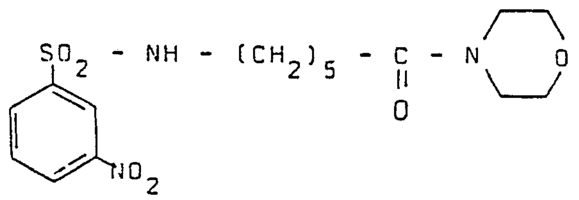

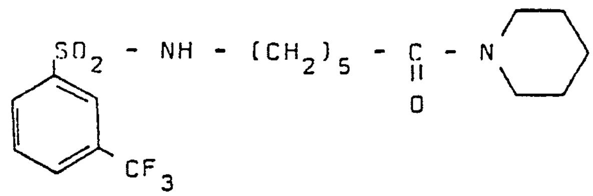

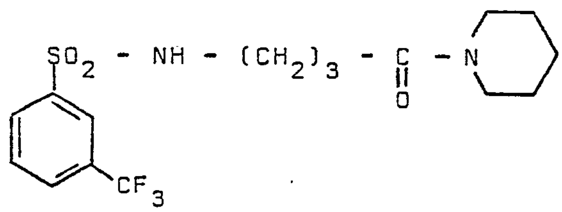

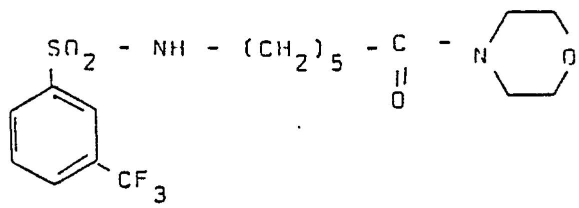

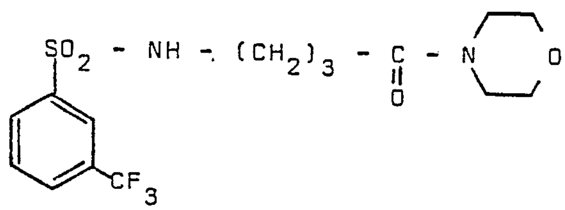

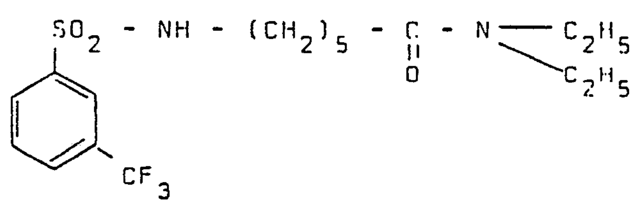

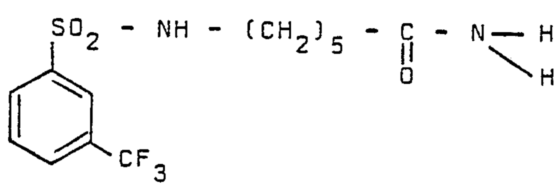

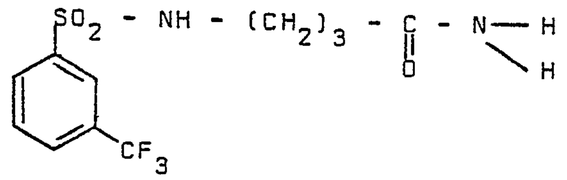

Les composés entrant dans les compositions pharmaceutiques (médicaments) constituent des produits industriels nouveaux, sous la réserve que lorsque Ar représente le noyau benzénique et lorsque $R_4$ représente un groupe hydroxy ou le radical $OR_7$, l'un au moins des trois substituants $R_1$, $R_2$ ou $R_3$ représente le radical $CF_3$.

Font donc partie de l'invention et sont revendiqués, les groupes de produits nouveaux, correspondant aux groupes respectifs de médicaments ci-dessus définis, avec la condition que lorsque Ar représente le noyau benzénique et que $R_4$ représente le groupe hydroxy ou le radical $OR_7$, l'un au moins des trois substituants $R_1$, $R_2$ ou $R_3$ représente $CF_3$.

Par conséquent, les produits industriels nouveaux de l'invention correspondent aux composés entrant dans les compositions pharmaceutiques ci-dessus définies, sous la réserve que lorsque Ar représente le noyau benzénique et lorsque $R_4$ est différent du radical

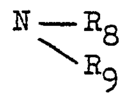

l'un au moins des trois substituants $R_1$, $R_2$ ou $R_3$ représente $CF_3$.

Les classes de composés préférées correspondent aux classes préférées de médicaments ci-dessus définis, avec la condition ci-dessus indiquée.

Les composés particulièrement préférés selon l'invention sont ceux de formule :

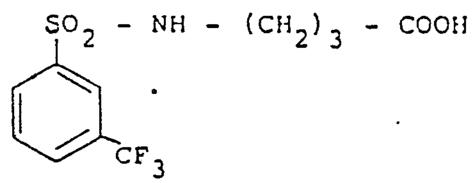

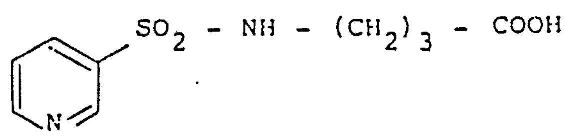

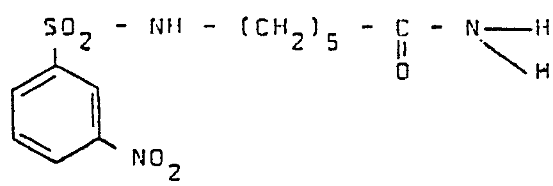

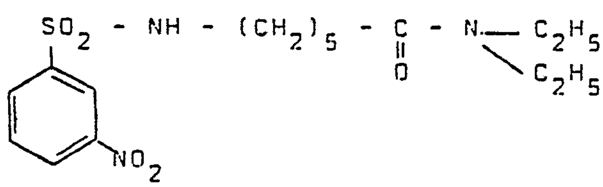

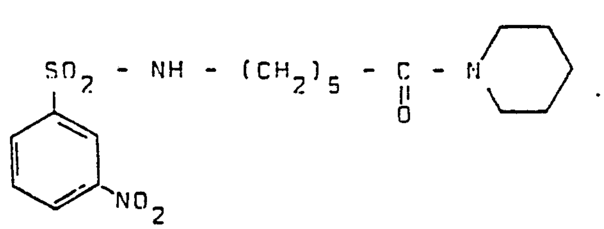

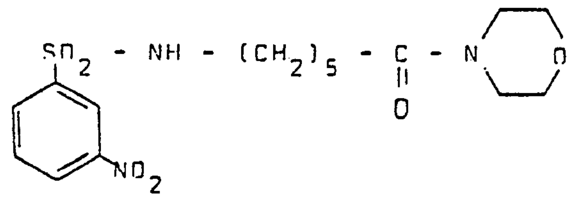

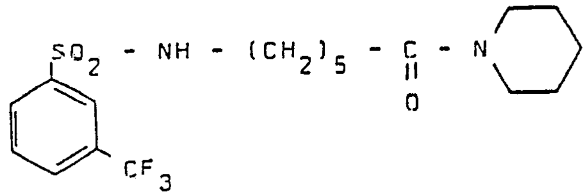

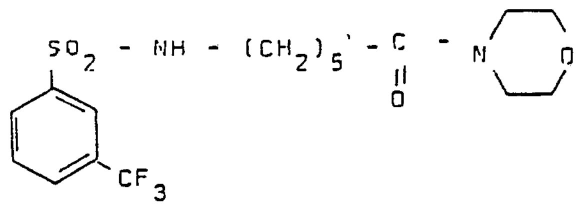

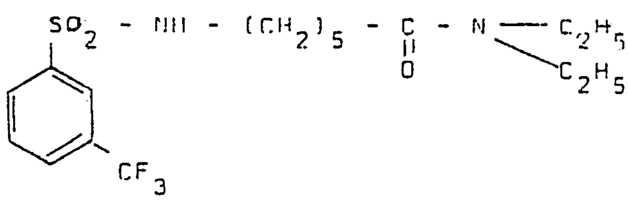

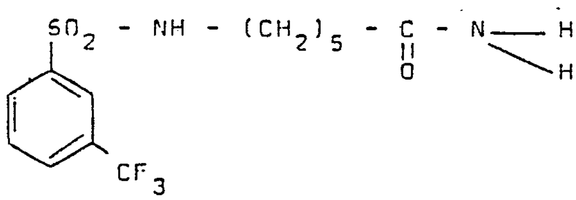

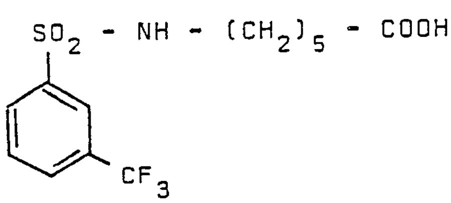

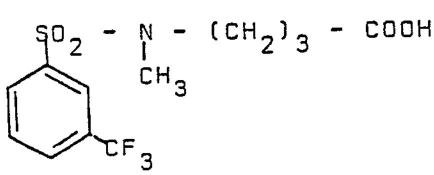

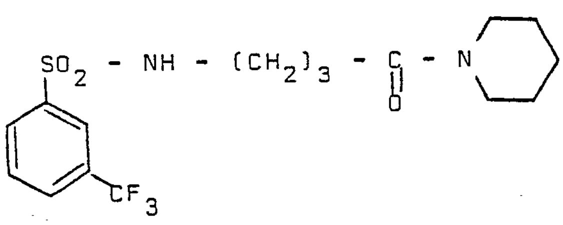

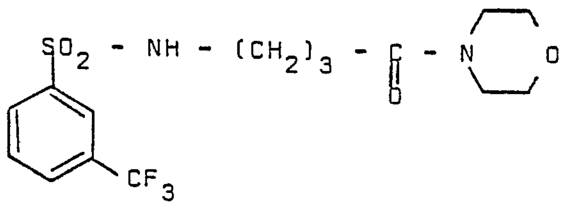

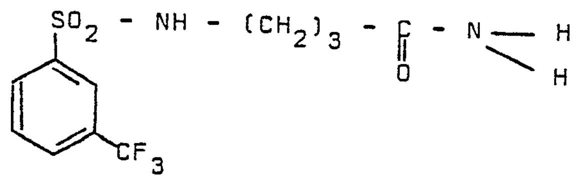

Synthèse des médicaments et des composés nouveaux selon l'invention

Dans ce qui suit, on décrit la synthèse des composés nouveaux selon l'invention ainsi que celle des médicaments selon l'invention.

Un premier mode de préparation (PROCEDE IA) des médicaments de formule :

$$Ar-SO_2-N(R_5)-(CH_2)_n-CH(R_6)-(CH_2)_m-C(=O)-R_4$$

dans laquelle :

$R_4$ représente le radical $OR_7$, $R_7$ étant un groupe alkyle ayant de 1 à 6 atomes de carbone,

$R_5$ et $R_6$ ont les significations indiquées ci-dessus,

et des composés nouveaux de formule :

$$Ar-SO_2-N(R_5)-(CH_2)_n-CH(R_6)-(CH_2)_m-C(=O)-R_4$$

dans laquelle

$R_4$ représente le radical $OR_7$, $R_7$ étant un groupe alkyle ayant de 1 à 6 atomes de carbone,

$R_5$ et $R_6$ ont les significations indiquées ci-dessus, avec la condition que lorsque Ar représente le noyau benzénique, celui-ci soit substitué par au moins un groupe $CF_3$ ;

consiste à faire réagir un halogénure, notamment un chlorure d'acide approprié de formule :

$$Ar-SO_2-Cl$$

avec un amino-ester de formule :

$$HN(R_5)-(CH_2)_n-CH(R_6)-(CH_2)_m-C(=O)-OR_7$$

ou son chlorhydrate de formule :

$$HN(R_5)-(CH_2)_n-CH(R_6)-(CH_2)_m-C(=O)-OR_7,\ HCl$$

selon le schéma suivant :

$$Ar-SO_2-Cl+HN(R_5)-(CH_2)_n-CH(R_6)-(CH_2)_m-C(=O)-OR_7,\ HCl$$

$$\longrightarrow Ar-SO_2-N(R_5)-(CH_2)_n-CH(R_6)-(CH_2)_m-C(=O)-OR_7$$

Dans ces formules, Ar, n, m, $R_5$, $R_6$, $R_7$ ont les significations indiquées précédemment.

D'un point de vue pratique, on procède avantageusement comme suit.

L'amino-ester ou son chlorhydrate ($2 \times 10^{-2}$ mole) dans du benzène anhydre (50 $cm^3$) est agité vigoureusement à une température de 0° à 5 °C ; la triéthylamine ou toute autre base organique du type amine tertiaire, est alors ajoutée (15 à 20 $cm^3$), puis, 5 à 10 mn plus tard, le chlorure d'acide ($2 \times 10^{-2}$ mole) est ajouté par petites portions ou goutte à goutte, selon qu'il se présente sous forme solide ou liquide. La triéthylamine est ajoutée en excès car elle permet de neutraliser, le cas échéant le chlorhydrate de l'amino-acide sous forme de sa base correspondante, et dans tous les cas, de neutraliser l'acide chlorhydrique formé au cours de la réaction de condensation entre $ArSO_2Cl$ et l'amino-ester. Le milieu réactionnel revient progressivement à température ambiante et, après 12 heures, les cristaux de chlorhydrate de triéthylamine formés sont filtrés et rincés au benzène. La phase organique est concentrée, puis reprise par de l'acétate d'éthyle (200 $cm^3$) ; cette dernière solution est lavée successivement par des solutions d'acide chlorhydrique dilué et du bicarbonate de sodium dilué, séchée et le solvant est évaporé. Le résidu ainsi obtenu est chromatographié si nécessaire.

De façon générale, le chlorure d'acide est disponible dans le commerce.

Lorsqu'il n'est pas disponible, on peut le synthétiser par des méthodes classiques.

Par exemple, dans le cas du chlorure de thiophène-2 sulfonyle, on procède comme il est indiqué par E. MACCARONE, G. MUSUMARRA et G. A. TOMASELLI, Ann. Chim. Rom, 1973, *63*, 861, selon le schéma suivant :

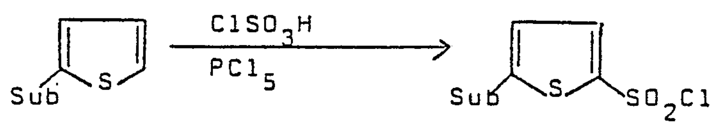


Un groupe préféré des sulfochlorures de thiophène est constitué par ceux de formule :

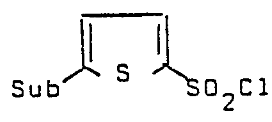

dans laquelle Sub représente un atome d'halogène, un groupe alkyle ayant de 1 à 3 atomes de carbone et de préférence 1 atome de carbone, notamment le chlore ou le radical $CH_3$.

Dans le cas du chlorure de pyridine-3 sulfonyle, on procède, comme il est indiqué par M. F. ZIENTY, J. Amer. Pharm. Assoc., 1948, *37*, 97 selon le schéma suivant :

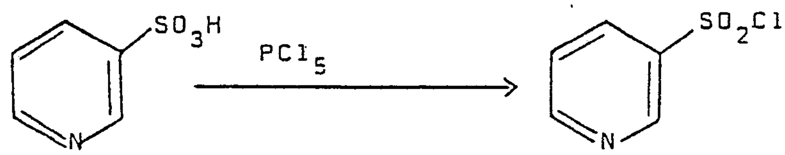


L'exemple suivant illustre le mode de préparation de médicaments selon l'invention, préparation pour laquelle on a recours au procédé ci-dessus indiqué dans sa généralité.

Exemple I — Préparation de l'ester éthylique de l'acide (thiophène-2-sulfonylamino)-11 undécanoïque

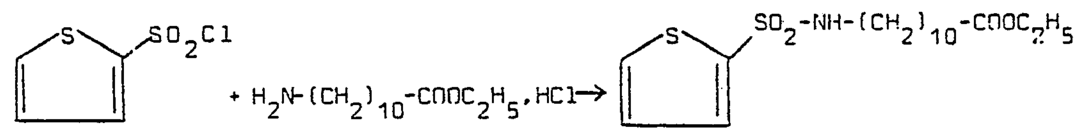

PM = 182,5 PM = 265,83 PM = 375,56

A une suspension de chlorhydrate de l'ester éthylique de l'acide amino-11 undécanoïque ($4{,}5 \times 10^{-2}$ mole = 11,96 g) dans du benzène anhydre (100 $cm^2$), refroidie dans un bain d'eau et de glace et sous agitation magnétique, est ajoutée, après cinq minutes, de la triéthylamine (15 $cm^3$). Après dix minutes, est additionné le chlorure de thiophènesulfonyl ($4{,}5 \times 10^{-2}$ mole = 8,6 g) dans du benzène anhydre (10 $cm^3$).

Après une nuit sous agitation, à température ambiante, le milieu réactionnel est filtré (les cristaux de chlorhydrate de triéthylamine sont éliminés après avoir été rincés par du benzène) et le filtrat concentré sous pression réduite. Le résidu est extrait par de l'acétate d'éthyle (200 $cm^3$) en présence d'une solution d'acide chlorhydrique à 5 % (200 $cm^3$) ; la phase organique est ensuite lavée successivement par de l'eau, une solution de bicarbonate de sodium à 5 % et de l'eau, séchée sur du sulfate de sodium et concentrée sous pression réduite. Le produit est chromatographié sur silice H et le produit désiré, élué, par le mélange benzène(acétate d'éthyle (9 : 1), est obtenu pur avec un rendement de 91 %.

Ont été préparés, selon ce procédé, les médicaments figurant dans le tableau I ci-après, ainsi que les composés nouveaux figurant dans le tableau I' ci-après.

Tableau I

Premier mode de synthèse des médicaments de formule : $Ar-SO_2-NH-(CH_2)_{n+m+1}-C(=O)-OR_7$

| n° | Ar | n+m+1 | $R_7$ | F °C | Formule brute/PM | Rendement de la réaction (%) |
|---|---|---|---|---|---|---|
| 486 | 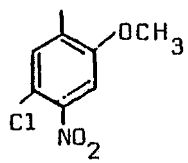 | 3 | $C_2H_5$ | 110 | $C_{13}H_{17}ClN_2O_7S$ = 380,60 | 65 (95) |
| 735 | " | 4 | " | 116 | $C_{14}H_{19}ClN_2O_7S$ = 394,85 | 84 |
| 380 | " | 5 | " | 68 | $C_{15}H_{21}ClN_2O_7S$ = 408,86 | 64 (55) |
| 508 | " | 7 | " | 70 | $C_{17}H_{25}ClN_2O_7S$ = 436,91 | 63 |
| 502 | " | 10 | " | 83 | $C_{20}H_{31}ClN_2O_7S$ = 478,99 | 83 (97) |
| 807 | " | 11 | " | 88 | $C_{21}H_{33}ClN_2O_7S$ = 493,03 | 69 |
| 1 027 | 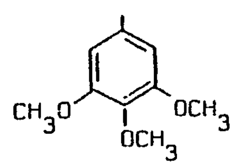 | 3 | " | 94 | $C_{15}H_{23}NO_7S$ = 361,42 | 55 |

| n° | Ar | n+m+1 | $R_7$ | F °C | Formule brute/PM | Rendement de la réaction (%) |
|---|---|---|---|---|---|---|
| 865 | 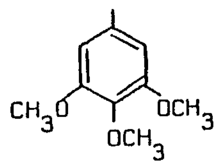 | 5 | $C_2H_5$ | 120 | $C_{17}H_{27}NO_7S$ = 389,48 | 40 |
| 1 028 | " | 10 | " | 77 | $C_{22}H_{37}NO_7S$ = 459,61 | 40 |
| 625 | 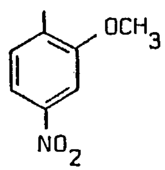 | 3 | " | 97 | $C_{13}H_{18}N_2O_7S$ = 346,36 | (72) |
| 608 | " | 5 | " | 88 | $C_{15}H_{22}N_2O_7S$ = 374,41 | 86 |
| 611 | " | 10 | " | 68 | $C_{20}H_{32}N_2O_7S$ = 444,55 | 83 |
| 623 | 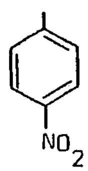 | 3 | " | 94 | $C_{12}H_{16}N_2O_6S$ = 316,38 | (88) |
| 563 | " | 5 | " | 83 | $C_{14}H_{20}N_2O_6S$ = 344,39 | 89 |

| n° | Ar | n+m+1 | $R_7$ | F °C | Formule brute/PM | Rendement de la réaction (%) |
|---|---|---|---|---|---|---|
| 610 | 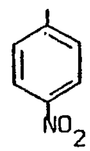 | 10 | $C_2H_5$ | 71 | $C_{19}H_{30}N_2O_6S$ = 414,52 | 66 |
| 679 | 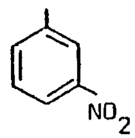 | 3 | " | 91 | $C_{12}H_{16}N_2O_6S$ = 316,33 | 80 |
| 666 | " | 5 | " | 65 | $C_{14}H_{20}N_2O_6S$ = 344,39 | 93 |
| 676 | " | 10 | " | 66 | $C_{19}H_{30}N_2O_6S$ = 414,52 | 96 |
| 737 | 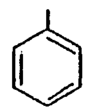 | 3 | " | liquide | $C_{12}H_{17}NO_4S$ = 271,34 | 97 |
| 750 | " | 5 | " | " | $C_{14}H_{21}NO_4S$ = 299,40 | 88 |
| 766 | " | 10 | " | 43 | $C_{19}H_{31}NO_4S$ = 369,53 | 99 |

| n° | Ar | n+m+1 | R 7 | F °C | Formule brute/PM | Rendement de la réaction (%) |
|---|---|---|---|---|---|---|
| 739 | 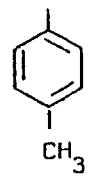 | 3 | $C_2H_5$ | 49 | $C_{13}H_{19}NO_4S$ = 285,37 | 95 |
| 752 | " | 5 | " | 47 | $C_{15}H_{23}NO_4S$ = 313,42 | 80 |
| 768 | " | 10 | " | 58 | $C_{20}H_{33}NO_4S$ = 383,56 | 99 |
| 743 | 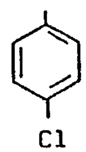 | 3 | " | 84 | $C_{12}H_{16}ClO_4S$ = 305,79 | 90 |
| 756 | " | 5 | " | 49 | $C_{14}H_{20}ClNO_4S$ = 333,85 | 99 |
| 728 | " | 10 | " | 60 | $C_{19}H_{30}ClNO_4S$ = 403,98 | 99 |
| 741 | 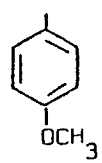 | 3 | " | liquide | $C_{13}H_{19}NO_5S$ = 301,37 | 99 |

| n° | Ar | n+m+1 | $R_7$ | F °C | Formule brute/PM | Rendement de la réaction (%) |
|---|---|---|---|---|---|---|
| 754 | 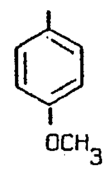 | 5 | $C_2H_5$ | liquide | $C_{15}H_{23}NO_5S$ = 329,42 | 89 |
| 729 | " | 10 | " | 47 | $C_{20}H_{33}NO_5S$ = 399,56 | 89 |
| 745 | 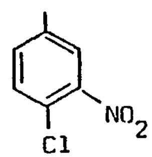 | 3 | " | 79 | $C_{12}H_{15}ClN_2O_6S$ = 350,79 | 91 |
| 758 | " | 5 | " | 67 | $C_{14}H_{19}Cl_2N_2O_6S$ = 376,55 | 85 |
| 772 | " | 10 | " | 68 | $C_{19}H_{29}ClN_2O_6S$ = 448,98 | 87 |
| 784 | 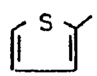 | 3 | " | 46 | $C_{10}H_{15}NO_4S_2$ = 277,37 | 84 |
| 802 | " | 5 | " | 42 | $C_{12}H_{19}NO_4S_2$ = 305,42 | 78 |
| 806 | " | 10 | " | 54 | $C_{17}H_{29}NO_4S_2$ = 375,55 | 91 |

| n° | Ar | n+m+1 | $R_7$ | F °C | Formule brute/PM | Rendement de la réaction (%) |
|---|---|---|---|---|---|---|
| 852 | 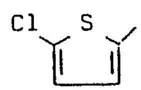 | 5 | butyl | liquide | $C_{14}H_{22}ClNO_4S_2$ = 367,93 | 72 |
| 866 | " | 5 | $C_2H_5$ | " | $C_{12}H_{18}ClNO_4S_2$ = 339,87 | 68 |
| 867 | " | 3 | " | " | $C_{10}H_{14}ClNO_4S_2$ = 311,68 | 75 |
| 853 | 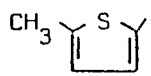 | 3 | " | | $C_{11}H_{17}NO_4S_2$ = 291,40 | 83 |
| 787 | 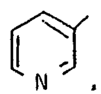 , HCl | 3 | " | 111 | $C_{11}H_{17}ClN_2O_4S$ = 308,88 | 76 |
| 790 | " , HCl | 5 | " | 93 | $C_{13}H_{21}ClN_2O_4S$ = 336,83 | 73 |
| 791 | | 10 | " | 53 | $C_{18}H_{30}N_2O_4S$ = 370,62 | 77 |

Tableau I'

Premier mode de synthèse des composés de formule : $Ar—SO_2—NH—(CH_2)_{n+m+1}—C(=O)—OR_7$

| n° | Ar | n + m + 1 | $R_7$ | F °C | Formule brute/PM | Rendement de la réaction (%) |
|---|---|---|---|---|---|---|
| 784 | 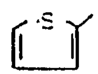 | 3 | $C_2H_5$ | 46 | $C_{10}H_{15}NO_4S_2$ = 277,37 | 84 |
| 802 | " | 5 | " | 42 | $C_{12}H_{19}NO_4S_2$ = 305,42 | 78 |
| 806 | " | 10 | " | 54 | $C_{17}H_{29}NO_4S_2$ = 375,56 | 91 |
| 852 | 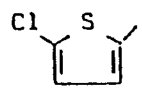 | 5 | butyl | liquide | $C_{14}H_{22}ClNO_4S_2$ = 367,93 | 72 |
| 866 | " | 5 | $C_2H_5$ | " | $C_{12}H_{18}ClNO_4S_2$ = 339,87 | 68 |
| 867 | " | 3 | " | " | $C_{10}H_{14}ClNO_4S_2$ = 311,80 | 75 |
| 853 | 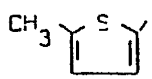 | 3 | " | | $C_{11}H_{17}NO_4S_2$ = 291,40 | 83 |
| 787 | 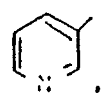 , HCl | 3 | " | 111 | $C_{11}H_{17}ClN_2O_4S$ = 308,39 | 76 |
| 790 | " HCl | 5 | " | 93 | $C_{13}H_{21}ClN_2O_4S$ = 336,83 | 73 |
| 791 | | 10 | " | 53 | $C_{18}H_{30}N_2O_4S$ = 370,52 | 77 |

Un deuxième mode de préparation (PROCEDE IIA) des médicaments de formule :

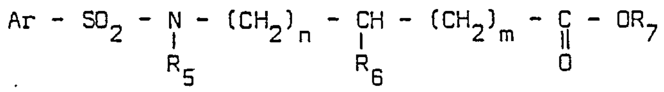

et des composés nouveaux correspondants, consiste à partir d'un halogénure d'acide, notamment le chlorure d'acide $ArSO_2Cl$ approprié et à le faire réagir sur un amino-acide de formule :

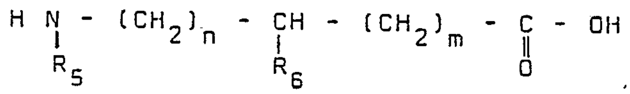

selon le schéma suivant :

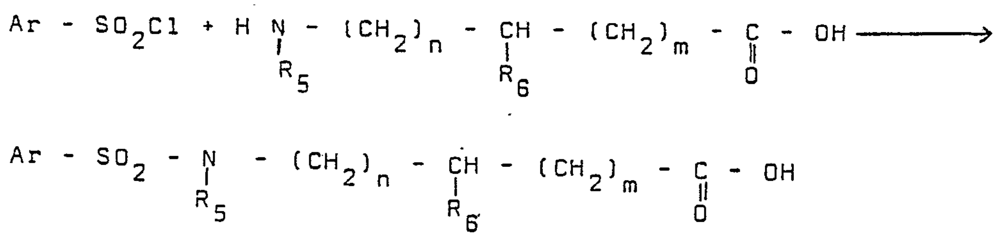

puis à estérifier l'acide ainsi obtenu, en le faisant réagir sur un alcool approprié de formule $R_7OH$ selon le schéma suivant :

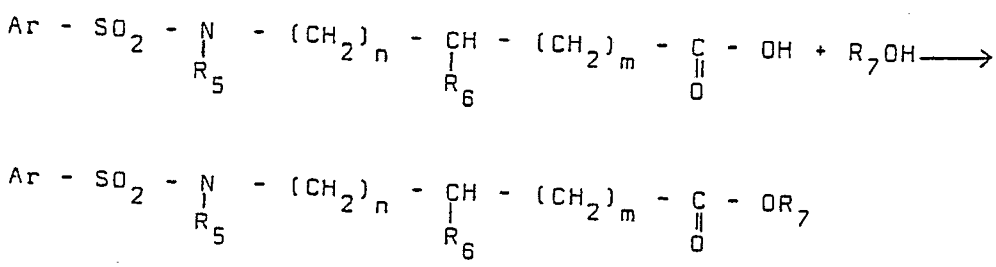

Dans ces formules, Ar, n, $R_5$, $R_6$ et $R_7$ ont les mêmes significations que celles indiquées précédemment.

D'un point de vue pratique, on procède comme suit : l'obtention de l'acide a lieu de la façon suivante : l'amino-acide (40 nM) est mis en suspension ou solubilisé dans une base organique anhydre telle que la pyridine anhydre, la 2,6-diméthylpyridine anhydre (15 $cm^3$). Le chlorure d'acide (10 nM) est alors ajouté par petites fractions (ou goutte à goutte) ; le mélange réactionnel est agité vigoureusement pendant cette addition et maintenu à une température inférieure ou égale à 20 °C. Puis on chauffe une heure à 40 °C et on laisse 12 heures sous agitation à température ambiante. La pyridine est chassée par concentration sous pression réduite ; une solution refroidie d'acide dilué est ajoutée pour amener le pH à une valeur inférieure à 3, tel que l'acide chlorhydrique dilué. Si le produit précipite, il est filtré, lavé à l'eau, dissous dans un alcool inférieur (éthanol ou méthanol), éventuellement décoloré sur charbon actif et enfin chromatographié. Si le produit ne précipite pas, il est extrait du milieu par de l'acétate d'éthyle (ou tout autre solvant organique de même polarité) : cette phase est séchée sur sulfate de sodium, éventuellement décolorée sur un charbon actif, concentrée sous pression réduite et chromatographiée.

Une variante a également été suivie ; elle consiste à verser une solution éthérée de chlorure d'acide sur l'amino-acide en solution dans de la soude aqueuse N (raf. : MAC CHESMY, SWAM, J.A.C.S., *59*, 1 116).

L'estérification, quant à elle, a lieu de façon suivante :

l'acide ($2 \times 10^{-2}$ mole) est solubilisé dans 30 $cm^3$ d'alcool anhydre ; est ensuite ajouté de l'acide perchlorique (1,5 $cm^3$) ; puis le mélange est chauffé à 50 °C jusqu'à disparition du produit de départ au profit de l'ester.

L'exemple suivant illustre le mode de préparation des médicaments, préparation pour laquelle on a recours au procédé ci-dessus indiqué dans sa généralité.

Exemple II — Préparation de l'acétate d'éthyle de l'acide ε(chloro 5-nitro 4-méthoxy 2-benzènesulfonamido) caproïque

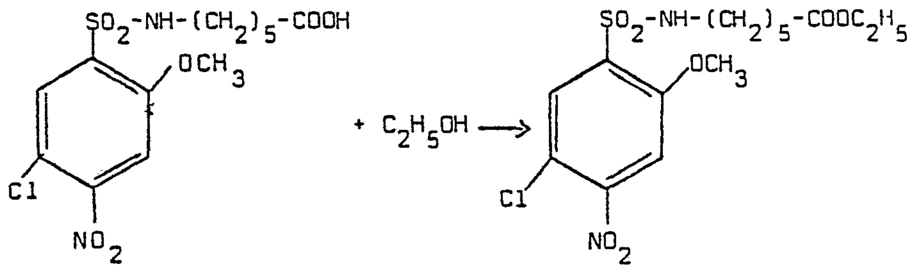

PM = 380,5 PM = 408,9

L'acide (3 g) est agité magnétiquement dans de l'éthanol anhydre (100 cm$^3$) en présence d'acide perchlorique (2 cm$^3$) à 50 °C durant 48 heures (fin de réaction contrôlée par ccm). Le milieu est refroidi, neutralisé par du carbonate disodique, extrait par de l'éther. La phase organique est séchée sur sulfate de sodium, puis concentrée sous pression réduite. Par chromatographie sur silice H du résidu ainsi obtenu et l'élution par le mélange benzène/acétate d'éthyle (4 : 1), on isole l'ester éthylique de l'acide ε(chloro 5-nitro 4-méthoxy 2-benzènesulfonamido) caproïque avec un rendement de 55 %.

Ont été préparés, selon ce procédé, les médicaments suivants : 380, 486, 502 dont les formules chimiques développées se trouvent dans le tableau I.

Un troisième mode de préparation (PROCEDE III/A) des médicaments ainsi que des composés nouveaux correspondants, de formule :

$$Ar-SO_2-\underset{R_5}{N}-(CH_2)_n-\underset{R_6}{CH}-(CH_2)_m-\underset{O}{\overset{}{C}}-OR_7$$

consiste à partir d'un acide approprié de formule : $Ar-SO_2-H$ et à le faire réagir sur un amino-ester approprié de formule :

$$\underset{R_5}{HN}-(CH_2)_n-\underset{R_6}{CH}-(CH_2)_m-\underset{O}{C}-OR_7$$

selon le schéma réactionnel suivant :

$$Ar-SO_2-H+\underset{R_5}{HN}-(CH_2)_n-\underset{O}{C}-OR_7 \longrightarrow$$

$$Ar-SO_2-\underset{R_5}{N}-(CH_2)_n-\underset{R_6}{CH}-(CH_2)_m-\underset{O}{C}-OR_7$$

Dans ces formules correspondant aux médicaments, Ar, n, m, $R_5$, $R_6$ et $R_7$ ont des significations précédemment indiquées. Lorsqu'il s'agit des composés nouveaux, Ar, n, m, $R_5$, $R_6$ et $R_7$ ont également les significations précédemment indiquées avec la condition que lorsque Ar représente un noyau benzénique, celui-ci soit substitué par au moins un groupe $CF_3$.

D'un point de vue pratique, on procède comme suit : une solution d'acide ($10^{-2}$ mole) solubilisée dans le minimum de diméthylformamide (DMF) ou d'hexaméthylphosphoramide (HMPT) est refroidie à − 15 °C sous agitation magnétique. Sont ajoutés successivement à cette solution, une N-alkylamine tertiaire de 1 à 4 atomes de carbone, telle que la N-méthyl morpholine (1,1 cm$^3$ = $10^{-2}$ mole) et du chloroformiate d'alkyle de 1 à 4 atomes de carbone tel que l'isobutyle (1,3 cm$^3$). Au bout de 5 mn, est versé dans le milieu l'amino-ester ($10^{-2}$ mole) dans le minimum de DMF ou de HMPT (s'il s'agit du chlorhydrate de l'amino-ester, il convient de neutraliser par de la N-méthyl morpholine (1 cm$^3$ = $10^{-2}$ mole). 4 heures plus tard, le mélange réactionnel est versé dans une solution diluée de bicarbonate de sodium ou de potassium à 0 °C. Si le produit précipite, on lave les cristaux à l'eau, puis on les dissout dans de l'acétate d'éthyle et on lave la phase organique par un acide dilué pour amener le pH à une valeur inférieure à 3, tel que l'acide chlorhydrique dilué. Après avoir séché la solution sur sulfate de sodium, le solvant est évaporé et le résidu ainsi obtenu est chromatographié.

Un premier mode de préparation (PROCEDE IB) des médicaments et des composés nouveaux, de formule :

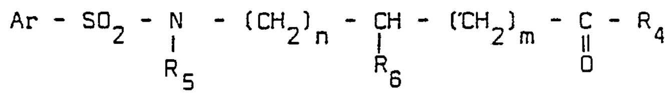

dans laquelle $R_4$ représente le radical hydroxy, consiste à faire réagir un chlorure d'acide approprié de formule :

$$Ar—SO_2—Cl$$

sur un amino-acide approprié de formule :

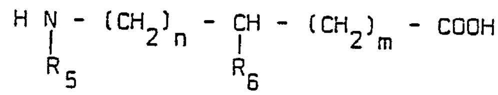

selon le schéma réactionnel suivant :

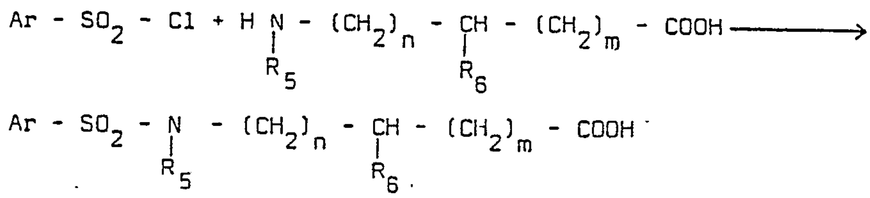

comme il a été indiqué précédemment dans le deuxième mode de préparation (PROCEDE IIA) des produits de formule :

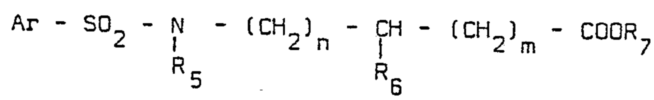

Les exemples suivants illustrent le mode de préparation de médicaments, préparation pour laquelle on a recours au procédé ci-dessus indiqué dans sa généralité.

Exemple III — Préparation de l'acide ε(nitro 4-méthoxy 2-benzènesulfonamido) caproïque

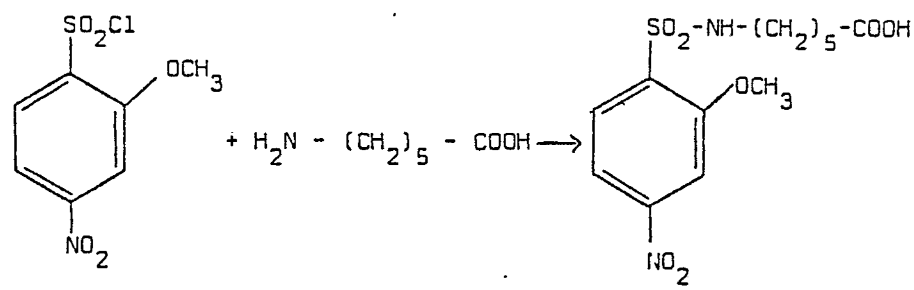

PM = 254,5 PM = 131 PM = 318,3

A de l'acide ε-amino caproïque ($5 \times 10^{-2}$ mole = 6,55 g) dans de la pyridine (150 $cm^3$), sous agitation magnétique et maintenu à une température d'environ 20 °C par un bain d'eau, est ajouté par petites fractions du chlorure de méthoxy-2 nitro-4 benzène sulfonyl ($2 \times 10^{-2}$ mole = 5,08 g).

Après addition, le mélange réactionnel est chauffé une heure à 40 °C. Le solvant est évaporé et le résidu ainsi obtenu est lavé par de l'acide chlorhydrique (200 $cm^3$) à 3 % puis extrait par de l'acétate d'éthyle (400 $cm^3$, puis deux fois 100 $cm^3$). Les phases organiques sont réunies, séchées sur sulfate de sodium et concentrées sous pression réduite. Le produit est solubilisé dans du méthanol, décoloré sur du charbon actif, concentré et chromatographié sur silice H par l'éluant toluène/acétate d'éthyle/acide acétique (80 : 20 : 2).

Les fractions contenant l'acide ε(nitro 4-méthoxy 2-benzènesulfonamido- caproïque sont concentrées et le composé est recristallisé dans un mélange eau/éthanol (rendement de 50 %).

## Exemple IV — Préparation de l'acide (trifluorométhyl-benzènesulfonylamino)-4 butyrique

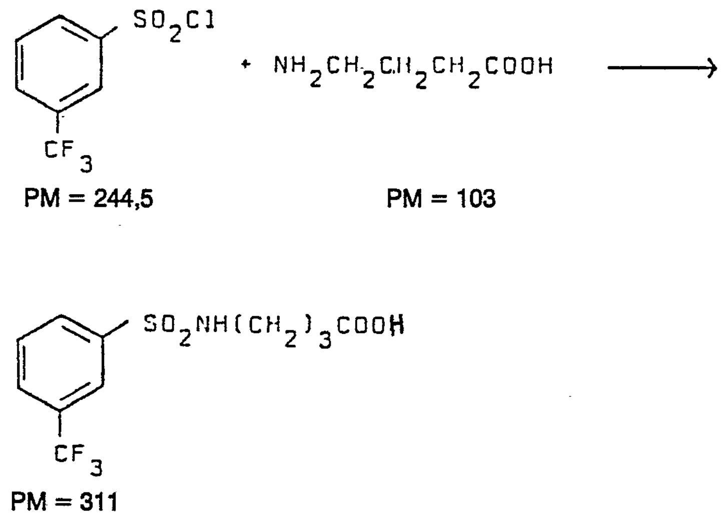


A de l'acide ω-aminobutyrique ($5 \times 10^{-2}$ mole = 5,15 g) dans de la pyridine (100 $cm^3$), sous agitation magnétique et maintenu à une température d'environ 20 °C par un bain d'eau, est ajouté goutte à goutte du chlorure de trifluorométhyl-benzènesulfonyl ($2 \times 10^{-2}$ mole : 4,89 g).

Après addition, le mélange réactionnel est chauffé une heure à 40 °C. Le solvant est évaporé et le résidu ainsi obtenu est repris dans 100 $cm^3$ d'eau. La solution est alors amenée à un pH de 2,3 par addition d'acide chlorhydrique 2N.

Le précipité essoré puis séché est recristallisé dans du benzène (rendement 42 %).

Ont été préparés selon le procédé, les médicaments suivants rassemblés dans le tableau II ci-après, ainsi que les composés nouveaux, rassemblés dans le tableau II' ci-après.

(Voir Tableau II pages suivantes)

Tableau II

Préparation des médicaments de formule :

$$Ar-SO_2-NH-(CH_2)_n-CH_2-(CH_2)_m-\underset{\underset{O}{\|}}{C}-OR_7$$

| n° | Ar | n+m+1 | $R_7$ | F °C | Formule brute/PM | Rendement de la réaction (%) |
|---|---|---|---|---|---|---|
| 387 | 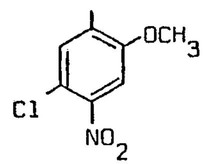 | 3 | H | 130 | $C_{11}H_{13}ClN_2O_7S$ = 352,75 | 16 |
| 362 | " | 5 | " | 145 | $C_{13}H_{17}ClN_2O_7S$ = 380,80 | 17 |
| 504 | " | 7 | " | 126 | $C_{15}H_{21}ClN_2O_7S$ = 408,86 | 31 |
| 501 | " | 10 | " | 126 | $C_{18}H_{27}ClN_2O_7S$ = 450,94 | 72 |
| 660 | 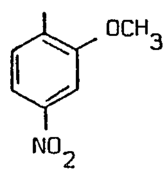 | 3 | " | 135 | $C_{11}H_{14}N_2O_7S$ = 318,30 | 32 |
| 696 | " | 5 | " | 134 | $C_{13}H_{18}N_2O_7S$ = 346,36 | 50 |
| 661 | " | 10 | " | 121 | $C_{18}H_{28}N_2O_7S$ = 416,49 | 52 |

| n° | Ar | n+m+1 | $R_7$ | F °C | Formule brute/PM | Rendement de la réaction (%) |
|---|---|---|---|---|---|---|
| 548 | 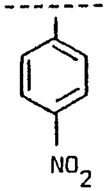 | 3 | H | 145 | $C_{10}H_{12}N_2O_6S$ = 288,28 | 19 |
| 562 | " | 5 | " | 130 | $C_{12}H_{16}N_2O_6S$ = 316,33 | 35 |
| 564 | " | 10 | " | 104 | $C_{17}H_{26}N_2O_6S$ = 386,47 | 51 |
| 674 | 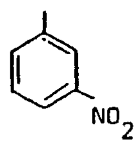 | 3 | " | 120 | $C_{10}H_{12}N_2O_6S$ = 288,28 | 42 |
| 675 | " | 5 | " | 115 | $C_{12}H_{16}N_2O_6S$ = 316,33 | 67 |
| 697 | " | 10 | " | 120 | $C_{17}H_{26}N_2O_6S$ = 386,47 | 72 |

| n° | Ar | n+m+1 | $R_7$ | F °C | Formule brute/PM | Rendement de la réaction (%) |
|---|---|---|---|---|---|---|
| 1 038 | 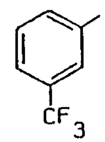 | 3 | H | 116 | $C_{11}H_{12}F_3NO_4S$ = 311,29 | 41 |
| 786 | 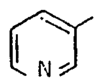 | 3 | " | 150 | $C_9H_{12}N_2O_4S$ = 244,28 | 38 |

Tableau II'

Préparation des composés nouveaux de formule :

$$Ar—SO_2—NH—(CH_2)_n—CH_2—(CH_2)_m—\underset{\underset{O}{\|}}{C}—OR_7$$

| n° | Ar | n+m+1 | $R_7$ | F °C | Formule brute/PM | Rendement de la réaction (%) |
|---|---|---|---|---|---|---|
| 1 038 | 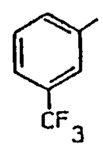 | 3 | H | 115 | $C_{11}H_{12}F_3NO_4S$ = 311 | 41 |
| 786 | 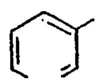 | 3 | " | 140 | $C_9H_{12}N_2O_4S$ = 244,23 | 38 |

Une variante du procédé décrit à l'exemple III consiste à verser du chlorure d'acide en solution dans un solvant organique inerte vis-à-vis du chlorure d'acide et non miscible avec l'eau, tel que le dioxanne, le benzène, le toluène, de préférence l'éther, sur l'amino-acide en solution aqueuse tel que la soude aqueuse ou la potasse aqueuse (réf. MAC CHESMY, SWAM, JACS, *59*, 1 116).

L'exemple suivant illustre ce mode de réalisation.

Exemple V — Préparation du composé n° 1 038

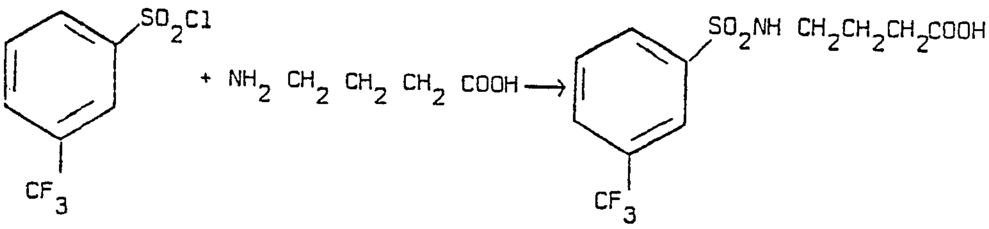

A | B | C
PM = 244,5 | PM = 103 | PM = 311

A 1,545 g (0,015 mole) d'acide amino-4 butyrique dans 30 $cm^3$ de soude N, est ajouté goutte à goutte sous agitation vigoureuse une solution de 3,7 g (0,015 mole) de trifluoro méthyl 3 benzène sulfochlorure dans 30 $cm^3$ d'éther éthylique.

Après 4 heures d'agitation, la phase organique est éliminée. La phase aqueuse est alors amenée à ph3 par de l'acide chlorhydrique 2N. Le précipité formé est essoré, lavé à l'eau jusqu'à absence d'ion $Cl^-$ puis séché.

Ont été préparés selon le procédé, les médicaments rassemblés dans le tableau III ci-après, ainsi que les composés nouveaux, rassemblés dans le tableau III' ci-après.

(Voir Tableaux pages suivantes)

Tableau III

| n° | Ar | n+m+1 | $R_7$ | F °C | Formule brute/PM | Rendement de la réaction (%) |
|---|---|---|---|---|---|---|
| 1 038 | 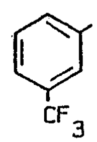 | 3 | H | 116 | $C_{11}H_{12}F_3NO_4S$ = 311,29 | 72 |
| 1 131 | " | 1 | " | 157 | $C_9H_8F_3NO_4S$ = 283,24 | 70 |
| 1 150 | 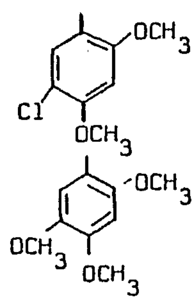 | 3 | " | 166 | $C_{12}H_{16}ClNO_6S$ = 337,8 | 65 |
| 1151 | | 3 | " | 177 | $C_{12}H_{19}NO_7S$ = 321,36 | 70 |
| | | | | | | 72 |

Tableau III'

| n° | Ar | n+m+1 | $R_7$ | F °C | Formule brute/PM | Rendement de la réaction (%) |
|---|---|---|---|---|---|---|
| 1 038 | 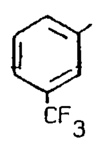 | 3 | H | 116 | $C_{11}H_{12}F_3NO_4S$ = 311,29 | 41 |
| 1 131 | " | 1 | H | 157 | $C_9H_8F_3NO_4S$ = 283,24 | 70 |
| 1 123 | " | 5 | H | 96 | $C_{13}H_{16}F_3NO_4S$ = 339,35 | 90 |

Un deuxième mode de préparation (PROCEDE IIB) des médicaments et des composés nouveaux, selon l'invention, de formule :

$$Ar - SO_2 - \underset{R_5}{N} - (CH_2)_n - \underset{R_6}{CH} - (CH_2)_m - COOH$$

consiste à fabriquer :

dans une première étape, un ester de l'acide que l'on veut obtenir, par l'un des deux procédés IA ou IIA qui ont été ci-dessus envisagés, pour la synthèse des médicaments et des composés nouveaux de formule :

$$Ar - SO_2 - \underset{R_5}{N} - (CH_2)_n - \underset{R_6}{CH} - (CH_2)_m - COOR_7$$

puis dans une deuxième étape, à hydrolyser l'ester obtenu en son acide correspondant selon le schéma :

$$Ar - SO_2 - \underset{R_5}{N} - (CH_2)_n - \underset{R_6}{CH} - (CH_2)_m - COOR_7 \longrightarrow$$

$$Ar - SO_2 - \underset{R_5}{N} - (CH_2)_n - \underset{R_6}{CH} - (CH_2)_m - COOH$$

D'un point de vue pratique, on opère de la façon suivante : $10^{-2}$ mole d'ester dans 50 $cm^3$ d'une base alcaline ou alcalino-terreuse en milieu alcoolique dont l'alcool correspondant à 1 à 4 atomes de carbone de concentration de 1 à 5N, telle que la soude éthanolique 1N à 5N, de préférence la soude éthanolique 2N, est agitée magnétiquement pendant 12 heures à température ambiante. Le milieu réactionnel est neutralisé à froid par un acide pour amener le pH à une valeur inférieure à 3, tel que l'acide chlorhydrique concentré de 2 à 10N, extrait par un solvant organique non miscible à l'eau, de préférence l'acétate d'éthyle. La phase organique est lavée à l'eau, séchée sur sulfate de sodium et concentrée sous pression réduite.

L'exemple suivant illustre le mode de préparation de médicaments et de composés nouveaux selon l'invention, préparation pour laquelle on a eu recours au procédé indiqué ci-dessus dans sa généralité.

Exemple VI — Préparation du composé n° 744

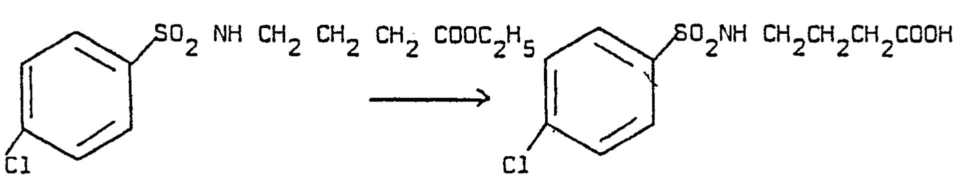

A 743 — PM = 305,5

B — PM = 277,5

L'ester A (120 g) est mis en suspension dans un mélange de soude 2N (400 $cm^3$) et d'éthanol (300 $cm^3$).

Après une nuit sous agitation magnétique à température ambiante, le milieu réactionnel est évaporé à sec sous vide, le résidu est repris dans 200 $cm^3$ eau, puis amené à ph 3 par addition d'acide chlorhydrique 2N. Le précipité est essoré, lavé avec de l'eau jusqu'à absence d'ion cl° puis séché sous vide. Le rendement est de 86 %.

Exemple VII — Préparation de l'acide (trifluorométhyl-3 benzènesulfonylamino)-6 hexanoïque

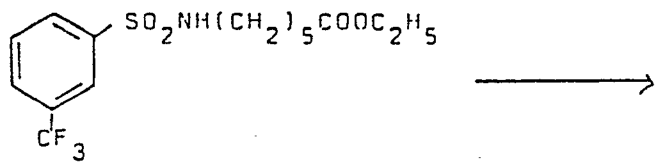

A

PM = 367,3

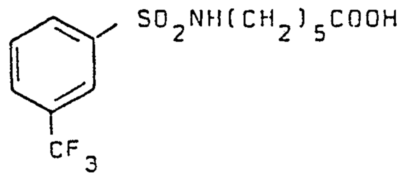

B

PM = 339,3

L'ester A ($2 \times 10^{-2}$ mole = 7,35 g) est mis en suspension dans un mélange de soude 2N (20 $cm^3$) et d'éthanol (10 $cm^3$).

Après une nuit, sous agitation magnétique, à température ambiante, le milieu réactionnel est évaporé à sec sous-vide.

Le résidu est repris dans 30 $cm^3$ d'eau, puis amené à un pH de 8 par addition d'acide chlorhydrique 2N. Le précipité est essoré, lavé avec de l'eau jusqu'à la disparition d'ions Cl, puis est séché sous-vide (rendement 80 %).

Ont été préparés, selon ce procédé, les médicaments suivants, réunis dans le tableau IV ci-après, ainsi que les composés nouveaux, réunis dans le tableau IV' ci-après.

(Voir Tableau IV pages suivantes)

Tableau IV

Préparation des médicaments de formule :

$Ar-SO_2-NH-(CH_2)_n-CH_2-(CH_2)_m-COOR_7$

| n° | Ar | n+m+1 | $R_7$ | F °C | Formule brute/PM | Rendement de la réaction (%) |
|---|---|---|---|---|---|---|
| 387 | 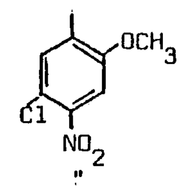 | 3 | H | 130 | $C_{11}H_{13}ClN_2O_7S$ = 352,75 | 99 |
| 789 | " | 4 | " | 95 | $C_{12}H_{15}ClN_2O_7S$ = 366,79 | 95 |
| 362 | " | 5 | " | 145 | $C_{13}H_{17}ClN_2O_7S$ = 380,80 | 96 |
| 854 | 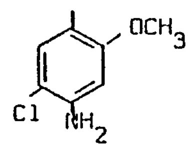 | 4 | " | 128 | $C_{12}H_{13}ClN_2O_5S$ = 332,76 | 85 |
| 738 | 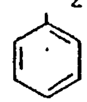 | 3 | " | 90 | $C_{10}H_{13}NO_4S$ = 243,29 | 99 |
| 751 | " | 5 | " | 121 | $C_{12}H_{17}NO_4S$ = 271,34 | 99 |
| 767 | " | 10 | " | 95 | $C_{17}H_{27}NO_4S$ = 341,48 | 95 |

| n° | Ar | n+m+1 | $R_7$ | F °C | Formule brute/PM | Rendement de la réaction (%) |
|---|---|---|---|---|---|---|
| 740 | 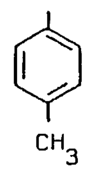 | 3 | H | 135 | $C_{11}H_{15}NO_4S$ = 257,32 | 94 |
| 753 | " | 5 | " | 111 | $C_{13}H_{19}NO_4S$ = 285,37 | 87 |
| 769 | " | 10 | " | 90 | $C_{18}H_{29}NO_4S$ = 355,50 | 95 |
| 744 | 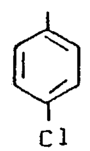 | 3 | " | 134 | $C_{10}H_{12}ClNO_4S$ = 277,74 | 97 |
| 757 | " | 5 | " | 130 | $C_{12}H_{16}ClNO_4S$ = 305,79 | 95 |
| 771 | " | 10 | " | 126 | $C_{17}H_{26}ClNO_4S$ = 375,93 | 89 |
| 1129 | 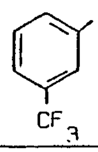 | 5 | " | 96 | $C_{13}H_{16}F_3NO_4S$ = 339,35 | 80 |

| n° | Ar | n+m+1 | $R_7$ | F °C | Formule brute/PM | Rendement de la réaction (%) |
|---|---|---|---|---|---|---|
| 742 | 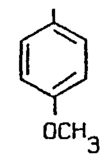 | 3 | H | 110 | $C_{11}H_{15}NO_5S$ = 273,32 | 99 |
| 755 | " | 5 | " | 89 | $C_{13}H_{19}NO_5S$ = 301,37 | 96 |
| 770 | " | 10 | " | 98 | $C_{18}H_{29}NO_5S$ = 371,50 | 96 |
| 746 | 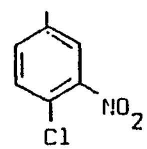 | 3 | " | 135 | $C_{10}H_{11}ClN_2O_6S$ = 322,74 | 91 |
| 759 | " | 5 | " | 119 | $C_{12}H_{15}ClN_2O_6S$ = 350,79 | 99 |
| 792 | " | 10 | " | 115 | $C_{17}H_{25}ClN_2O_6S$ = 420,93 | 86 |
| 800 | 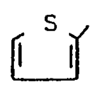 | 5 | " | 112 | $C_{10}H_{15}NO_4S_2$ = 277,37 | 80 |
| 823 | | 10 | " | 91 | $C_{15}H_{25}NO_4S_2$ = 347,51 | 90 |

| n° | Ar | n+m+1 | $R_7$ | F °C | Formule brute/PM | Rendement de la réaction (%) |
|---|---|---|---|---|---|---|
| 785 | 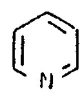 | 3 | H | 140 | $C_9H_{12}N_2O_4S$ = 244,28 | 85 |
| 804 | " | 5 | " | 124 | $C_{11}H_{16}N_2O_4S$ = 272,33 | 82 |
| 824 | " | 10 | " | 110 | $C_{16}H_{26}N_2O_4S$ = 342,47 | 91 |

Tableau IV'
Préparation des composés nouveaux de formule :

$Ar-SO_2-NH-(CH_2)_n-CH_2-(CH_2)_m-COOR_7$

| n° | Ar | n+m+1 | $R_7$ | F °C | Formule brute/PM | Rendement de la réaction (%) |
|---|---|---|---|---|---|---|
| 1 125 | 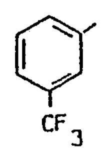 | 5 | H | 56 | $C_{13}H_{16}F_3NO_4S$ = 333,35 | 80 |
| 800 | 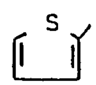 | 5 | " | 112 | $C_{10}H_{15}NO_4S_2$ = 277,37 | 80 |
| 823 | " | 10 | " | 91 | $C_{15}H_{25}NO_4S_2$ = 347,51 | 90 |
| 786 | 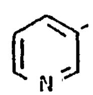 | 3 | H | 140 | $C_9H_{12}N_2O_4S$ = 244,28 | 85 |
| 804 | " | 5 | " | 124 | $C_{11}H_{16}N_2O_4S$ = 272,33 | 82 |
| 824 | " | 10 | " | 110 | $C_{16}H_{26}N_2O_4S$ = 342,47 | 91 |

Un mode de préparation des médicaments, selon l'invention, de formule :

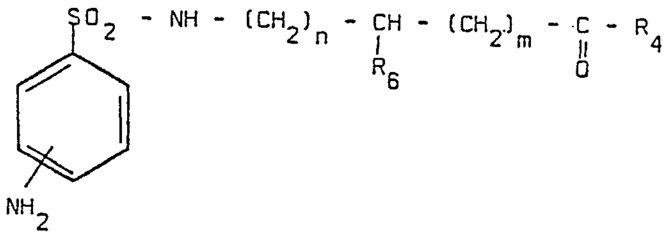

dans laquelle $R_4$ représente :

le groupe hydroxy,

le groupe $OR_7$, $R_7$ étant un radical alkyle de 1 à 6 atomes de carbone, consiste à utiliser, comme produit de départ, le médicament de formule :

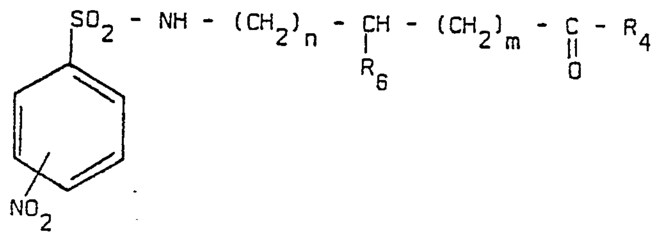

et à soumettre à une hydrogénation catalytique.

D'un point de vue pratique, on met l'ester ou l'acide en solution dans l'acétat d'éthyle que l'on agite de façon magnétique, sous atmosphère d'hydrogène, en présence de charbon palladié, la quantité du catalyseur étant de 5 % dans le cas où le médicament de départ est un acide et de 10 % lorsqu'il s'agit d'un ester. Au bout de quatre heures, l'absorption d'hydrogène est terminée, le catalyseur est filtré et le filtrat est concentré sous pression réduite.

L'exemple suivant illustre le mode de préparation de médicaments, selon l'invention, préparation pour laquelle on a recours au procédé ci-dessus indiqué dans sa généralité.

Exemple VIII — Préparation de l'acide ε(amino 4-méthoxy 2-benzènesulfonamido) caproïque

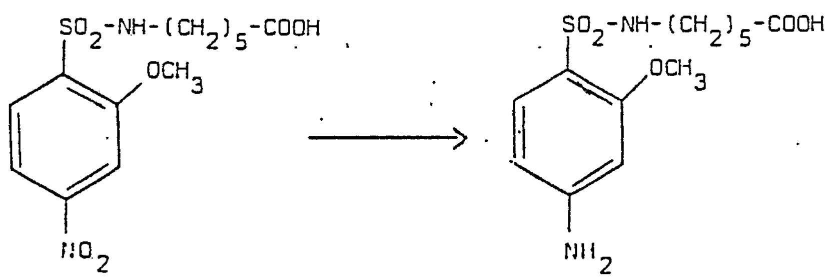

PM = 346,36 PM = 316,38

Une solution de l'acide ε(nitro 4-méthoxy 2-benzènesulfonamido)caproïque (1,4 g) en solution dans de l'acétate d'éthyle (150 $cm^3$) est agitée magnétiquement sous atmosphère d'hydrogène en présence de charbon palladié à 5 % (120 mg). Au bout de quatre heures, l'absorption d'hydrogène étant terminée, le catalyseur est filtré et le filtrat concentré sous pression réduite. On obtient l'acide ε(amino 4-méthoxy 2-benzènesulfonamido)caproïque pur, avec un rendement de 98 %.

Ont été préparés, en ayant recours au mode de préparation, les médicaments suivants réunis dans le tableau V ci-après.

(Voir Tableau V pages suivantes)

Tableau V

Préparation des médicaments de formule : 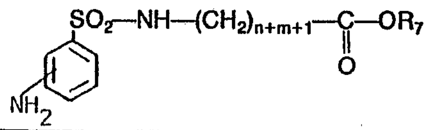

| n° | Ar | | n+m+1 | $R_7$ | F °C | Formule brute/PM | Rendement de la réaction (%) |
|---|---|---|---|---|---|---|---|
| 484 | 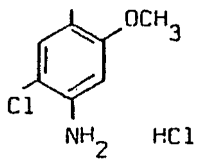 | HCl | 3 | $C_2H_5$ | 137 | $C_{13}H_{20}Cl_2N_2O_5S$ = 387,28 | 84 |
| 748 | " | | 3 | H | 127 | $C_{11}H_{15}ClN_2O_5S$ = 322,78 | 77 |
| 798 | " | TsOH | 4 | $C_2H_5$ | 164 | $C_{21}H_{29}ClN_2O_8S_2$ = 537,07 | 92 |
| 469 | " | HCl | 5 | " | 133 | $C_{15}H_{24}Cl_2N_2O_5S$ = 415,34 | 83 |
| 753 | " | | 5 | H | 92 | $C_{13}H_{19}ClN_2O_5S$ = 350,83 | 94 |
| 730 | " | TsOH | 7 | $C_2H_5$ | 124 | $C_{24}H_{35}ClN_2O_8S_2$ = 579,11 | 61 |
| 503 | " | | 10 | " | 132 | $C_{27}H_{41}ClN_2O_8S_2$ = 621,21 | 66 |
| 805 | " | | 10 | H | 100 | $C_{16}H_{29}ClN_2O_5S$ = 420,97 | 90 |

| n° | Ar | n+m+1 | $R_7$ | F °C | Formule brute/PM | Rendement de la réaction (%) |
|---|---|---|---|---|---|---|
| 656 | 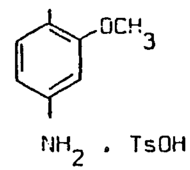 . TsOH | 3 | $C_2H_5$ | 187 | $C_{20}H_{28}N_2O_8S_2$ = 487,78 | 98 |
| 612 | " | 5 | " | 188 | $C_{22}H_{32}N_2O_8S_2$ = 516,63 | 57 |
| 694 | " | 5 | H | 116 | $C_{13}H_{20}N_2O_5S$ = 316,38 | 100 |
| 627 | " | 10 | $C_2H_5$ | 80 | $C_{20}H_{34}N_2O_5S$ = 414,57 | 100 |
| 668 | " | 10 | H | 105 | $C_{18}H_{30}N_2O_5S$ = 386,51 | 94 |
| 624 | 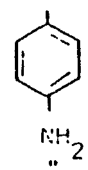 TsOH | 3 | $C_2H_5$ | 158 | $C_{19}H_{26}N_2O_7S_2$ = 458,55 | 99 |
| 609 | " | 5 | " | 86 | $C_{14}H_{22}N_2O_4S$ = 314,40 | 96 |
| 665 | " | 5 | H | 131 | $C_{12}H_{18}N_2O_4S$ = 286,35 | 97 |
| 626 | " | 10 | $C_2H_5$ | 85 | $C_{19}H_{32}N_2O_4S$ = 384,54 | 100 |

| n° | Ar | | n+m+1 | $R_7$ | F °C | Formule brute/PM | Rendement de la réaction (%) |
|---|---|---|---|---|---|---|---|
| 667 | 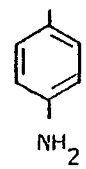 | , TsOH - | 10 | H | 118 | $C_{17}H_{28}N_2O_4S$ = 356,49 | 88 |
| 680 | 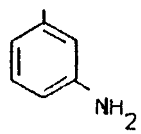 | | 3 | " | 99 | $C_{10}H_{14}N_2O_4S$ = 258,30 | 100 |
| 693 | " | TsOH | 3 | $C_2H_5$ | 144 | $C_{19}H_{26}N_2O_7S_2$ = 458,55 | 97 |
| 681 | " | | 5 | H | 86 | $C_{12}H_{18}N_2O_4S$ = 286,35 | 100 |
| 682 | " | TsOH | 5 | $C_2H_5$ | 109 | $C_{21}H_{30}N_2O_7S_2$ = 486,51 | 93 |
| 683 | " | | 10 | " | 56 | $C_{19}H_{32}N_2O_4S$ = 384,54 | 96 |
| 695 | " | | 10 | H | 105 | $C_{17}H_{28}N_2O_4S$ = 356,49 | 99 |

| n° | Ar | n+m+1 | $R_7$ | F °C | Formule brute/PM | Rendement de la réaction (%) |
|---|---|---|---|---|---|---|
| 747 | 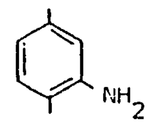 Cl , TsOH | 3 | $C_2H_5$ | 174 | $C_{19}H_{25}ClN_2O_7S_2$ = 493,01 | 57 |
| 785 | " | 3 | H | 71 | $C_{10}H_{13}ClN_2O_4S$ = 292,75 | 89 |
| 760 | " , TsOH | 5 | $C_2H_5$ | 147 | $C_{21}H_{29}ClN_2O_7S_2$ = 521,07 | 79 |
| 761 | " | 5 | H | 83 | $C_{12}H_{17}ClN_2O_4S$ = 320,81 | 80 |
| 773 | " , TsOH | 10 | $C_2H_5$ | 139 | $C_{26}H_{39}ClN_2O_7S_2$ = 591,20 | 80 |

Un mode de préparation des médicaments, et des composés nouveaux de formule :

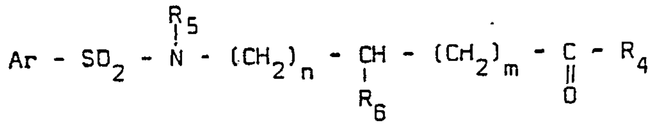

à partir de :

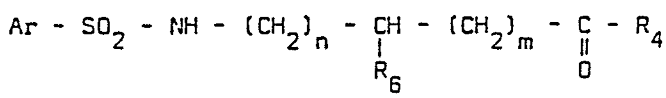

formules dans lesquelles $R_4$ représente OH ou un radical alcoxy de 1 à 6 atomes de carbone, — avec la condition, lorsqu'il s'agit des composés nouveaux, que lorsque Ar représente un benzène, celui-ci soit substitué par au moins un groupe $CF_3$—, consiste à faire réagir un halogénure d'alcoyle tel qu'un iodure d'alcoyle $R_5I$ selon le schéma suivant :

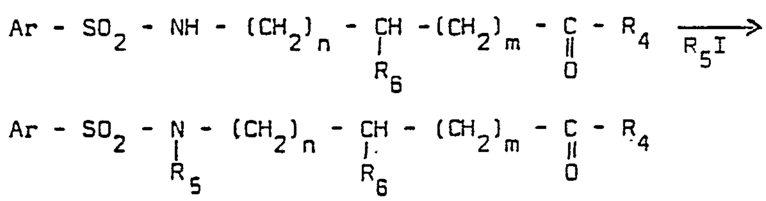


D'un point de vue pratique, on procède comme suit :

Le sulfonylamino acide (1 mM) est mis en solution par addition d'une base forte aqueuse en excès (soude, potasse). L'agent d'alcoylation peut être un halogénure d'alkyle ou d'aralkyle (l'halogénure étant un chlore, un brome ou iode) ou un sulfate d'alkyle (diméthylsulfate, diéthylsulfate).

Cet agent d'alcoylation est ajouté à froid, goutte à goutte, le pH de la réaction étant, si nécessaire, maintenu à une valeur supérieure ou égale à 10.

Après chauffage du milieu réactionnel de 3 h à 24 h, suivant l'agent et à une température comprise entre 60° et 100 °C. La phase aqueuse est extraite après refroidissement par un solvant non miscible (éther, TMF, benzène, acétate d'éthyle), de préférence l'éther.

La phase aqueuse est ensuite amenée à pH ≤ 3 par addition d'un acide minéral (acide chlorhydrique, bromhydrique, sulfurique), de préférence l'acide chlorhydrique.

Le précipité est essoré et recristallisé dans le solvant organique ou aqueux approprié.

L'exemple suivant illustre le mode de préparation de médicaments, préparation pour laquelle on a recours au procédé indiqué ci-dessus dans sa généralité.

Exemple IX — Préparation du composé n° 1 045

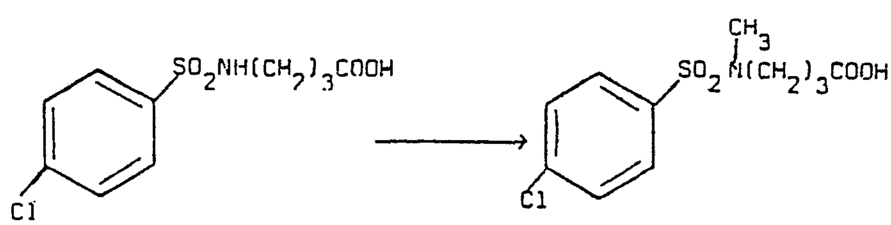

A B

PM = 277,5 PM = 201,5

A 6,2 g ($2.23.10^{-2}$ mole) du produit A dans 55 $cm^3$ d'une solution de soude N, 8 g d'iodure de méthyle sont ajoutés goutte à goutte sous agitation.

Le mélange réactionnel est porté pendant cinq heures à 80 °C. La phase aqueuse après refroidissement est extraite à l'éther. La phase organique est éliminée, la phase aqueuse est alors amenée à pH 3 par de l'acide chlorhydrique 2N. Le précipité fermé est essoré, lavé à l'eau puis séché. Après recristallisation dans un mélange benzène : 1, cyclohexane : 1, 5,85 g de cristaux fondant à 121° sont recueillis (Rdt 90 %).

Cette méthode d'alkylation a été appliquée à d'autres sulfonamido acides et a permis en particulier la préparation du composé n° 1044 : acide N-méthyl-nitro-3' benzènesulfonamido-4 butyrique.

Exemple X — Méthylation de l'acide (trifluorométhyl-3 benzènesulfonylamino)-4 butyrique (composé n° 1038)

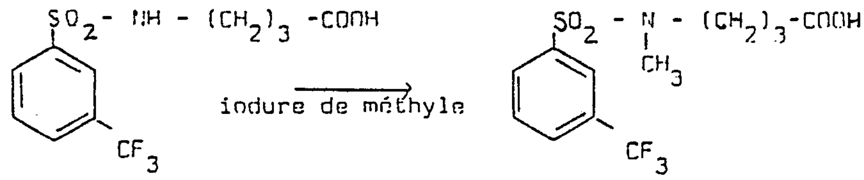


PM = 311 — composé n° 1 038

PM = 325 — composé n° 1 174

A 3,1 g d'acide (trifluorométhyl-3 benzènesulfonylamino)-4 butyrique ($1 \times 10^{-2}$ mole) dans 30 $cm^3$ de soude N, 4 g d'iodure de méthyle sont ajoutés goutte à goutte sous agitation.

Le mélange réactionnel est porté pendant 4 heures à 80 °C. La phase aqueuse après refroidissement est extraite à l'éther. La phase organique est éliminée.

La phase aqueuse est alors amenée à pH 3 par de l'acide chlorhydrique 2N.

Le précipité est essoré, lavé à l'eau puis séché.

Le point de fusion du composé obtenu (composé n° 1 174) est de 115 °C et le rendement est de 80 %.

Un mode de préparation des médicaments et des composés nouveaux de formule :

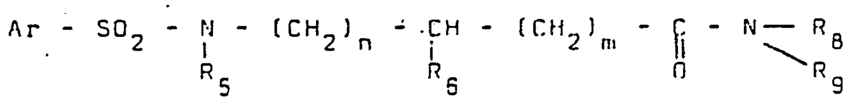

dans laquelle $R_5$, $R_6$, $R_8$ et $R_9$ ont les significations ci-dessus indiquées, à partir de :

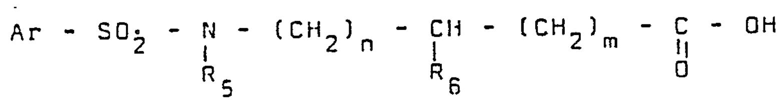

consiste à :

faire réagir le chlorure de thionyle sur l'acide suivant le schéma réactionnel :

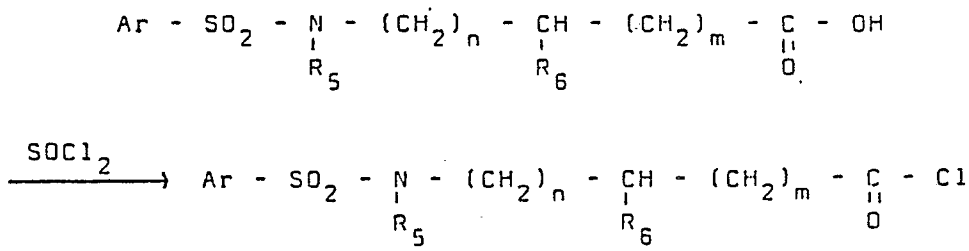


puis à faire réagir sur le chlorure d'acide, l'amine appropriée suivant le schéma réactionnel :

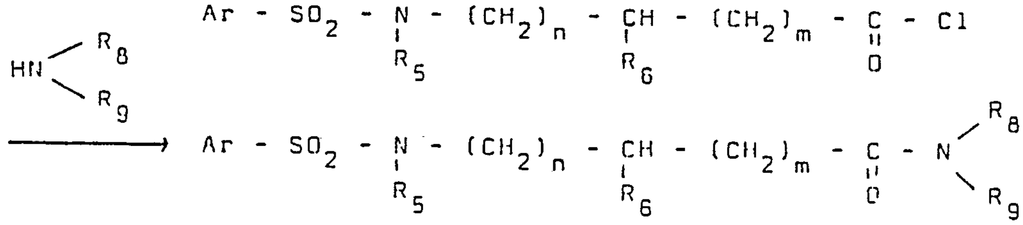

D'un point de vue pratique, on opère de la façon suivante.

On met l'acide en solution dans du benzène anhydre et on ajoute un excès de chlorure de thionyle. Après reflux, le mélange réactionnel est évaporé à sec et le résidu est repris dans l'amine appropriée.

Après chauffage entre 40° et 60 °C, le mélange réactionnel est repris dans l'acétate d'éthyle. La phase organique lavée à l'acide chlorhydrique 2N puis à l'eau, puis par une solution de carbonate de sodium, est évaporée pour donner un résidu cristallin correspondant à l'amide.

L'exemple suivant illustre le mode de préparation de médicaments, selon l'invention, préparation pour laquelle on a recours au procédé ci-dessus indiqué dans sa généralité.

Exemple XI — Préparation du composé n° 1 031

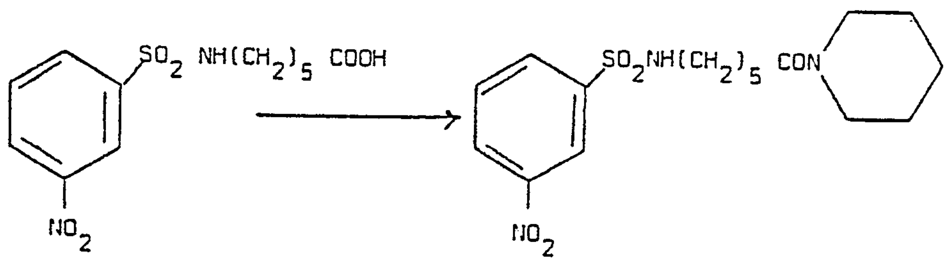

A

PM = 316

B

PM = 383

A 3,16 g (0,01 mole) de l'acide A dans 20 $cm^3$ de benzène anhydre est ajouté 0,85 ml (1,43 g soit 0,012 mole) de chlorure de thionyle distillé. Le milieu réactionnel est porté à 60 °C pendant cinq heures. Après évaporation à sec sous vide, le résidu est repris dans 10 $cm^3$ de benzène, puis 10 $cm^3$ de pipéridine fraîchement distillée sont ajoutés goutte à goutte. Le milieu réactionnel est alors porté à 70 °C pendant deux heures, puis évaporé à sec sous vide. Le résidu est dissous dans 150 $cm^3$ d'acétate d'éthyle. La phase organique obtenue lavée à l'eau, puis à l'acide chlorhydrique N, puis à l'eau, est séchée sur sulfate de sodium. Les cristaux obtenus par évaporation sont recristallisés dans le benzène (3 g, Rdt : 78 %).

Ont été préparés selon l'exemple XI, les composés nouveaux ainsi que les médicaments suivants rassemblés dans le tableau VI ci-après.

Dans ce cas, les médicaments selon l'invention sont tous des composés nouveaux.

(Voir Tableau VI pages suivantes)

Tableau VI

| n° | Ar | n+m+1 | $R_4$ | F °C | Formule brute/PM | Rendement de la réaction (%) |
|---|---|---|---|---|---|---|
| 1 029 | 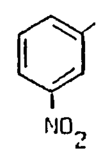 | 5 | 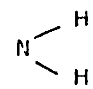 | 103 | $C_{12}H_{17}N_3O_5S$ = 315,36 | 48 |
| 1 030 | " | 5 | 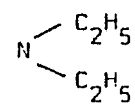 | 54 | $C_{16}H_{25}N_3O_5S$ = 371,47 | 54 |
| 1 031 | " | 5 | 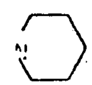 | 80 | $C_{17}H_{25}N_3O_5S$ = 383,48 | 78 |
| 1 032 | " | 5 | 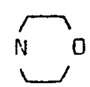 | 80 | $C_{16}H_{23}N_3O_6S$ = 385,45 | 52 |
| 1 130 | 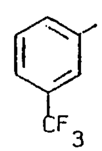 | 5 | 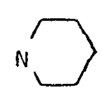 | 102 | $C_{18}H_{25}F_2N_2O_3S$ = 406,48 | 73 |

Tableau VI (suite)

| n° | Ar | n+m+1 | R4 | F °C | Formule brute/PM | Rendement de la réaction (%) |
|---|---|---|---|---|---|---|
| 1 168 | 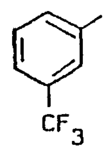 | 5 | 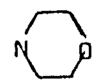 | 120 | $C_{17}H_{23}F_3N_2O_4S$ = 408,45 | 70 |
| 1 170 | " | 5 | 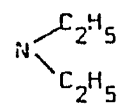 | 66 | $C_{17}H_{25}F_3N_2O_3S$ = 394,45 | 72 |
| 1 171 | " | 5 | 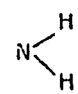 | 104 | $C_{13}H_{17}F_3N_2O_3S$ = 338,36 | 66 |
| 1 176 | " | 3 | 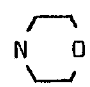 | 91 | $C_{15}H_{19}F_3N_2O_4S$ = 380,39 | 70 |
| 1 177 | " | 3 | 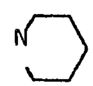 | 95 | $C_{16}H_{21}F_3N_2O_3S$ = 378,48 | 72 |
| 1 178 | " | 3 | 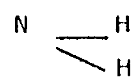 | 108 | $C_{11}H_{13}F_3N_2O_3S$ = 319,30 | 60 |

Les composés chimiques ci-dessus définis, selon l'invention, et leurs sels physiologiquement acceptables, peuvent entrer, à titre de substances actives, dans la préparation de médicaments présentant un ensemble de propriétés pharmacologiques et thérapeutiques de grande valeur.

Parmi les sels physiologiquement acceptables des composés selon l'invention, on peut notamment citer :

en ce qui concerne les sels formés à partir d'un acide :

— les sels alcalins ou alcalino-terreux ;

— ou des sels de base organique tels que le méglumate, acéglumate ;

en ce qui concerne les anions :

— les chlorhydrates, bromhydrates, sulfates, phosphates, méthanesulfonates, tosylates, tartrates, acétates, fumarates, le succinate, le pyruvate, phénoxyacétate et chlofibrate.

Les sels des médicaments selon l'invention sont :

1) soit les sels formés à partir de la salification du groupement carboxylique —COOH dans les composés de formule :

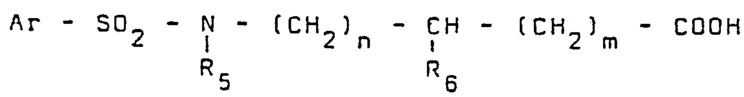

2) soit les sels formés à partir de la salification par une base du groupement —NH dans les composés de formule :

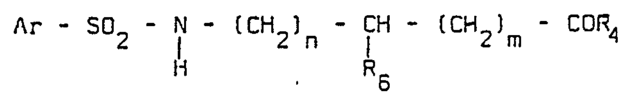

3) soit les sels formés à partir de la salification du radical

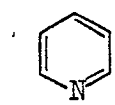

dans les composés de formule :

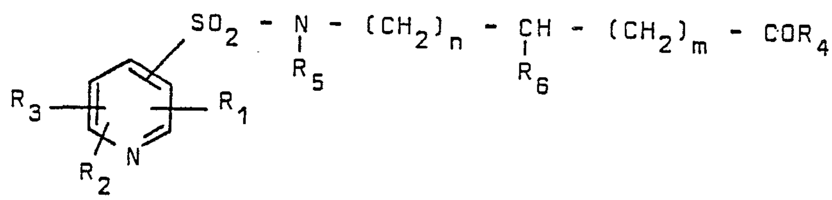

4) soit les sels formés à partir des composés, de la salification du groupement amino substituant du noyau aromatique dans les composés de formule :

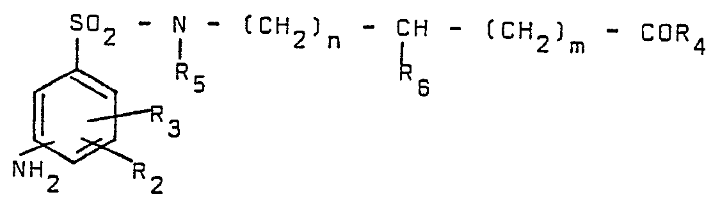

Dans le premier cas, on passe du composé non salifié au sel en faisant réagir en quantité équimolaire et de façon connue en soi la base et le produit choisis.

Dans le deuxième cas, on passe du composé non salifié au sel en faisant réagir en quantité équimolaire et de façon connue en soi la base et le produit choisis.

Dans le troisième et le quatrième cas, on passe du composé non salifié au sel en faisant réagir en quantité équimolaire et de façon connue en soi l'acide et le produit choisis.

Les médicaments selon l'invention présentent des propriétés remarquables dans le domaine des troubles de la lipidémie.

## Activité normolipémiante

1) Test au Triton

Dans une première série d'expériences, des produits ont été testés sur une hyperlipidémie provoquée par l'administration de Triton selon le protocole suivant.

Les essais ont été réalisés sur des groupes de 5 rats Wistar mâles dont le poids varie de 150 à 175 g.

Les produits testés sont administrés par voie orale, sous forme de solution aqueuse à 3 % de gomme adragante à raison de 1 ml pour 100 g de poids du corps, en quantité correspondant à 250 mg/kg.

Pour effectuer ces essais, on provoque une hyperlipidémie chez les animaux, au moyen du produit commercialisé par la société Rohm et Haas sous le nom de Triton W. R.

Chez les rats à jeun, l'injection intraveineuse du Triton entraîne une hyperlipidémie particulièrement nette pour ce qui est des triglycérides et de façon moins importante pour ce qui est des lipides totaux.

On observe, sur cette hyperlipidémie, les effets obtenus par administration des substances actives selon l'invention.

Les essais sont réalisés comme suit :

les rats sont mis à jeuner pendant 18 heures, puis on administre le Triton W. R. par voie intraveineuse en solution aqueuse à 10 % à raison de 200 mg/kg ;

on administre simultanément par voie orale 250 mg/kg du produit à tester ;

6 heures et/ou 24 heures plus tard, on effectue les dosages sur le sang de façon traditionnelle.

Les résultats de ces essais figurent dans le tableau VII de la page suivante.

Les résultats sont exprimés en pourcentage de baisse provoqué à tester par rapport au témoin Triton ayant entraîné une hyperlipidémie, ce pourcentage de baisse étant respectivement relatif :

au cholestérol total,

aux lipides totaux,

aux triglycérides.

Parmi les produits testés, les produits présentant des propriétés pharmacologiques remarquables vis-à-vis de la normolipémie sont ceux appartenant aux groupes définis ci-dessus, suivants :

$B_1$, $E_1$, $E_2$, $G_1$, $G_2$, $H_1$, $H_2$, K, L, M, N, $S_1$, $S_2$, T, Y, Z.

Les médicaments particulièrement préférés sont rassemblés dans le tableau VIII.

Tableau VII : Activité normolipémiante

| | % baisse par rapport au témoin Triton | | |
|---|---|---|---|
| | Cholestérol total | Lipides totaux | Triglycérides |
| 362 | - 10 | - 39 | - 88 |
| 380 | - 19 | - 31 | - 86 |
| 387 | (*) | - 39 | - 90 |
| 469 | - 3 | - 30 | - 28 |
| 472 | | - 9 | |
| 484 | | - 41 | |
| 486 | | - 66 | - 21 |
| 501 | - 24 | - 17 | - 18 |
| 502 | - 16 | - 45 | - 32 |
| 503 | | - 26 | - 18 |
| 504 | - 45 | | |
| 505 | | - 48 | - 17 |
| 508 | - 8 | - 17 | |
| 562 | | - 6 | |
| 563 | - 8 | - 27 | |
| 564 | | - 37 | - 15 |
| 608 | - 19 | - 15 | |

* L'absence de valeur indique que l'effet est nul ou que le lot témoin n'a pas répondu normalement à l'administration de Triton.

(Tableau VII Suite)

| | % baisse par rapport au témoin Triton | | |
|---|---|---|---|
| | Cholestérol total | Lipides totaux | Triglycérides |
| 609 | - 40 | | - 2 |
| 610 | - 20 | - 2 | |
| 611 | - 29 | - 15 | |
| 612 | - 1 | - 48 | - 13 |
| 624 | - 18 | | |
| 625 | | | - 5 |
| 626 | - 18 | | |
| 656 | - 14 | - 3 | - 29 |
| 660 | - 42 | - 36 | - 90 |
| 661 | - 37 | | - 86 |
| 665 | - 15 | - 30 | - 20 |
| 666 | | - 20 | |
| 667 | - 7 | - 45 | |
| 668 | - 27 | - 20 | - 29 |
| 675 | - 19 | - 45 | - 27 |
| 676 | - 10 | - 43 | - 24 |
| 679 | - 30 | - 61 | - 34 |
| 680 | | - 46 | - 16 |
| 681 | - 32 | - 51 | - 33 |
| 682 | | - 22 | - 34 |
| 683 | | - 19 | - 52 |
| 693 | - 31 | - 18 | - 45 |
| 694 | - 32 | - 31 | - 65 |
| 695 | - 24 | - 20 | - 27 |
| 696 | - 56 | - 46 | - 59 |
| 697 | - 46 | - 35 | - 50 |
| 728 | | - 32 | - 53 |
| 729 | | | - 17 |
| 730 | | | - 10 |
| 786 | - 36 | - 24 | - 17 |
| 800 | - 13 | - 33 | - 5 |
| 823 | - 41 | - 25 | |

Tableau VIII
Médicaments préférés

| Composé n° | Normolip. | Composé n° | Normolip |
|---|---|---|---|
| 362 | + | 679 | +++ |
| 380 | + | 680 | + |
| 387 | + | 681 | +++ |
| 503 | | 682 | + |
| 563 | + | 683 | + |
| 612 | | 693 | + |
| 660 | +++ | 694 | ++ |
| 661 | + | 696 | ++++ |
| 667 | + | 697 | ++ |
| 668 | ++++ | 728 | ++ |
| 675 | +++ | 786 | + |
| 676 | | | |

2) Régime athéromateux

Dans une deuxième série expérimentale, on a testé l'activité de certains produits de l'invention sur des animaux ayant reçu pendant une durée prolongée un régime athéromateux. L'essai s'effectue sur des rats Wistar femelles, d'un poids d'environ 160-180 g. Les animaux reçoivent une alimentation provenant de l'usine d'alimentation nationale de Villemoisson-sur-Orge, France, et désignée sous la référence UAR A03, enrichie en cholestérol (0,5 %) et en acide cholique (0,5 %). Après 15 jours de régimes athéromateux et tout en maintenant les animaux sous régime athéromateux, on leur administre par voie orale pendant 4 jours 250 000 ui/kg de vitamine $D_2$ commercialisée sous le nom de Stérogyl 25H, en solution dans de l'huile d'olive. On administre ensuite les produits à étudier pendant 4 semaines, dès la fin de l'administration de l'agent lésionnel. En fin d'étude, on effectue sur tous les animaux des examens biochimiques [cholestérol total, cholesterol high density lipoprotein (HDL) et cholesterol low density lipoprotein (LDL), et triglycérides].

Autopsie et étude histologique

En fin d'étude, tous les animaux encore vivants sont sacrifiés, et l'on prélève divers organes dont particulièrement l'aorte, pour l'étude histologique de cette dernière. Deux critères sont étudiés : le nombre de zones athéromateuses et l'intensité des lésions.

Le calcul des cotations s'effectue de la façon suivante :

en ce qui concerne l'intensité des lésions, on effectue une cotation allant de 0 pour l'absence de lésion à 5 + pour les lésions les plus fortes, et ceci pour chaque type d'altération considéré (augmentation des espaces interlamellaires, interruption des lames élastiques, épaississement des lames élastiques, dépôt de calcium, dépôt de mucopolysaccharides) ; puis on fait l'addition de la totalité des résultats par lot d'animaux ;

en ce qui concerne le nombre de zones athéromateuses, par comptage de ces dernières et addition de la totalité des chiffres obtenus par lot d'animaux.

On calcule ensuite le pourcentage de diminution par rapport aux témoins régime et le score, c'est-à-dire le rapport de l'intensité de l'activité du produit testé vis-à-vis de celle de l'activité du clofibrate, compté pour 1.

Le tableau IX donne les résultats détaillés des examens biochimiques :

a) en % de diminution par rapport au témoin régime ;

b) en score par rapport au produit de référence, le clofibrate, ce dernier étant compté pour 1.

Le tableau X donne le résultat des examens macroscopiques :

a) en % de diminution des animaux atteints de lésions ;

b) en score par rapport au clofibrate de référence, dont le score est compté pour 1.

Le tableau XI donne les résultats microscopiques avec cotation histologique concernant l'intensité des lésions, et le nombre de zones athéromateuses.

(Voir Tableaux pages suivantes)

Tableau IX

Examens biochimiques

| LOT | CHOLESTEROL T % | CHOLESTEROL T Score | CHOLESTEROL HDL % | CHOLESTEROL HDL Score | CHOLESTEROL LDL % | CHOLESTEROL LDL Score | TRIGLYCERIDES % | TRIGLYCERIDES Score |
|---|---|---|---|---|---|---|---|---|
| TEM REGIME | | | | | | | | |
| CLOFIBRATE | - 48,75 | 1,00 | + 104,17 | 1,00 | - 55,37 | 1,00 | - 24,14 | 1,00 |
| 679 | - 53,13 | 1,09 | + 58,33 | 0,56 | - 60,14 | 1,09 | 0 | 0 |
| 786 | - 54,22 | 1,11 | - 20,83 | - | - 57,07 | 1,03 | - 17,24 | 0,71 |
| 1038 | - 51,88 | 1,06 | + 83,33 | 0,80 | - 58,26 | 1,05 | - 20,69 | 0,86 |

Tableau X
Examens macroscopiques

| LOT | ANIMAUX ATTEINTS | | |
|---|---|---|---|
| | % | % DE DIMINUTION / TEM-REGIME | SCORE / CLOFIBRATE |
| TEM-TEM | 0 | - | - |
| TEM-REGIME | 100 | - | - |
| CLOFIBRATE | 81,82 | 18,18 | 1,00 |
| 679 | 92,86 | 7,14 | 0,39 |
| 786 | 93,33 | 6,67 | 0,37 |
| 1038 | 66,67 | 33,33 | 1,83 |

Tableau XI

Observations microscopiques

| LOT | COTATION HISTOLOGIQUE % | COTATION HISTOLOGIQUE % DE DIMINUTION TEM-REGIME | COTATION HISTOLOGIQUE SCORE CLOFIBRATE | NOMBRE DE ZONES ATHEROMATEUSES % | NOMBRE DE ZONES ATHEROMATEUSES % DE DIMINUTION TEM-REGIME | NOMBRE DE ZONES ATHEROMATEUSES SCORE CLOFIBRATE |
|---|---|---|---|---|---|---|
| TEM-TEM | | - | - | | - | - |
| TEM-REGIME | 132 | - | - | 160 | - | - |
| CLOFIBRATE | 49 | 62,83 | 1,00 | 72 | 54,93 | 1,00 |
| 679 | 79 | 39,97 | 0,64 | 82 | 48,57 | 0,88 |
| 786 (*) | | | | | | |
| 1038 | 44 | 66,31 | 1,06 | 35 | 78,18 | 1,42 |

(*) Non examiné du point de vue histologique

Toxicité

La toxicité aiguë est exprimée par la dose léthale 50, c'est-à-dire la dose entraînant la mort de 50 % des animaux.

Cette étude a été effectuée sur des souris, auxquelles on a administré les produits à tester par voie intrapéritonéale.

Les essais effectués ont montré que la dose léthale 50 des substances actives était très élevée. Elle s'établit comme suit, pour les substances suivantes :

— 362 : la dose létale 50 (mg/kg) est = 1 760
— 387 : la dose létale 50 (mg/kg) est = 3 070
— 502 : la dose létale 50 (mg/kg) est = 3 070
— 665 : la dose létale 50 (mg/kg) est > 6 400
— 675 : la dose létale 50 (mg/kg) est = 930
— 676 : la dose létale 50 (mg/kg) est > 6 400
— 729 : la dose létale 50 (mg/kg) est > 6 400

L'étude de la toxicité pour le composé n° 1 038 a été effectuée par voie orale, sur des rats et des souris.

Les résultats concernant la dose léthale 50 ($DL_{50}$), la dose léthale 0 ($DL_0$) et la dose léthale 100 ($DL_{100}$), exprimés en g/kg, sont rassemblés dans le tableau ci-après.

| | Mâles. | Femelles |
|---|---|---|
| Rats | | |
| $DL_{50}$ | 13 | 15,5 |
| $DL_0$ | 5 | 5 |
| $DL_{100}$ | 20 | > 20 |
| Souris | | |
| $DL_{50}$ | 6 | 6,6 |
| $DL_0$ | 4 | 5 |
| $DL_{100}$ | 9 | 9 |

Ces résultats montrent que le composé n° 1 038 n'est pas toxique.

Les médicaments selon l'invention sont avantageusement utilisés à titre de principe actif dans le traitement de certaines maladies du métabolisme des lipides.

Les médicaments selon l'invention sont dépourvus de toxicité.

Le rapport dose active/dose toxique peut être comparé favorablement à celui des substances connues présentant des propriétés de même nature.

A titre d'exemple, les essais de toxicité subaiguë effectués sur le composé n° 1 038, pendant une durée d'environ un mois, aux doses de 150, 500 et 1 000 mg/kg, montrent que l'indice thérapeutique de ce composé est très satisfaisant.

En plus des substances actives constituées par les composés de formule (I), les médicaments selon l'invention peuvent contenir d'autres substances actives compatibles avec les premières.

Par exemple, les composés entrant dans la constitution des médicaments selon l'invention peuvent être associés avec des normolipémiants du type biguanide, tels que la 1,1-Diméthylbiguanide ou la 1-butylbiguanide, ou avec des normolipémiants dérivés d'acide nicotinique.

Dans ces médicaments selon l'invention, les substances actives sont associées, dans la mesure où cela est nécessaire, avec des excipients et adjuvants traditionnels destinés à faciliter et à améliorer leur utilisation, leur conservation, etc.

En particulier, les substances actives sont associées aux excipients solides ou liquides facilitant leur administration en fonction de la voie d'introduction.

Compte tenu de leurs activités et des traitements pour lequels ils sont utilisés, les médicaments selon l'invention peuvent être administrés par voie parentérale, de préférence lorsque la substance active est sous forme de sel. A cet effet, ils sont présentés sous forme de solutions stériles ou stérilisables, injectables ou propres à être utilisées, pour la préparation extemporanée de solutions injectables. Ces solutions peuvent être présentées sous forme de solutions aqueuses physiologiques, notamment

isotoniques d'un de ces composés, telles que les solutions isotoniques salines ou glucosées, les exemples ne présentant bien entendu aucun caractère limitatif quant à la définition des produits physiologiquement acceptables pouvant être utilisés pour former des solutions isotoniques injectables.

Les médicaments selon l'invention peuvent également être administrables par d'autres voies, notamment de suppositoires ou par voie orale. Administrés par voie orale, ils sont présentés sous des formes très variées : comprimés, dragées, capsules, gélules, poudres, solutions, suspensions, sirops, ou par voie topique telle que crème, pommade, lotion, gel, etc.

Dans des présentations pharmaceutiques pour administration par voie parentérale, la dose de produit à administrer, par kilogramme de poids du malade, est comprise d'environ 0,5 à environ 25 mg.

Par voie orale, la dose unitaire est comprise d'environ 10 mg à environ 500 mg, de préférence de 50 mg à 250 mg.

Par voie topique, les présentations comportent de 1 à 20 % en poids de la préparation.

**Revendications** (pour les Etats contractants : BE, CH, DE, GB, IT, LI, LU, NL, SE)

1. Composés nouveaux utiles pour la préparation de médicaments, ainsi que leurs isomères optiques correspondants, caractérisés en ce qu'ils répondent à la formule :

$$Ar - SO_2 - \underset{R_5}{N} - (CH_2)_n - \underset{R_6}{CH} - (CH_2)_m - \underset{O}{\overset{}{C}} - R_4$$

dans laquelle :

Ar représente l'un des cycles suivants :

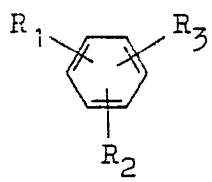
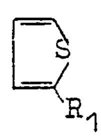
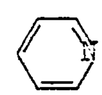

$R_1$, $R_2$, $R_3$ sont identiques ou différents et représentent un atome d'hydrogène, un atome d'halogène, le radical $NO_2$, le radical $NH_2$, le radical $CF_3$, un groupe alkyle ayant de 1 à 6 et de préférence de 1 à 4 atomes de carbone, un groupe alcoxy ayant de 1 à 6 et de préférence de 1 à 4 atomes de carbone, un groupe acide, un groupe ester ayant de 2 à 7 et de préférence de 2 à 5 atomes de carbone :

la somme n + m + 1 est comprise de 3 à 11 et de préférence égale à 3,5 ou 10, cette somme pouvant également être égale à 1 ou 2, lorsque Ar contient un noyau benzénique dont l'un au moins des substituants $R_1$, $R_2$ et $R_3$ est un groupe $CF_3$ ;

$R_5$ et $R_6$ représentent, indépendamment l'un de l'autre, un atome d'hydrogène, un radical alcoyle ayant de 1 à 6 atomes de carbone, de préférence de 1 à 4 atomes de carbone, un radical aralcoyle ayant de 7 à 9 atomes de carbone ;

$R_4$ représente soit un groupe hydroxy,

— soit un radical $OR_7$ dans lequel $R_7$ est un groupe alkyle ayant de 1 à 6 atomes de carbone et de préférence de 1 à 4 atomes de carbone,

— soit un radical

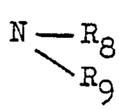

dans lequel $R_8$ et $R_9$, identiques ou différents, représentent un atome d'hydrogène, un groupe alkyle ayant de 1 à 6 atomes de carbone et de préférence de 1 à 4 atomes de carbone, ou conjointement forment avec l'azote un hétérocycle azoté à 5 ou 6 chaînons notamment un groupe pipéridino, morpholino, pyrrolidino, pyrrole ou pyrroline, sous réserve que lorsque Ar contient un noyau benzénique et que $R_4$ représente un groupe hydroxy ou le radical $OR_7$, l'un au moins des trois substituants $R_1$, $R_2$ ou $R_3$ représente le radical $CF_3$.

2. Composés selon la revendication 1, caractérisés en ce que l'un au moins des radicaux $R_5$ ou $R_6$ représente l'hydrogène.

3. Composés selon l'une quelconque des revendications 1 ou 2, de formule :

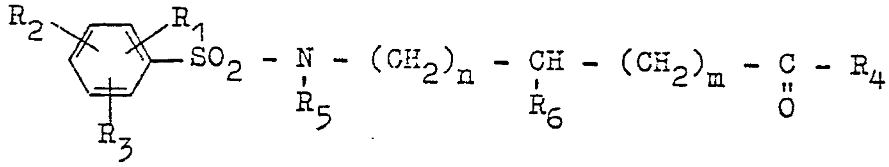

dans laquelle :

l'un au moins des substituants $R_1$, $R_2$, $R_3$ représente un radical $CF_3$ ;

$R_4$ représente :

— le groupe hydroxy ;

— le radical $OR_7$ dans lequel $R_7$ est un groupe alkyle ayant de 1 à 6 atomes de carbone, de préférence de 1 à 4 atomes de carbone ;

— le radical

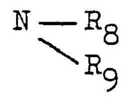

dans lequel $R_8$ et $R_9$, identiques ou différents, représentent un atome d'hydrogène, un groupe alkyle ayant de 1 à 6 atomes de carbone et de préférence de 1 à 4 atomes de carbone, ou conjoinement forment avec l'azote un hétérocycle azoté à 5 ou 6 chaînons, notamment un groupe pipéridino, morpholino, pyrrolidino, pyrrole ou pyrroline ;

n + m + 1 est compris de 1 à 11, étant de préférence égal à 3,5 ou 10.

4. Composés selon l'une quelconque des revendications 1 à 3, caractérisés en ce qu'ils répondent à la formule :

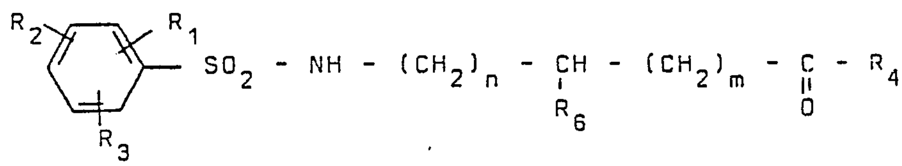

dans laquelle l'un au moins des substituants $R_1$, $R_2$, $R_3$ représente $CF_3$ ;

$R_4$ représente :

— le groupe hydroxy,

— le radical $OR_7$ dans lequel $R_7$ est un groupe alkyle ayant de 1 à 6 atomes de carbone, de préférence de carbone ;

— le radical

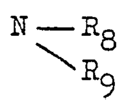

dans lequel $R_8$ et $R_9$, identiques ou différents, représentent un atome d'hydrogène, un groupe alkyle ayant de 1 à 6 atomes de carbone, de préférence de 1 à 4 atomes de carbone ; ou conjointement forment avec l'azote un hétérocycle azoté à 5 ou 6 chaînons, notamment un groupe pipéridino, morpholino, pyrrolidino, pyrrole ou pyrroline ;

et n + m + 1 est compris de 1 à 11, étant de préférence égal à 3, 5 ou 10.

5. Composés selon l'une quelconque des revendications 1 à 4, caractérisés en ce qu'ils répondent à la formule :

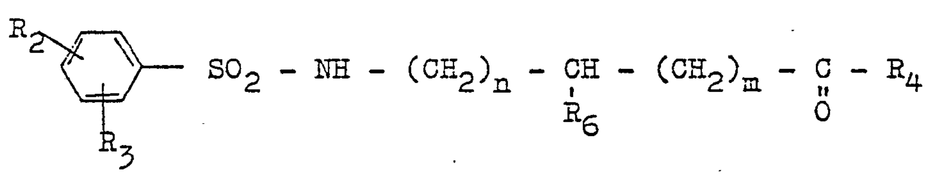

dans laquelle l'un au moins des substituants $R_2$ ou $R_3$ représente le radical $CF_3$ ;

$R_4$ représente :

— le groupe hydroxy,

— le radical $OR_7$ dans lequel $R_7$ est un groupe alkyle ayant de 1 à 6 atomes de carbone et de préférence de 1 à 4 atomes de carbone ;

— le radical

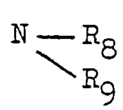

dans lequel $R_8$ et $R_9$ sont identiques ou différents et représentent un atome d'hydrogène, un groupe

alkyle ayant de 1 à 6 atomes de carbone, de préférence de 1 à 4 atomes de carbone ; ou conjointement forment avec l'azote un hétérocycle azoté à 5 ou 6 chaînons, notamment un groupe pipéridino, morpholino, pyrrolidino, pyrrole ou pyrroline ;

et n + m + 1 est compris de 1 à 11, étant de préférence égal à 3,5 ou 10.

6. Composés selon l'une quelconque des revendications 1 à 5, caractérisés en ce qu'ils répondent à la formule :

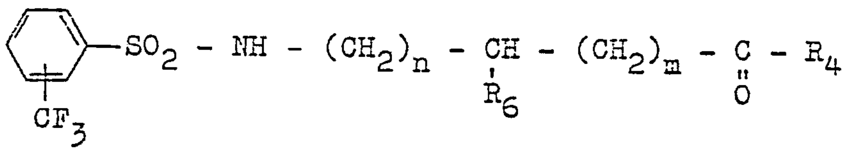

dans laquelle $R_4$ représente :

— le groupe hydroxy,

— le radical $OR_7$ dans lequel $R_7$ est un groupe alkyle ayant de 1 à 6 atomes de carbone, de préférence de 1 à 4 atomes de carbone,

— le radical

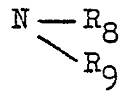

dans lequel

$R_8$ et $R_9$ sont identiques ou différents et représentent un atome d'hydrogène, un groupe alkyle ayant de 1 à 6 atomes de carbone, de préférence de 1 à 4 atomes de carbone, ou conjointement forment avec l'azote un hétérocycle azoté à 5 ou 6 chaînons, notamment un groupe pipéridino, morpholino, pyrrolidino, pyrrole ou pyrroline ;

et n + m + 1 est compris de 1 à 11, étant de préférence égal à 3,5 ou 10.

7. Composés selon la revendication 1, caractérisés en ce que Ar représente le cycle de formule :.

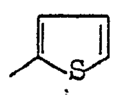

dans lequel le carbone en position alpha du $SO_2$ peut être éventuellement substitué par un atome d'halogène, notamment le chlore ou par un radical méthyle.

8. Composés selon la revendication 1, caractérisés en ce que Ar représente le cycle de formule :

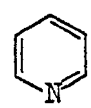

ce cycle pouvant éventuellement être substitué par $CF_3$.

9. Composés selon la revendication 1, caractérisés en ce qu'ils répondent à la formule :

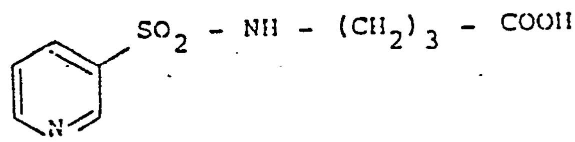

10. Composés selon la revendication 1, caractérisés en ce qu'ils répondent à la formule :

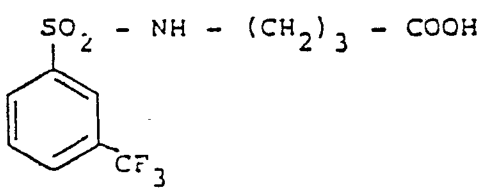

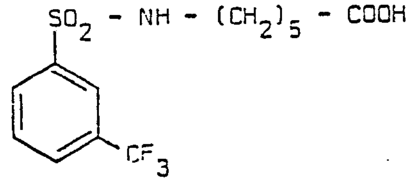

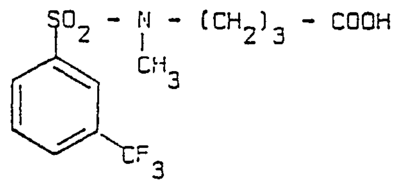

11. Composés selon la revendication 1, caractérisés en ce qu'ils répondent à la formule :

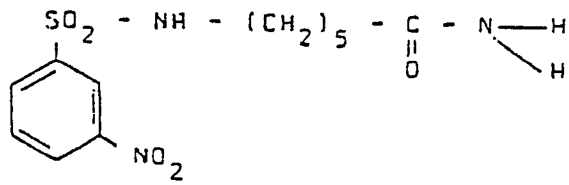

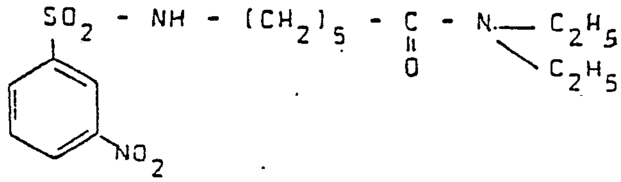

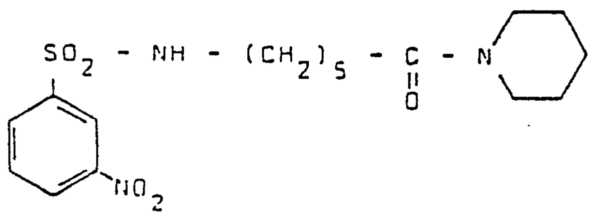

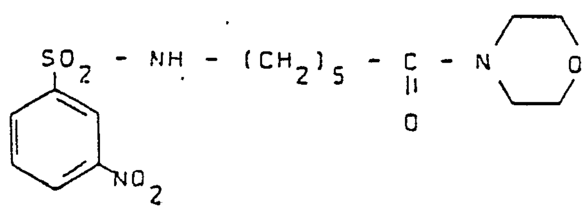

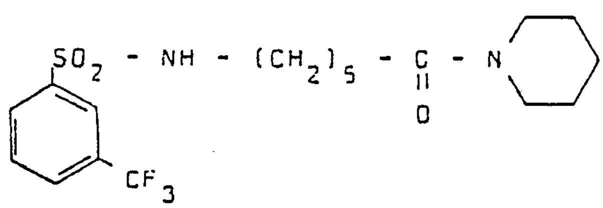

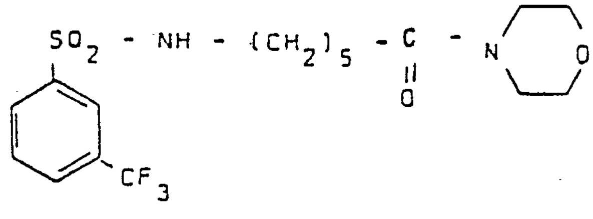

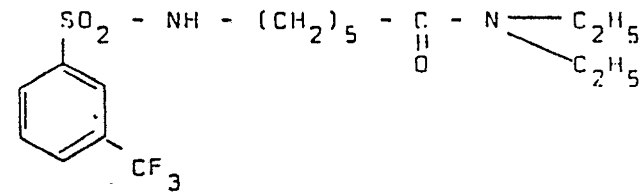

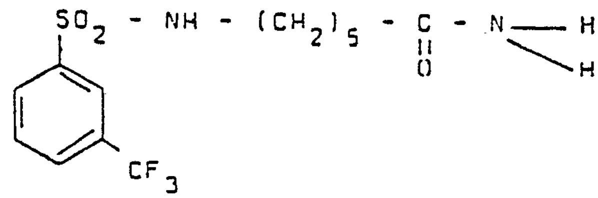

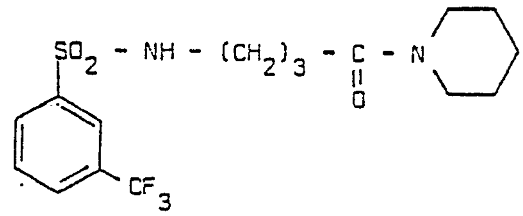

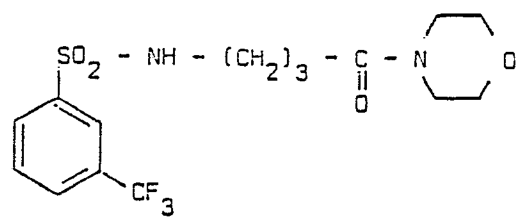

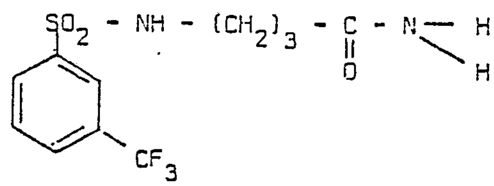

12. Médicaments caractérisés en ce qu'ils contiennent à titre de substance active, au moins un composé selon l'une quelconque des revendications 1 à 11.

13. Médicament présentant des propriétés normolipémiantes, caractérisé en ce qu'il contient à titre de substance active au moins un composé (ou son isomère optique correspondant) de type arylsulfonamide répondant à la formule :

$$Ar - SO_2 - \underset{R_5}{N} - (CH_2)_n - \underset{R_6}{CH} - (CH_2)_m - \underset{O}{\overset{}{C}} - R_4 \qquad (I)$$

dans laquelle :

Ar représente l'un des cycles suivants :

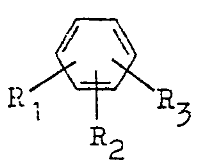

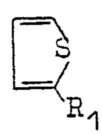

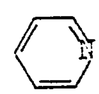

les substituants $R_1$, $R_2$, $R_3$ sont identiques ou différents et représentent un atome d'hydrogène, un atome d'halogène, le radical $NO_2$, le radical $NH_2$, $CF_3$, un groupe alkyle ayant de 1 à 6 atomes de carbone, de préférence de 1 à 4 atomes de carbone, un groupe alcoxy ayant de 1 à 6 atomes de carbone et de préférence de 1 à 4 atomes de carbone, un groupe acide, un groupe ester ayant de 2 à 7 atomes de carbone et de préférence de 2 à 5 atomes de carbone ;

la somme n + m + 1 est comprise de 3 à 11 et de préférence égale à 3, 5 ou 10, cette somme pouvant également être égale à 1 ou 2, lorsque Ar contient un noyau benzénique dont l'un au moins des substituants $R_1$, $R_2$ et $R_3$ est un groupe $CF_3$ ;

$R_5$ et $R_6$ représentent indépendamment l'un de l'autre un aotme d'hydrogène, un radical alcoyle ayant de 1 à 6 atomes de carbone, de préférence de 1 à 4 atomes de carbone, un radical aralcoyle ayant de 7 à 9 atomes de carbone ;

$R_4$ représente le groupe hydroxy,

— le radical $OR_7$ dans lequel $R_7$ est un groupe alkyle ayant de 1 à 6 atomes de carbone, et de préférence de 1 à 4 atomes de carbone ;

— le radical

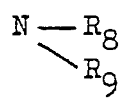

dans lequel $R_8$ et $R_9$, identiques ou différents, représentent un atome d'hydrogène, un groupe alkyle ayant de 1 à 6 atomes de carbone, et de préférence de 1 à 4 atomes de carbone, ou conjointement forment avec l'azote un hétérocycle azoté à 5 ou 6 chaînons, notamment un groupe pipéridino, morpholino, pyrrolidino, pyrrole ou pyrroline.

14. Médicament selon la revendication 13, caractérisé en ce que l'un au moins des radicaux $R_5$ ou $R_6$ représentent l'hydrogène.

15. Médicament selon l'une quelconque des revendications 13 ou 14, caractérisé en ce que le noyau benzénique substitué a pour formule :

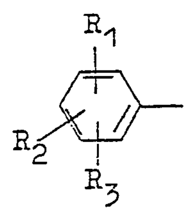

dans lequel $R_1$, $R_2$, $R_3$ représentent simultanément un atome d'hydrogène, ou sont simultanément différents de l'hydrogène et sont choisis dans le groupe constitué par un atome d'halogène, le radical $NO_2$, le radical $NH_2$, $CF_3$, un groupe alkyle ayant de 1 à 6 atomes de carbone, et de préférence de 1 à 4 atomes de carbone, un groupe alcoxy ayant de 1 à 6 atomes de carbone, et de préférence de 1 à 4 atomes de carbone, un groupe acide, un groupe ester ayant de 2 à 7 atomes de carbone, et de préférence de 2 à 5 atomes de carbone.

16. Médicament selon la revendication 15, caractérisé en ce que $R_1$, $R_2$ et $R_3$ représentent simultanément un atome d'hydrogène.

17. Médicament selon la revendication 15 caractérisé en ce qu'il répond à la formule (III) suivante :

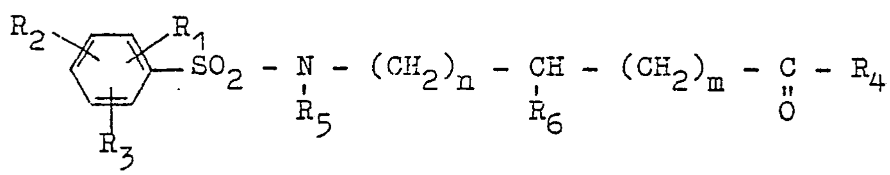 (III)

dans laquelle $R_1$, $R_2$ et $R_3$ sont simultanément différents de l'hydrogène.

18. Médicament selon la revendication 17, caractérisé en ce que le noyau benzénique est trisubstitué par les radicaux choisis dans le groupe comprenant $NO_2$, $NH_2$, $OCH_3$, $CH_3$, un atome d'halogène, et notamment le chlore.

19. Médicament selon l'une quelconque des revendications 13 à 15, caractérisé en ce qu'il répond à la formule (III) dans laquelle :

$R_1$ représente un atome d'hydrogène,

$R_2$ et $R_3$ représentent un atome d'halogène, le radical $NO_2$, le radical $NH_2$, un groupe alkyle ayant de 1 à 6 atomes de carbone et de préférence de 1 à 4 atomes de carbone, un groupe alcoxy ayant de 1 à 6 atomes de carbone et de préférence de 1 à 4 atomes de carbone, un groupe acide, un groupe ester ayant de 2 à 7 atomes de carbone, et de préférence de 2 à 5 atomes de carbone.

20. Médicament selon la revendication 19, caractérisé en ce que le noyau benzénique est substitué par les radicaux choisis dans le groupe constitué par $NO_2$, $NH_2$, $OCH_3$ ou par un halogène, notamment le chlore.

21. Médicament selon l'une quelconque des revendications 13 à 15, caractérisé en ce qu'il répond à la formule (III) dans laquelle :

$R_1$ et $R_2$ représentent simultanément un atome d'hydrogène ;

$R_3$ représente un atome d'halogène, le radical $NO_2$, le radical $NH_2$, un groupe alkyle ayant de 1 à 6 atomes de carbone, et de préférence de 1 à 4 atomes de carbone, un groupe alcoxy ayant de 1 à 6 atomes de carbone et de préférence de 1 à 4 atomes de carbone, un groupe acide, un groupe ester ayant de 2 à 7 atomes de carbone, et de préférence de 2 à 5 atomes de carbone.

22. Médicament selon la revendication 13, caractérisé en ce que Ar représente le cycle de formule :

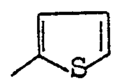

dans lequel le carbone en position alpha du $SO_2$ peut être éventuellement substitué par un atome d'halogène, notamment le chlore ou par un radical méthyle.

23. Médicament selon la revendication 13, caractérisé en ce que Ar représente le cycle de formule :

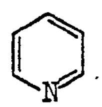

ce cycle pouvant éventuellement être substitué par $CF_3$.

24. Médicament selon la revendication 13, caractérisé en ce qu'il comprend à titre de substance active un des composés de formule :

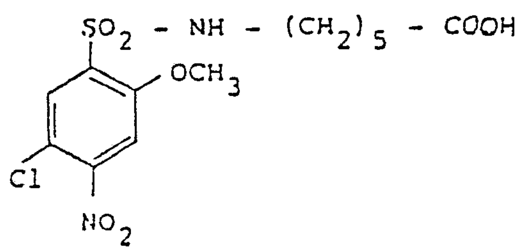

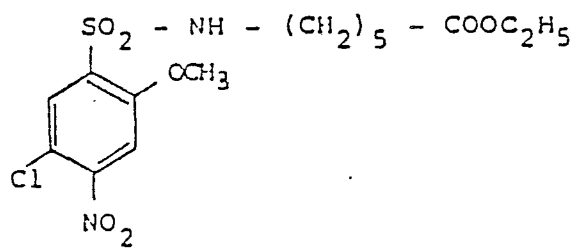

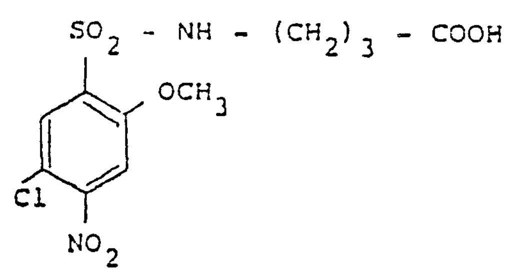

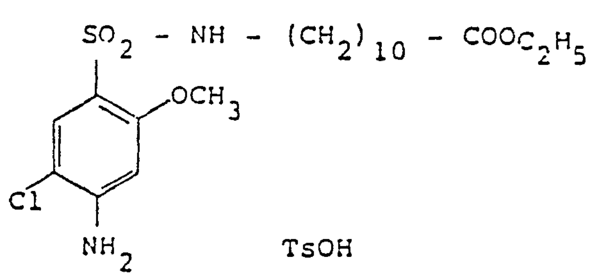

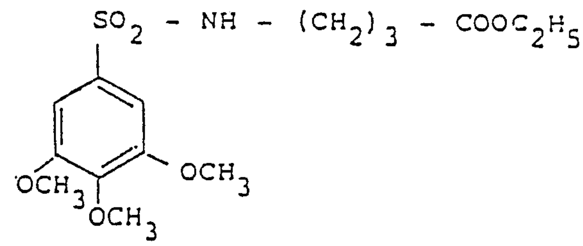

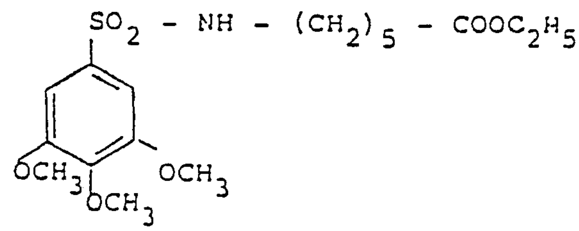

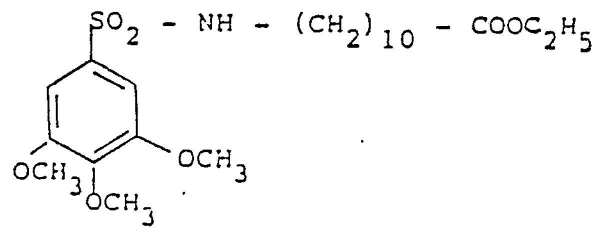

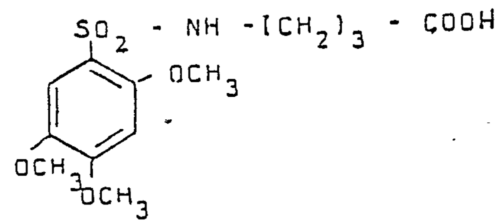

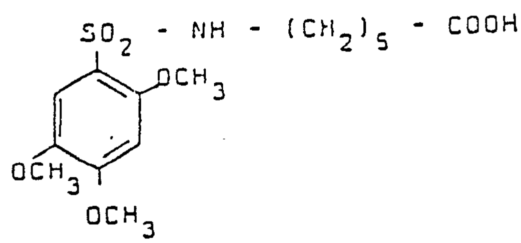

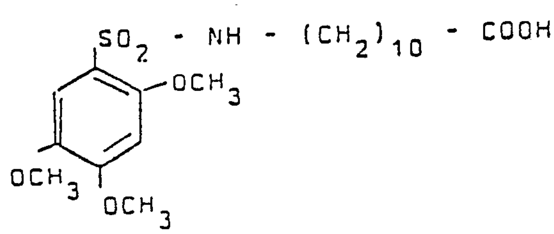

25. Médicament selon la revendication 13, caractérisé en ce qu'il comprend à titre de substance active un des composés de formule :

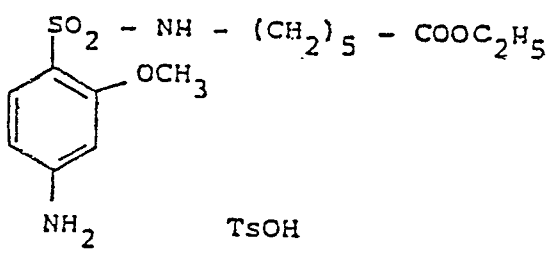

TsOH

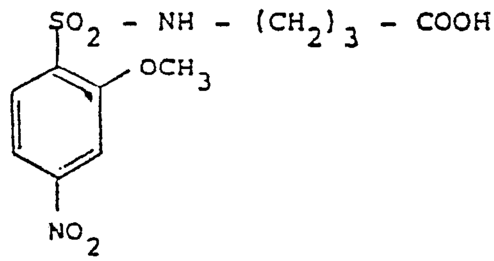

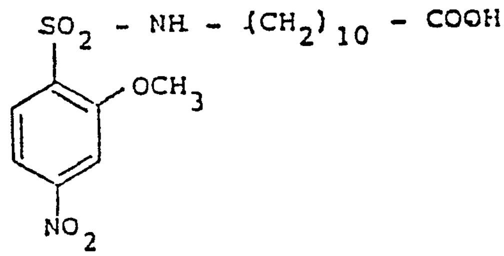

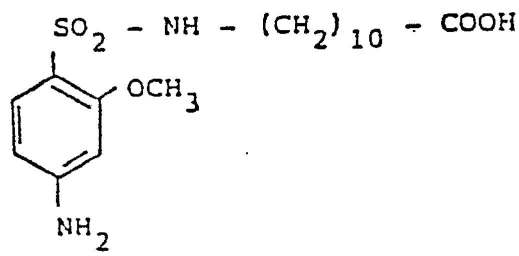

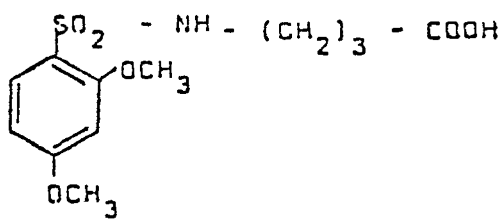

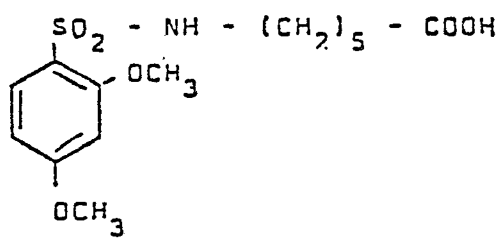

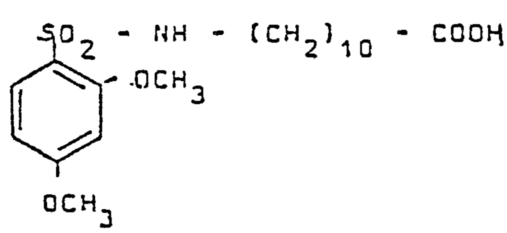

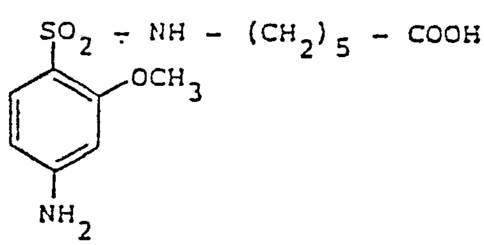

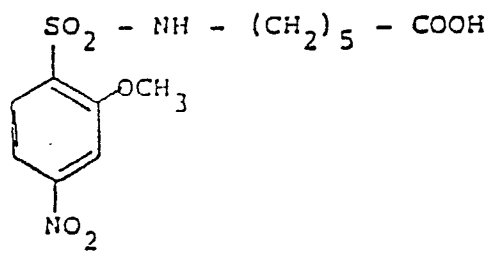

26. Médicament selon la revendication 13, caractérisé en ce qu'il comprend à titre de substance active un des composés de formule :

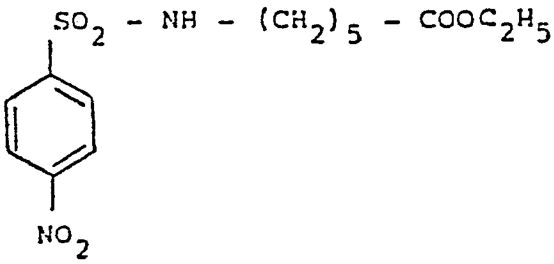

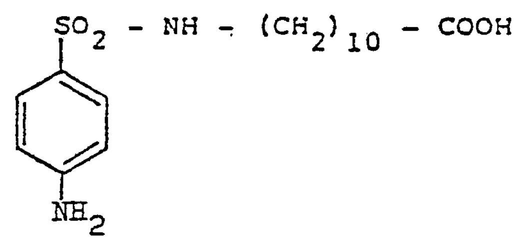

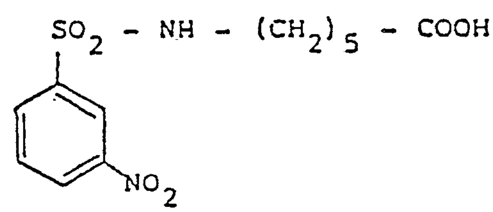

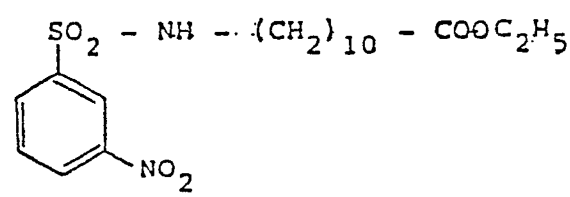

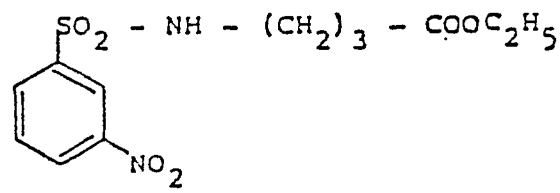

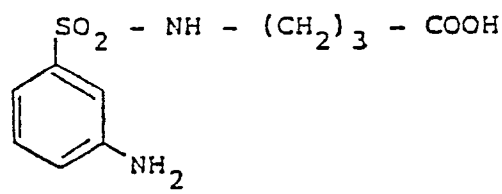

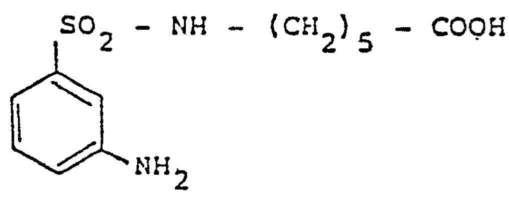

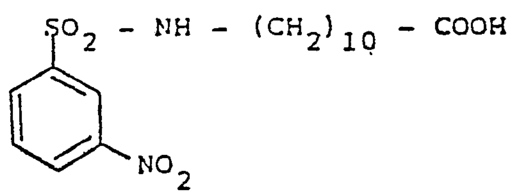

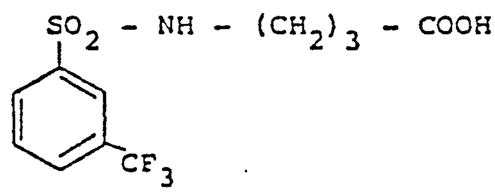

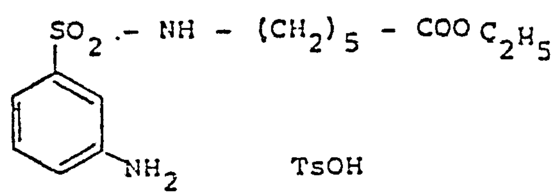

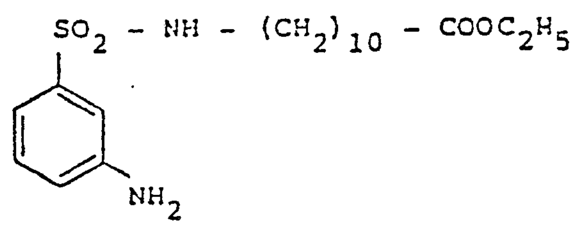

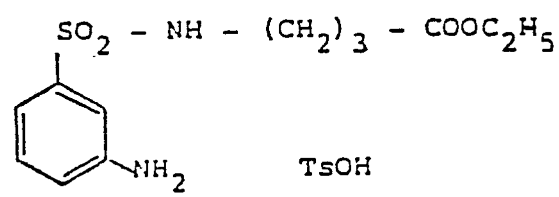

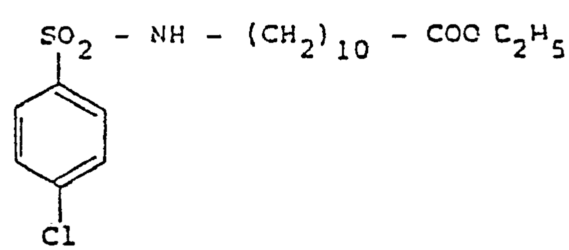

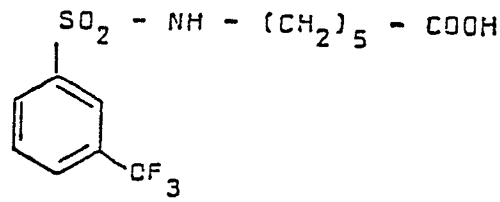

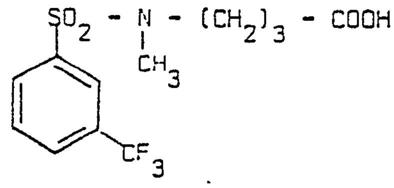

27. Médicament selon la revendication 13, caractérisé en ce qu'il comprend à titre de substance active un composé de formule :

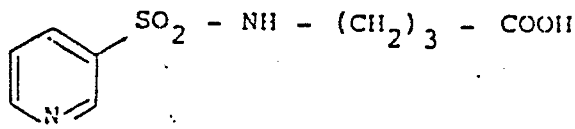

28. Médicament selon la revendication 13, caractérisé en ce qu'il comprend à titre de substance active un des composés de formule :

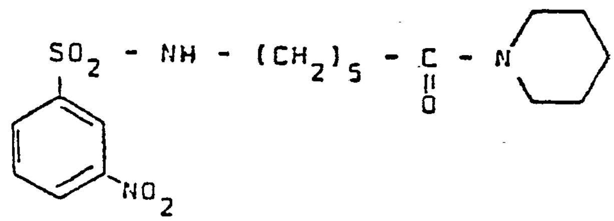

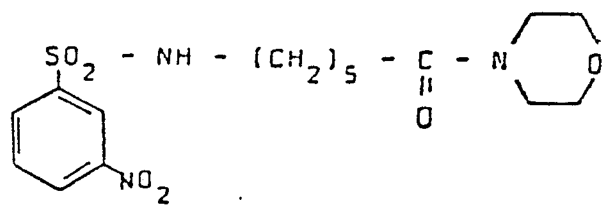

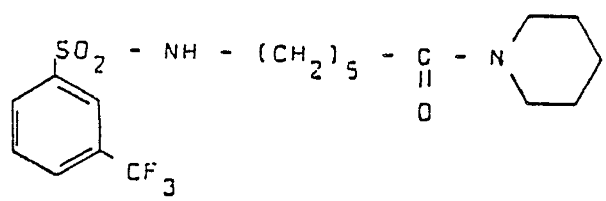

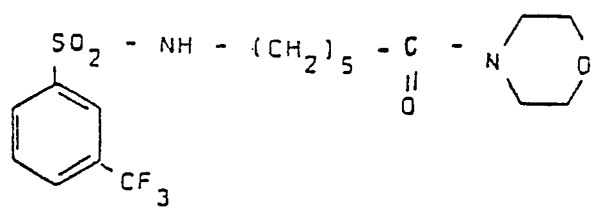

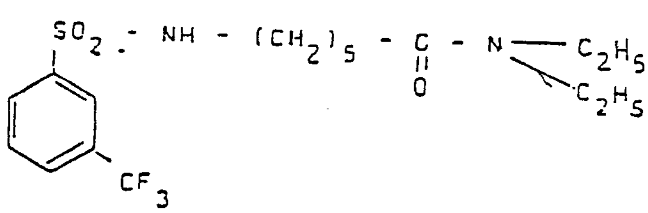

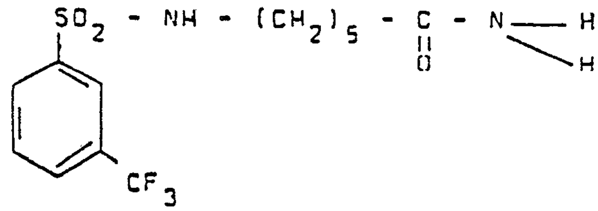

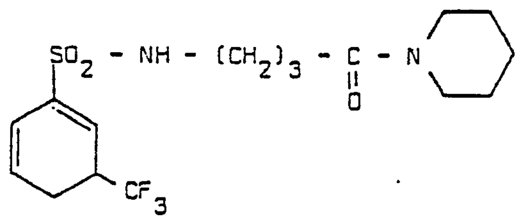

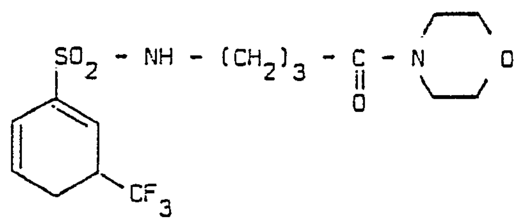

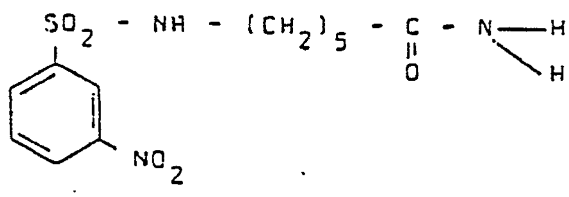

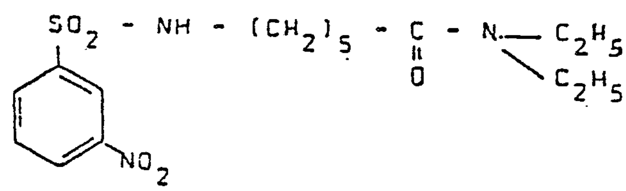

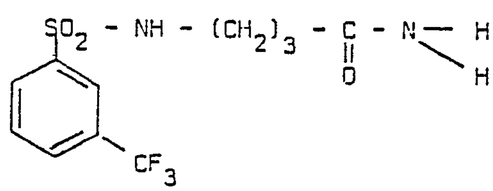

29. Sels pharmaceutiquement acceptables, notamment tosylate et chlorhydrate, des composés et médicaments selon l'une quelconque des revendications 1 à 28.

30. Médicament selon l'une quelconque des revendications 12 à 29, pour administration par voie orale, caractérisé en ce qu'il contient d'environ 10 mg à environ 500 mg, de préférence d'environ 50 à 250 mg de substance active.

31. Médicament selon l'une quelconque des revendications 12 à 29, pour administration par voie topique, caractérisé en ce que la préparation contient de 1 à 20 % en poids de substance active.

**Revendications** (pour l'Etat contractant AT)

1. Procédé de préparation de composés utiles pour la préparation de médicaments, de formule :

$$Ar - SO_2 - \underset{R_5}{\underset{|}{N}} - (CH_2)_n - \underset{R_6}{\underset{|}{CH}} - (CH_2)_m - \underset{O}{\underset{\|}{C}} - R_4 \qquad (I)$$

ainsi que les isomères optiques des composés de formule (I) dans laquelle :

Ar représente l'un des cycles suivants :

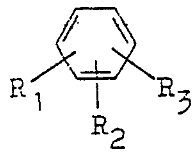

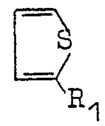

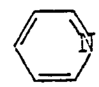

les substituants $R_1$, $R_2$, $R_3$ sont identiques ou différents et représentent un atome d'hydrogène, un atome d'halogène, le radical $NO_2$, le radical $NH_2$, $CF_3$, un groupe alcoyle ayant de 1 à 6 atomes de carbone, de préférence de 1 à 4 atomes de carbone, un groupe alcoxy ayant de 1 à 6 atomes de carbone et de préférence de 1 à 4 atomes de carbone, un groupe acide, un groupe ester ayant de 2 à 7 atomes de carbone et de préférence de 2 à 5 atomes de carbone ;

la somme n + m + 1 est comprise de 3 à 11 et de préférence égale à 3, 5 ou 10, cette somme pouvant également être égale à 1 ou 2, lorsque Ar contient un noyau benzénique dont l'un au moins des substituants $R_1$, $R_2$ et $R_3$ est un groupe $CF_3$ ;

$R_5$ et $R_6$ représentent indépendamment l'un de l'autre un atome d'hydrogène, un radical alcoyle ayant de 1 à 6 atomes de carbone, de préférence de 1 à 4 atomes de carbone, un radical aralcoyle ayant de 7 à 9 atomes de carbone ;

$R_4$ représente :

— le groupe hydroxy,

— le radical $OR_7$ dans lequel $R_7$ est un groupe alcoyle ayant de 1 à 6 atomes de carbone, et de préférence de 1 à 4 atomes de carbone ;

— le radical

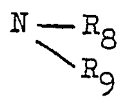

dans lequel $R_8$ et $R_9$, identiques ou différents représentent un atome d'hydrogène, un groupe alcoyle ayant de 1 à 6 atomes de carbone, et de préférence de 1 à 4 atomes de carbone, ou conjointement forment avec l'azote un hétérocycle azoté à 5 ou 6 chaînons, notamment un groupe pipéridino, morpholino, pyrrolidino, pyrrole ou pyrroline ; caractérisé en ce que :

lorsque $R_4$ représente le radical $OR_7$, $R_7$ étant un groupe alcoyle de 1 à 6 atomes de carbone :

— soit on fait réagir un halogénure, notamment un chlorure d'acide de formule :

$$ArSO_2Cl$$

sur un aminoester de formule :

$$\underset{R_5}{HN} - (CH_2)_n - \underset{R_6}{CH} - (CH_2)_m - \underset{O}{\overset{}{C}} - OR_7$$

ou son chlorhydrate de formule :

$$\underset{R_5}{HN} - (CH_2)_n - \underset{R_6}{CH} - (CH_2)_m - \underset{O}{C} - OR_7\ ,\ HCl$$

pour obtenir le composé de formule :

$$Ar - SO_2 - \underset{R_5}{N} - (CH_2)_n - \underset{R_6}{CH} - (CH_2)_m - \underset{O}{C} - OR_7$$

— soit on fait réagir un halogénure, notamment un chlorure d'acide de formule :

$$ArSO_2Cl$$

sur un aminoacide de formule :

$$H\underset{R_5}{N} - (CH_2)_n - \underset{R_6}{CH} - (CH_2)_m - \underset{O}{\overset{}{\underset{\|}{C}}} - OH$$

puis on estérifie l'acide ainsi obtenu, en le faisant réagir sur un alcool approprié de formule : $R_7OH$, pour obtenir le composé de formule :

$$Ar - SO_2 - \underset{R_5}{N} - (CH_2)_n - \underset{R_6}{CH} - (CH_2)_m - \underset{O}{\underset{\|}{C}} - OR_7$$

— soit on fait réagir un acide de formule :

$$ArSO_2H$$

sur un aminoester de formule :

$$\underset{R_5}{HN} - (CH_2)_n - \underset{R_6}{CH} - (CH_2)_m - \underset{O}{\underset{\|}{C}} - OR_7$$

pour obtenir le composé de formule :

$$Ar - SO_2 - \underset{R_5}{N} - (CH_2)_n - \underset{R_6}{CH} - (CH_2)_m - \underset{O}{\underset{\|}{C}} - OR_7$$

lorsque $R_4$ représente le radical hydroxy :
— soit on fait réagir un chlorure d'acide :

$$ArSO_2Cl$$

sur un aminoacide de formule :

$$\underset{R_5}{HN} - (CH_2)_n - \underset{R_6}{CH} - (CH_2)_m - COOH$$

pour obtenir le composé de formule :

$$Ar - SO_2 - \underset{R_5}{N} - (CH_2)_n - \underset{R_6}{CH} - (CH_2)_m - COOH$$

— soit on prépare, dans une première étape, un ester de l'acide que l'on veut obtenir en faisant réagir un halogénure, notamment un chlorure d'acide de formule :

$$ArSO_2Cl$$

sur un composé de formule :

$$\underset{R_5}{HN} - (CH_2)_n - \underset{R_6}{CH} - (CH_2)_m - COOR_7$$

pour obtenir le composé de formule :

$$Ar - SO_2 - \underset{R_5}{N} - (CH_2)_n - \underset{R_6}{CH} - (CH_2)_m - COOR_7$$

puis dans une deuxième étape, on hydrolyse l'ester obtenu en son acide correspondant de formule :

$$Ar - SO_2 - \underset{R_5}{N} - (CH_2)_n - \underset{R_6}{CH} - (CH_2)_m - COOH$$

lorsque $R_4$ représente OH ou un radical alcoxy de 1 à 6 atomes de carbone, on prépare, comme indiqué ci-dessus le composé de formule :

$$Ar\ SO_2 - NH - (CH_2)_n - \underset{R_6}{CH} - (CH_2)_m - COR_4$$

et on fait réagir un halogénure d'alcoyle, tel qu'un iodure d'alcoyle $R_5I$ pour obtenir le composé de formule :

$$Ar - SO_2 - \underset{R_5}{N} - (CH_2)_n - \underset{R_6}{CH} - (CH_2)_m - \underset{O}{\overset{}{C}} - R_4$$

lorsque $R_4$ représente :

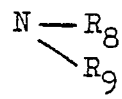

on prépare le composé de formule :

$$Ar - SO_2 - \underset{R_5}{N} - (CH_2)_n - \underset{R_6}{CH} - (CH_2)_m - \underset{O}{C} - OH$$

comme indiqué ci-dessus et on le fait réagir sur du chlorure de thionyle $SO_2Cl$ pour obtenir le composé de formule :

$$Ar - SO_2 - \underset{R_5}{N} - (CH_2)_n - \underset{R_6}{CH} - (CH_2)_m - \underset{O}{C} - Cl$$

puis on fait réagir l'amine de formule :

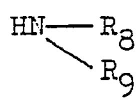

sur le chlorure d'acide mentionné ci-dessus pour obtenir le composé de formule :

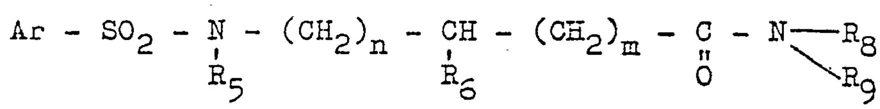

2. Procédé de préparation de composés utiles pour la préparation de médicaments selon la revendication 1, caractérisé en ce que l'un au moins des radicaux $R_5$ ou $R_6$ représente l'hydrogène.

3. Procédé de préparation de composés utiles pour la préparation de médicaments selon la revendication 1 ou 2, caractérisé en ce que le noyau benzénique substitué a pour formule :

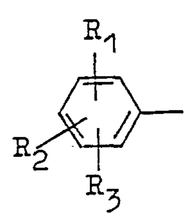

dans lequel $R_1$, $R_2$, $R_3$ sont identiques ou différents et représentent un atome d'hydrogène ou sont simultanément différents de l'hydrogène et sont choisis dans le groupe constitué par un atome d'halogène, le radical $NO_2$, le radical $NH_2$, $CF_3$ un groupe alcoyle ayant de 1 à 6 atomes de carbone, et de préférence de 1 à 4 atomes de carbone, et de préférence de 1 à 4 atomes de carbone, un groupe alcoxy ayant de 1 à 6 atomes de carbone, et de préférence de 1 à 4 atomes de carbone, un groupe acide, un groupe ester ayant de 2 à 7 atomes de carbone, et de préférence de 2 à 5 atomes de carbone.

4. Procédé de préparation de composés utiles pour la préparation de médicaments selon la

revendication 3, caractérisé en ce que $R_1$, $R_2$, $R_3$ représentent simultanément un atome d'hydrogène.

5. Procédé de préparation de composés utiles pour la préparation de médicaments selon la revendication 3, caractérisé en ce que les composés ont pour formule :

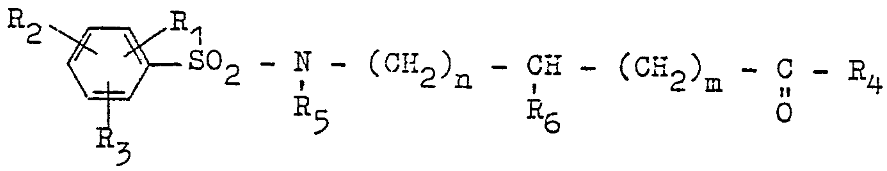

(III)

dans laquelle $R_1$, $R_2$, et $R_3$ sont simultanément différents de l'hydrogène.

6. Procédé de préparation de composés utiles pour la préparation de médicaments selon la revendication 5, caractérisé en ce que le noyau benzénique est trisubstitué par les radicaux choisis dans le groupe comprenant $NO_2$, $NH_2$, $OCH_3$, $CH_3$, un atome d'halogène, et notamment le chlore.

7. Procédé de préparation de composés utiles pour la préparation de médicaments selon les revendications 1 à 3, caractérisé en ce qu'ils répondent à la formule (III) dans laquelle :

$R_1$ représente un atome d'hydrogène ;

$R_2$ et $R_3$ représentent un atome d'halogène, le radical $NO_2$, le radical $NH_2$, un groupe alcoyle ayant de 1 à 6 atomes de carbone et de préférence de 1 à 4 atomes de carbone, un groupe alcoxy ayant de 1 à 6 atomes de carbone, et de préférence de 1 à 4 atomes de carbone, un groupe acide, un gorupe ester ayant de 2 à 7 atomes de carbone, et de préférence de 2 à 5 atomes de carbone.

8. Procédé de préparation de composés utiles pour la préparation de médicaments selon la revendication 7, caractérisé en ce que le noyau benzénique est substitué par les radicaux choisis dans le groupe constitué par $NO_2$, $NH_2$, $OCH_3$ ou par un halogène, notamment le chlore.

9. Procédé de préparation de composés utiles pour la préparation de médicaments selon les revendications 1 à 3, caractérisé en ce qu'ils répondent à la formule (III) dans laquelle :

$R_1$ et $R_2$ représentent simultanément un atome d'hydrogène ;

$R_3$ représente un atome d'halogène, le radical $NO_2$, le radical $NH_2$, un groupe alcoyle ayant de 1 à 6 atomes de carbone de préférence de 1 à 4 atomes de carbone, un groupe alcoxy ayant de 1 à 6 atomes de carbone et de préférence de 1 à 4 atomes de carbone, un groupe acide, un groupe ester ayant de 2 à 7 atomes de carbone, et de préférence de 2 à 5 atomes de carbone.

10. Procédé de préparation de composés utiles pour la préparation de médicaments selon la revendication 1, caractérisé en ce que Ar représente le cycle de formule :

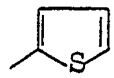

dans lequel le carbone en position alpha du $SO_2$ peut être éventuellement substitué par un atome d'halogène, notamment le chlore ou par un radical méthyle.

11. Procédé de préparation de composés utiles pour la préparation de médicaments selon la revendication 1, caractérisé en ce que Ar représente le cycle de formule :

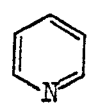

ce cycle pouvant éventuellement être substitué par $CF_3$.

12. Procédé de préparation de composés utiles pour la préparation de médicaments selon la revendication 1, caractérisé en ce qu'ils comprennent à titre de substance active un des composés de formule :

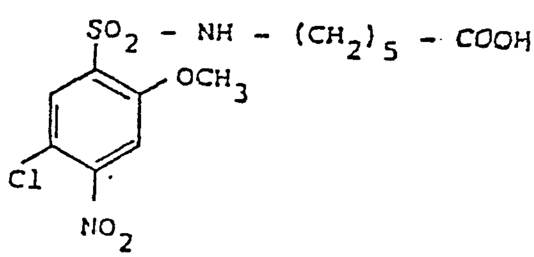

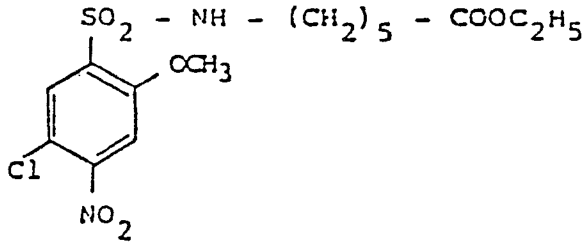

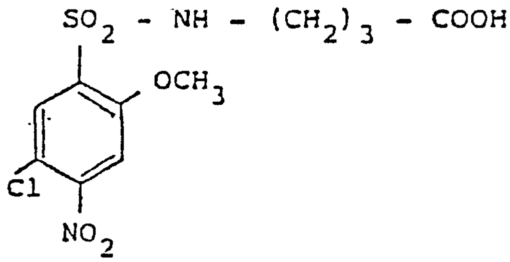

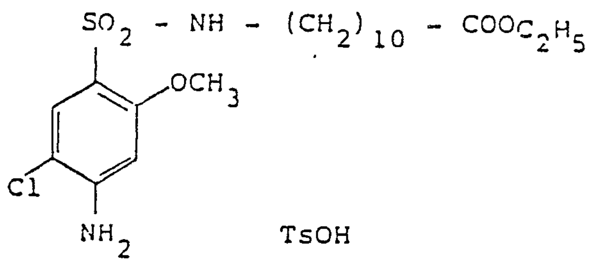

TsOH

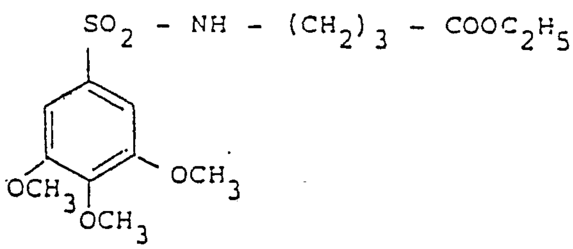

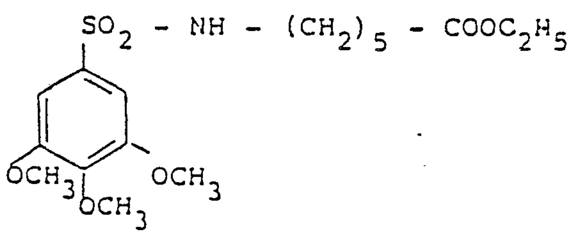

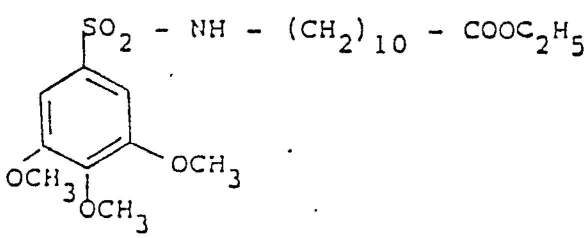

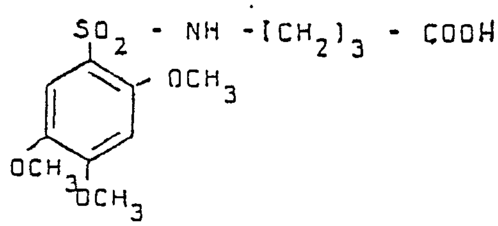

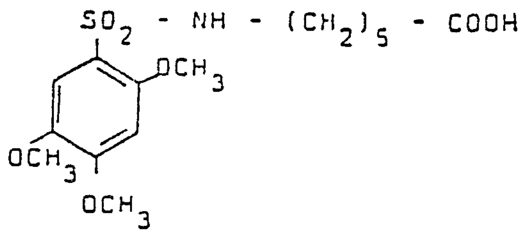

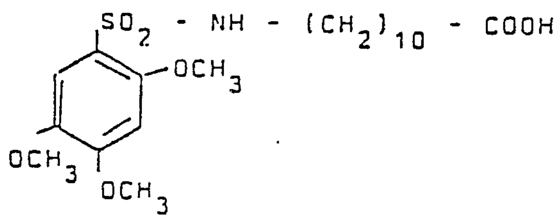

13. Procédé de préparation de composés utiles pour la préparation de médicaments selon la revendication 1, caractérisé en ce qu'ils comprennent à titre de substance active un des composés de formule :

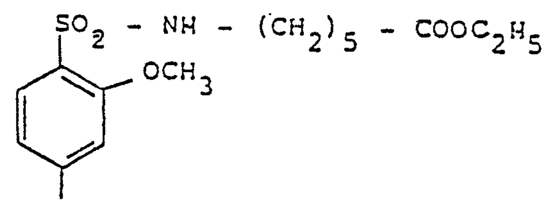

· TsOH

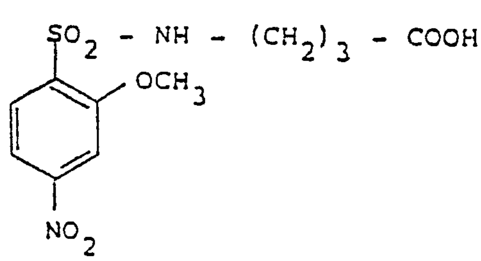

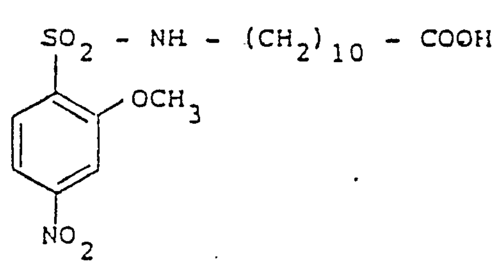

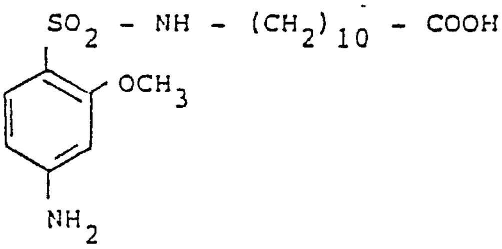

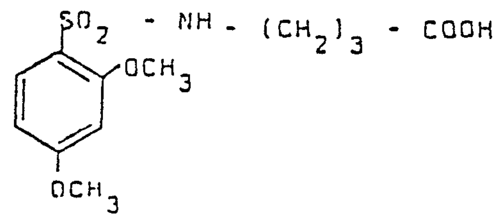

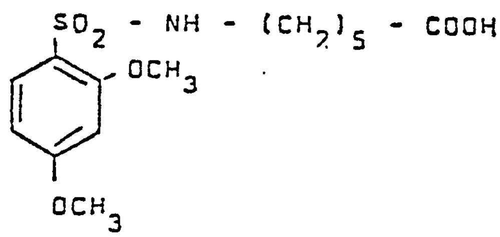

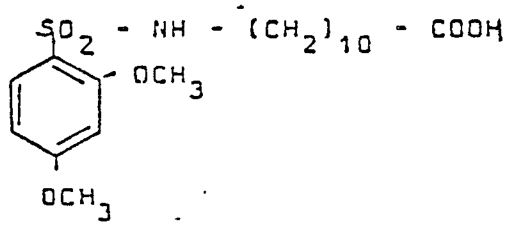

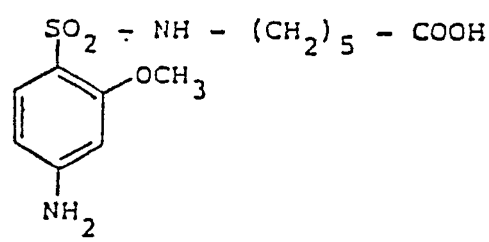

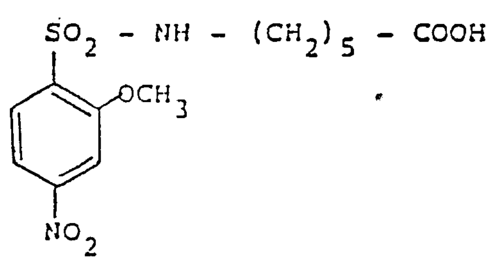

14. Procédé de préparation de composés utiles pour la préparation de médicaments selon la revendication 1, caractérisé en ce qu'ils comprennent à titre de substance active un des composés de formule :

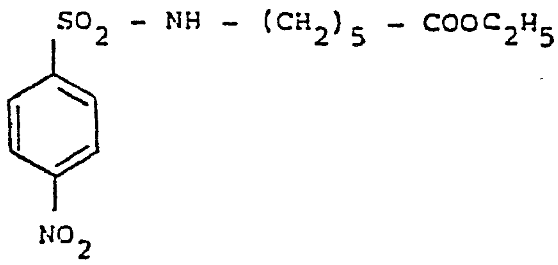

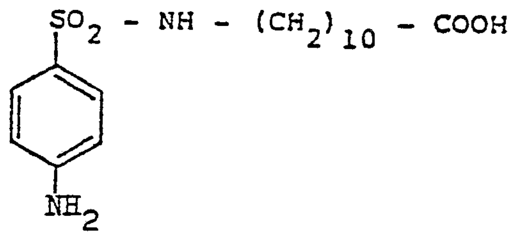

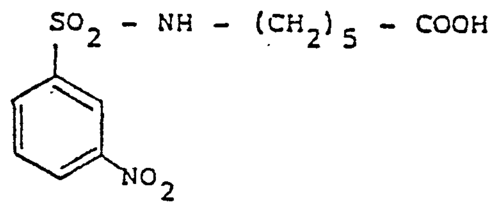

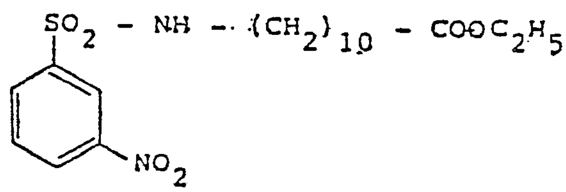

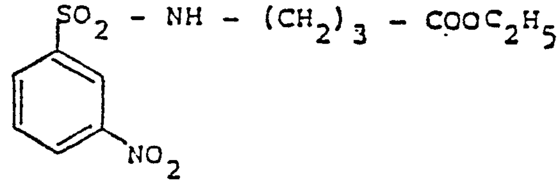

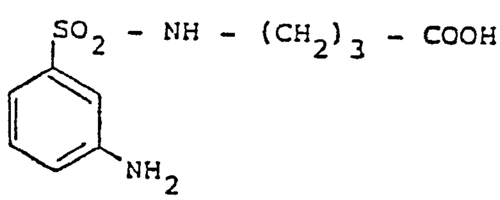

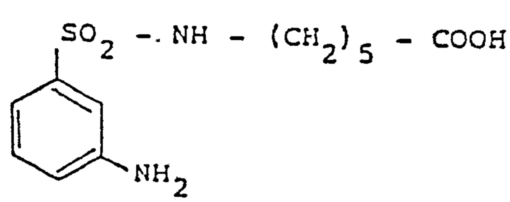

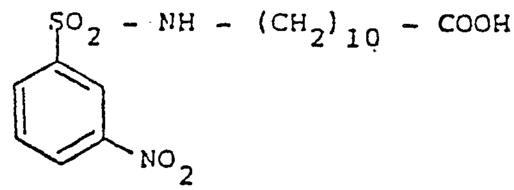

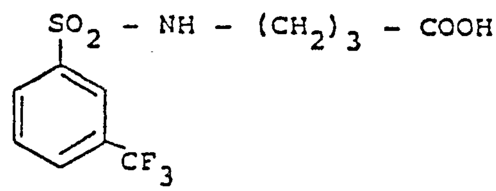

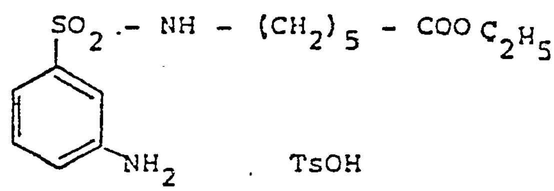

TsOH

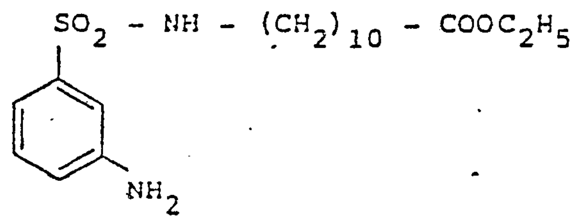

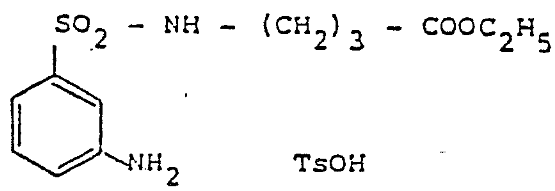

TsOH

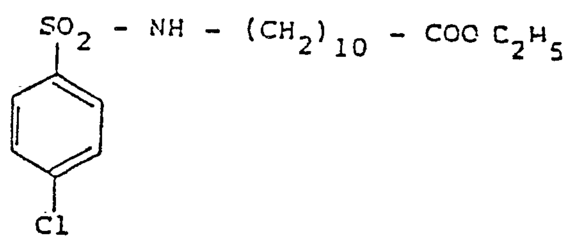

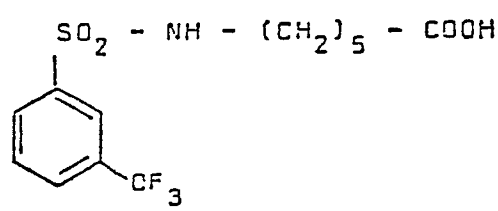

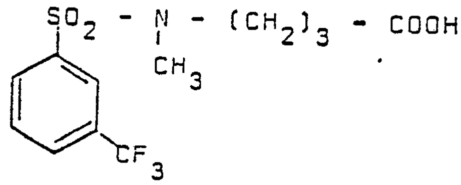

15. Procédé de préparation de composés utiles pour la préparation de médicaments selon la revendication 1, caractérisé en ce qu'ils comprennent à titre de substance active un composé de formule :

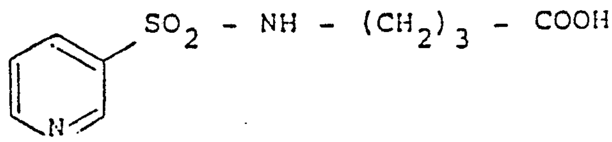

16. Procédé de préparation de composés utiles pour la préparation de médicaments selon la revendication 1, caractérisé en ce qu'ils comprennent à titre de substance active un des composés de formule :

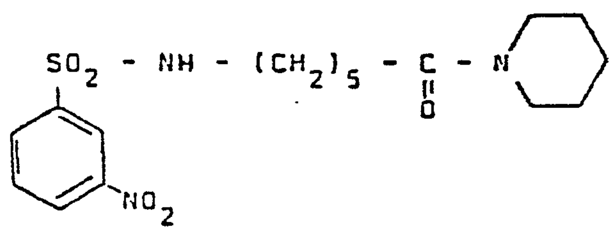

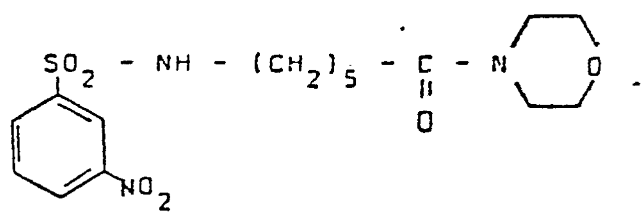

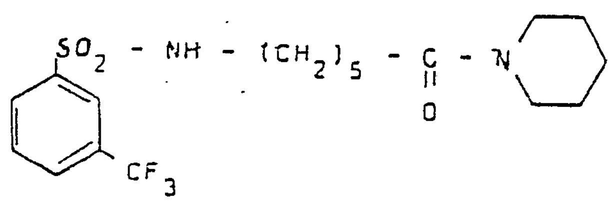

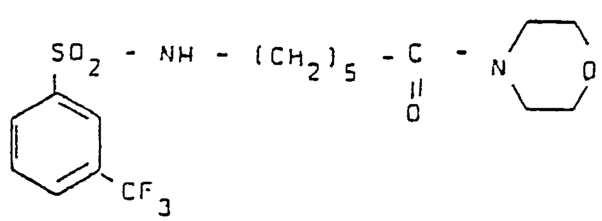

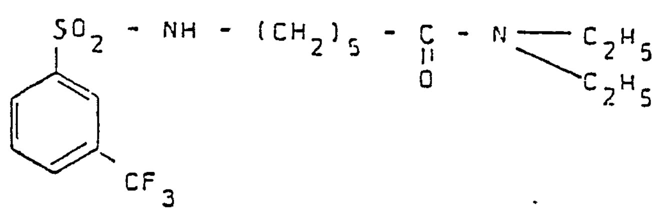

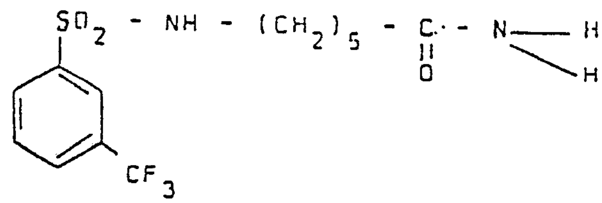

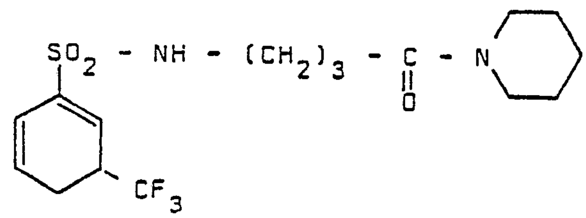

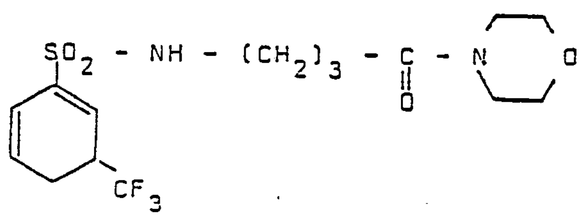

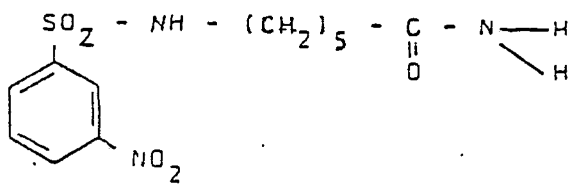

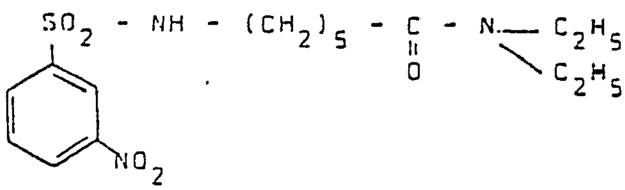

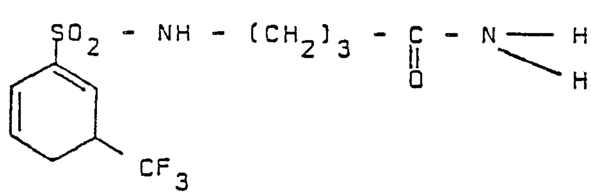

17. Procédé de préparation d'une composition pharmaceutique, caractérisé en ce que l'on associe l'un quelconque des composés obtenus à l'issue du procédé selon l'une quelconque des revendications 1 à 16, avec un véhicule pharmaceutiquement acceptable, dans des conditions permettant la production de ladite composition pharmaceutique.

**Claims** (for the Contracting States : BE, CH, DE, GB, IT, LI, LU, NL, SE)

1. New compounds useful for the preparation of medicaments and their corresponding optical isomers characterized by the fact that they correspond to the following formula :

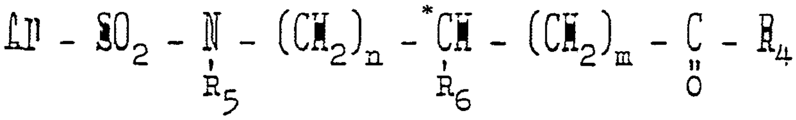

in which

Ar represents one of the following rings:

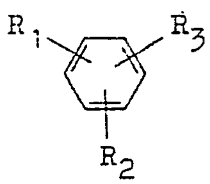

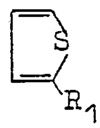

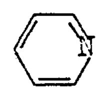

$R_1$, $R_2$, $R_3$ are identical or different and represent a hydrogen atom, a halogen atom, the $NO_2$ radical, the $NH_2$ radical, $CF_3$, an alkyl group having from 1 to 6 carbon atoms, preferably from 1 to 4 carbon atoms, an alkoxy group having from 1 to 6 carbon atoms, preferably from 1 to 4 carbon atoms, an acid group, an ester group having from 2 to 7 carbon atoms and preferably from 2 to 5 carbon atoms:

the sum n + m + 1 is comprised from 3 to 11, being preferably equal to 3, 5 or 10, this sum being also liable to be equal to 1 or 2, when Ar contains a benzenic ring of which one at least of the substituents $R_1$, $R_2$ and $R_3$ is a $CF_3$ group;

$R_5$ and $R_6$ represent independently of one another a hydrogen atom, an alkyl radical having from 1 to 6 carbon atoms, preferably from 1 to 4 carbon atoms, an aralkyl radical having from 7 to 9 carbon atoms;

$R_4$ represents:

— either a hydroxy group,

— or an $OR_7$ radical in which $R_7$ is an alkyl group having from 1 to 6 carbon atoms, and preferably from 1 to 4 carbon atoms;

— or a

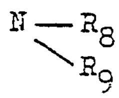

radical in which $R_8$ and $R_9$, identical or different, represent a hydrogen atom, an alkyl group having from 1 to 6 carbon atoms, and preferably from 1 to 4 carbon atoms, or conjointly from with the nitrogen a nitrogenous heterocyclic group with 5 or 6 links, particularly a piperidino, morpholino, pyrrolidino, pyrrole or pyrrolino group, with the proviso that when Ar contains a benzenic ring and when $R_4$ represents a hydroxy group or the $OR_7$ radical, one at least of the three substituents $R_1$, $R_2$ or $R_3$ represents the $CF_3$ radical.

2. Compounds according to claim 1, characterized by the fact that one at least of the radicals $R_5$ or $R_6$ represents hydrogen.

3. Compounds according to any of claims 1 or 2, of formula:

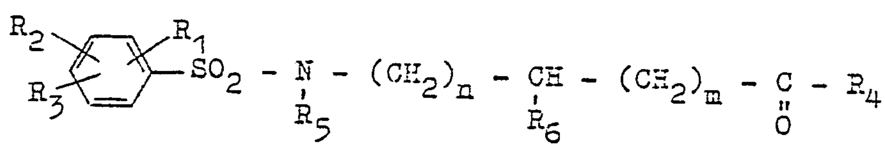

in which

one at least of the substituents $R_1$, $R_2$ or $R_3$ represents the $CF_3$ radical;

$R_4$ represents:

— the hydroxy group,

— the $OR_7$ radical in which $R_7$ is an alkyl group having from 1 to 6 carbon atoms, and preferably from 1 to 4 carbon atoms;

— the

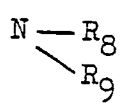

radical in which $R_8$ and $R_9$, identical or different, represents a hydrogen atom, an alkyl group having from 1 to 6 carbon atoms, and preferably from 1 to 4 carbon atoms, or conjointly form with the nitrogen a nitrogenous heterocyclic group with 5 or 6 links, particularly a piperidino, morpholino, pyrrolidino, pyrrole or pyrrolino group;

n + m + 1 is comprised from 3 to 11, being preferably equal to 3, 5 or 10.

4. Compounds according to claims 1 to 3, characterized by the fact that they correspond to the formula :

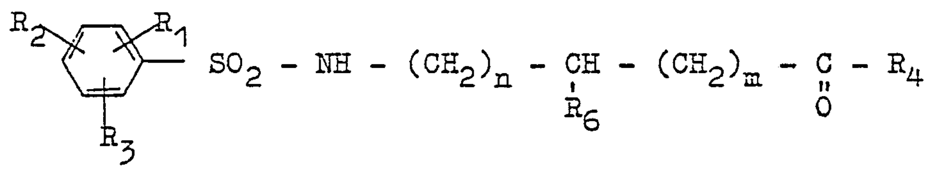

in which

one at least of the substituents $R_1$, $R_2$ or $R_3$ represents the $CF_3$ radical ;

$R_4$ represents :

— the hydroxy group,

— the $OR_7$ radical in which $R_7$ is an alkyl group having from 1 to 6 carbon atoms, and preferably from 1 to 4 carbon atoms ;

— the

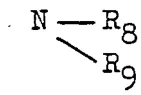

radical in which $R_8$ and $R_9$, identical or different, represents a hydrogen atom, an alkyl group having from 1 to 6 carbon atoms, and preferably from 1 to 4 carbon atoms, or conjointly form with the nitrogen a nitrogenous heterocyclic group with 5 or 6 links, particularly a piperidino, morpholino, pyrrolidino, pyrrole or pyrrolino group ;

n + m + 1 is comprised from 3 to 11, being preferably equal to 3, 5 or 10.

5. Compounds according to claims 1 to 4, characterized by the fact that they correspond to the formula :

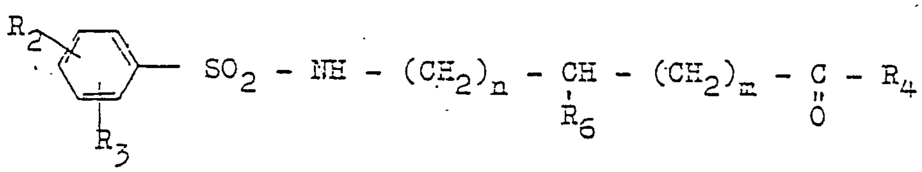

in which

one at least of the substituents $R_2$ or $R_3$ represents the $CF_3$ radical ;

$R_4$ represents :

— the hydroxy group,

— the $OR_7$ radical in which $R_7$ is an alkyl group having from 1 to 6 carbon atoms, and preferably from 1 to 4 carbon atoms ;

— the

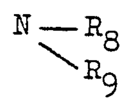

radical in which $R_8$ and $R_9$, identical or different, represents a hydrogen atom, an alkyl group having from 1 to 6 carbon atoms, and preferably from 1 to 4 carbon atoms, or conjointly form with the nitrogen a nitrogenous heterocyclic group with 5 or 6 links, particularly a piperidino, morpholino, pyrrolidino, pyrrole or pyrrolino group ;

n + m + 1 is comprised from 3 to 11, being preferably equal to 3, 5 or 10.

6. Compounds according to claims 1 to 5, characterized by the fact that they correspond to the formula :

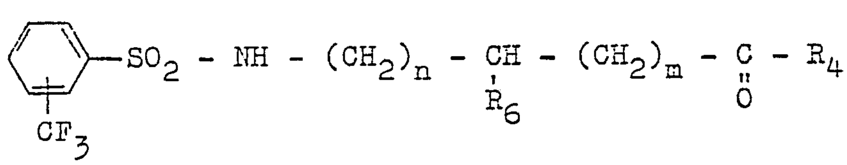

in which

$R_4$ represents :

— the hydroxy group,

— the $OR_7$ radical in which $R_7$ is an alkyl group having from 1 to 6 carbon atoms, and preferably from 1 to 4 carbon atoms ;

— the

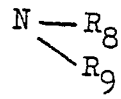

radical in which $R_8$ and $R_9$, identical or different, represents a hydrogen atom, an alkyl group having from 1 to 6 carbon atoms, and preferably form 1 to 4 carbon atoms, or conjointly form with the nitrogen a nitrogenous heterocyclic group with 5 or 6 links, particularly a piperidino, morpholino, pyrrolidino, pyrrole or pyrrolino group ;

n + m 1 is comprised from 3 to 11, being preferably equal to 3, 5 or 10.

7. Compounds according to claim 1, characterized by the fact that Ar represents the ring of formula :

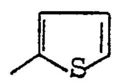

in which the carbon atom which is in position alpha of $SO_2$ can be optionally substituted by a halogen atom, notably chlorine or by a methyl radical.

8. Compounds according to claim 1, characterized by the fact that Ar represents the ring of formula :

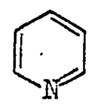

which can be optionally substituted by $CF_3$.

9. Compounds according to claim 1, characterized by the fact that they have the following formula :

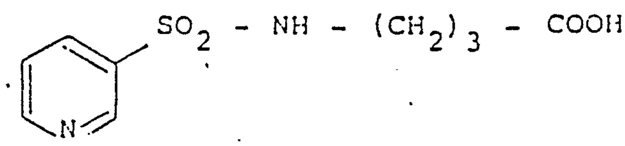

10. Compounds according to claim 1, characterized by the fact that they have the following formula :

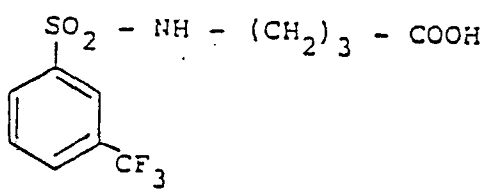

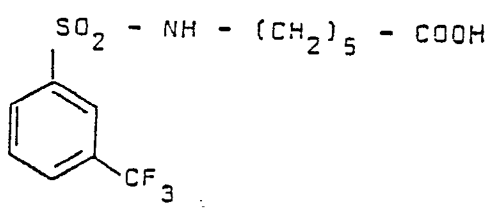

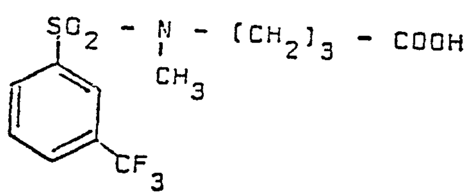

11. Compounds according to claim 1, characterized by the fact that they have the following formula :

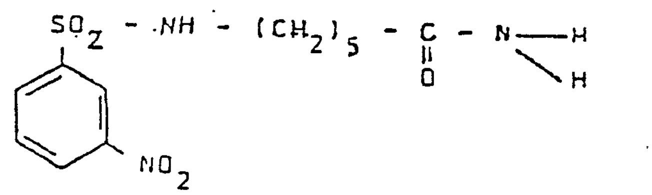

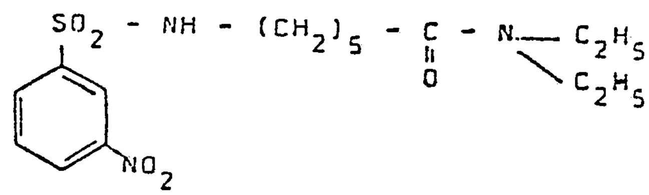

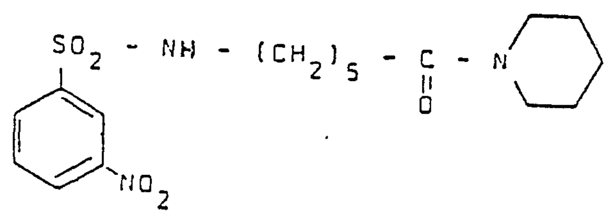

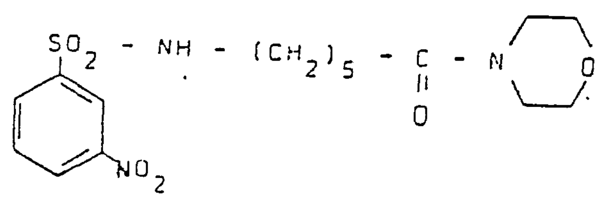

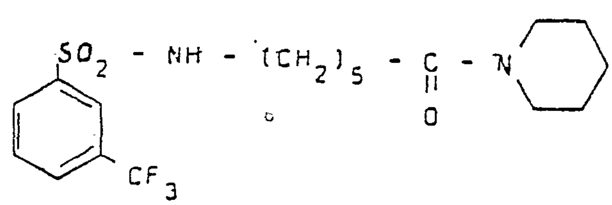

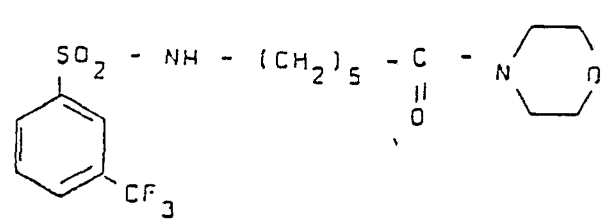

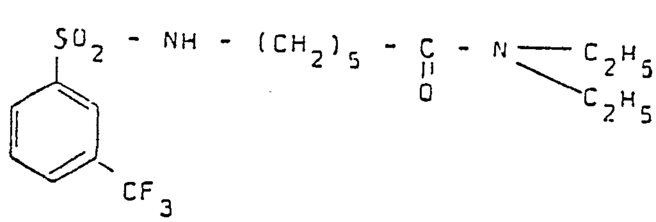

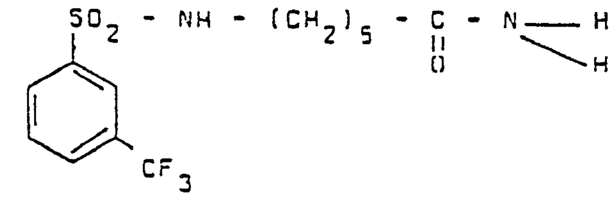

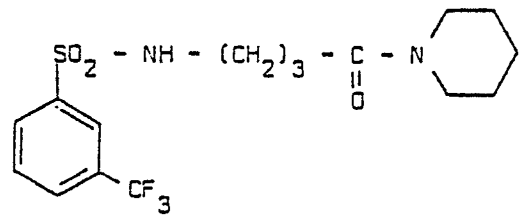

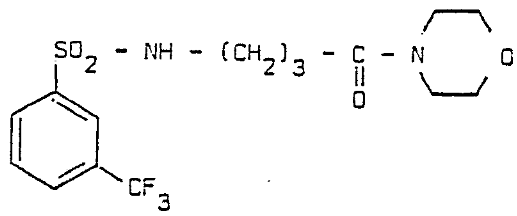

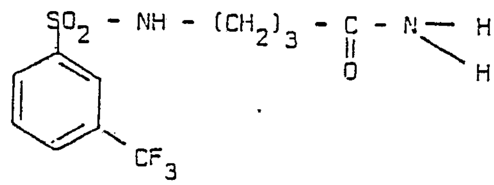

12. Medicaments characterized by the fact that they contain as active substance one at least of the compounds according to anyone of claims 1 to 11.

13. Medicament having lipid regulating properties, characteirzed by the fact that they contain as active substance at least one compound (or its corresponding optical isomer) of the arylsulfonamide type corresponding to the formula :

$$Ar - SO_2 - \underset{R_5}{\underset{|}{N}} - (CH_2)_n - \underset{R_6}{\underset{|}{CH}} - (CH_2)_m - \underset{O}{\underset{\|}{C}} - R_4 \qquad (I)$$

in which

Ar represents one of the following rings :

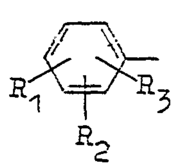

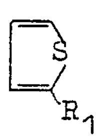

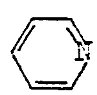

the substituents $R_1$, $R_2$, $R_3$ are identical or different and represent a hydrogen atom, a halogen atom, the $NO_2$ radical, the $NH_2$ radical, $CF_3$, an alkyl group having from 1 to 6 carbon atoms, preferably from 1 to 4 carbon atoms, an alkoxy group having from 1 to 6 carbon atoms and preferably from 1 to 4 carbon atoms, an acid group, an ester group having from 2 to 7 carbon atoms and preferably from 2 to 5 carbon atoms ;

the sum n + m + 1 is comprised from 1 to 11, being preferably equal to 3, 5 or 10, this sum being also liable to be equal to 1 or 2, when Ar contains a benzenic ring, of which one at least of the substituents $R_1$, $R_2$, $R_3$ is a $CF_3$ group ;

$R_5$ and $R_6$ represent independently of one another, a hydrogen atom, an alkyl radical having from 1 to 6 carbon atoms, preferably from 1 to 4 carbon atoms, an aralkyl radical having from 7 to 9 carbon atoms ;

$R_4$ represents :

— the hydroxy group,

— the $OR_7$ radical in which $R_7$ is an alkyl group having from 1 to 6 carbon atoms, and preferably from 1 to 4 carbon atoms ;

— the

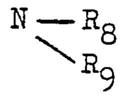

radical in which $R_8$ and $R_9$, identical or different, represents a hydrogen atom, an alkyl group having from 1 to 6 carbon atoms, and preferably from 1 to 4 carbon atoms, or conjointly form with the nitrogen a nitrogenous heterocyclic group with 5 or 6 links, particularly a piperidino, morpholino, pyrrolidino, pyrrole or pyrrolino group.

14. Medicament according to claim 13, characterized by the fact that at least one of the radicals $R_5$ or $R_6$ represents hydrogen.

15. Medicament according to any of claims 13 or 14, characterized by the fact that the substituted benzene nucleus has the formula :

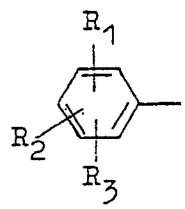

in which $R_1$, $R_2$, $R_3$ represent simultaneously a hydrogen atom, or are simultaneously different from hydrogen and are selected from the group constituted by a halogen atom, the $NO_2$ radical, the $NH_2$ radical, $CF_3$, an alkyl group having from 1 to 6 carbon atoms, and preferably from 1 to 4 carbon atoms, an alkoxy group having from 1 to 6 carbon atoms, and preferably from 1 to 4 carbon atoms, an acid group, an ester group having from 2 to 7 carbon atoms, and preferably from 2 to 5 carbon atoms.

16. Medicament according to claim 15, characterized by the fact that $R_1$, $R_2$ and $R_3$ represent simultaneously a hydrogen atom.

17. Medicament according to claim 15, characterized by the fact that it corresponds to the following formula (III) :

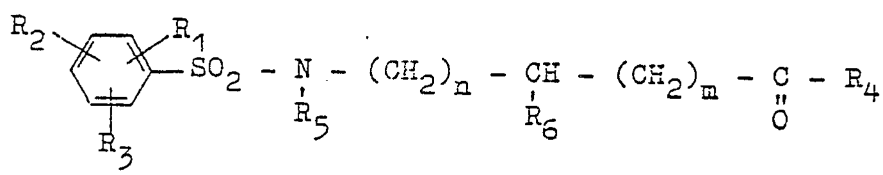 (III)

in which $R_1$, $R_2$ and $R_3$ are simultaneously different from hydrogen.

18. Medicament according to claim 17, characterized by the fact that the benzene nucleus is trisubstituted by the radical selected from the group comprising $NO_2$, $NH_2$, $OCH_3$, $CH_3$, a halogen atom and particularly chlorine.

19. Medicament according to any of claims 13 to 15, characterized by the fact that it corresponds to the formula (III) in which :

$R_1$ represents a hydrogen atom ;

$R_2$ and $R_3$ represent a halogen atom, the $NO_2$ radical, the $NH_2$ radical, an alkyl group having from 1 to 6 carbon atoms and preferably from 1 to 4 carbon atoms, an alkoxy group having from 1 to 6 carbon atoms, and preferably from 1 to 4 carbon atoms, an acid group, an ester group having from 2 to 7 carbon atoms, and preferably from 2 to 5 carbon atoms.

20. Medicament according to claim 17, characterized by the fact that the benzene nucleus is substitued by radicals selected from the group constituted by $NO_2$, $NH_2$, $OCH_3$ or by a halogen, particularly chlorine.

21. Medicament according to any of claims 13 to 15, characterized by the fact that it corresponds to formula (III) in which :

$R_1$ and $R_2$ represent simultaneously a hydrogen atom ;

$R_3$ represents a halogen atom, the $NO_2$ radical, the $NH_2$ radical, an alkyl group having from 1 to 6 carbon atoms, and preferably from 1 to 4 carbon atoms, an alkoxy group having from 1 to 6 carbon atoms

and preferably from 1 to 4 carbon atoms, an acid group, an ester group having from 2 to 7 carbon atoms, and preferably 2 to 5 carbon atoms.

22. Medicament according to claim 13, characterized by the fact that Ar represents the ring of formula :

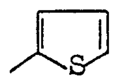

in which the carbon which is at the alpha position with respect to $SO_2$ may be possibly substituted by a halogen atom, particularly chlorine or by a methyl radical.

23. Medicament according to claim 13, characterized by the fact that Ar represents the ring of formula :

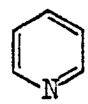

this ring being if necessary substitutable by $CF_3$.

24. Medicament according to claim 11, characterized by the fact that it comprises as active substance one of the compounds of formula :

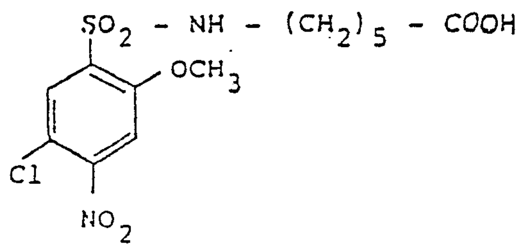

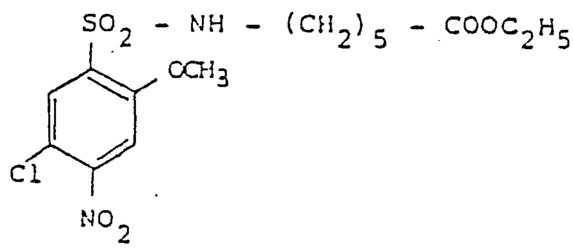

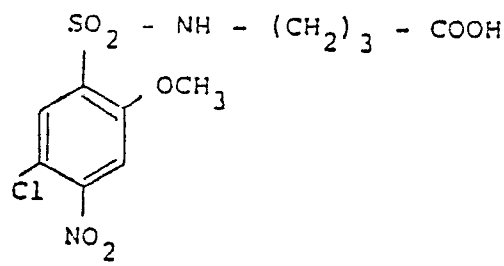

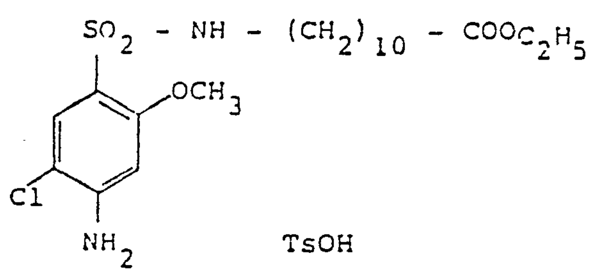

TsOH

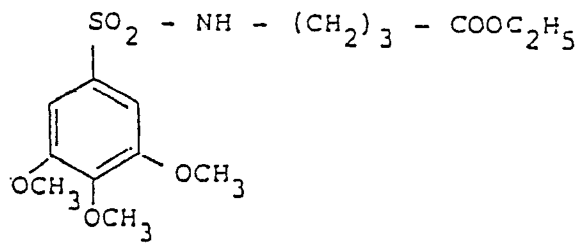

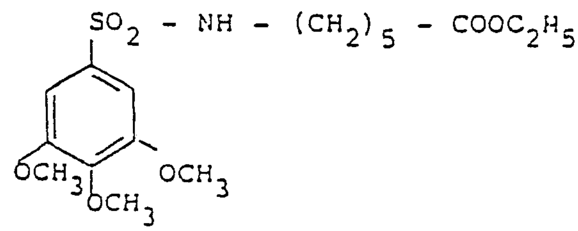

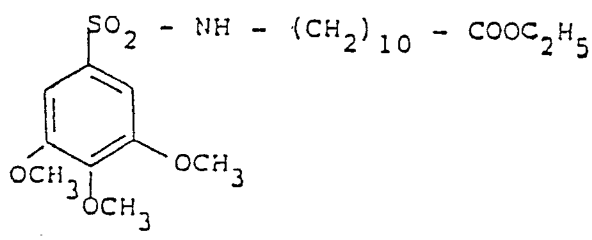

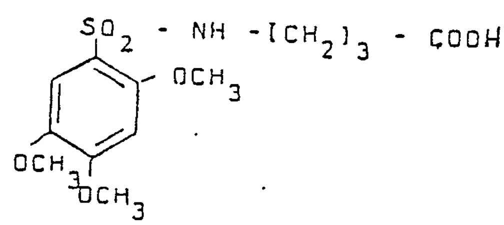

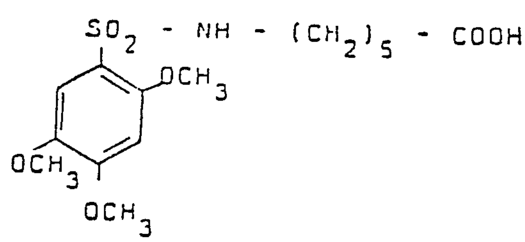

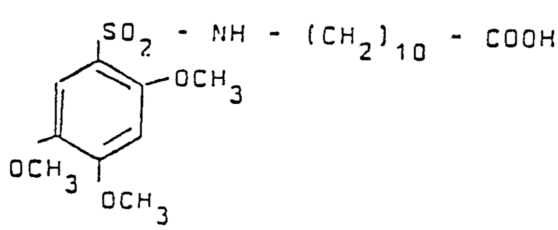

25. Medicament according to claim 13, characterized by the fact that it comprises as active substance a compound of formula :

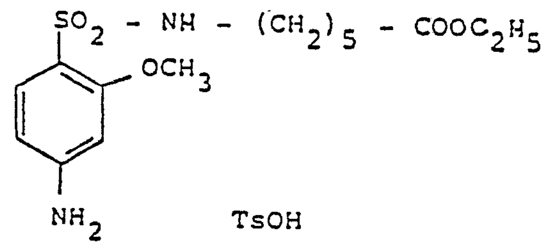

TsOH

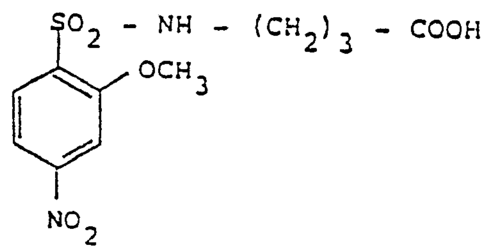

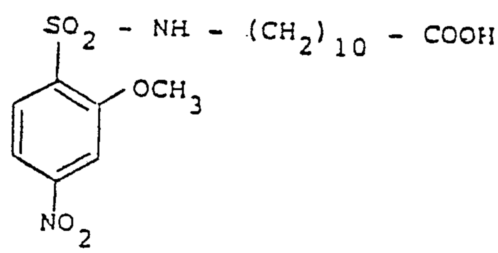

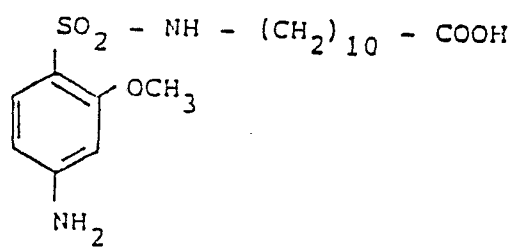

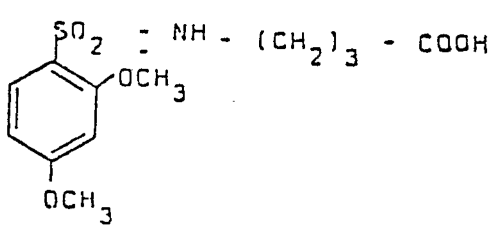

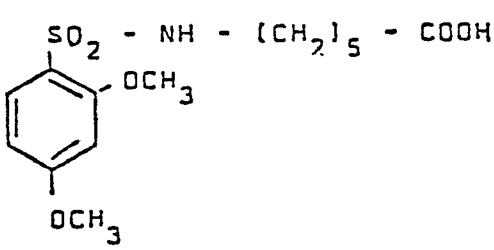

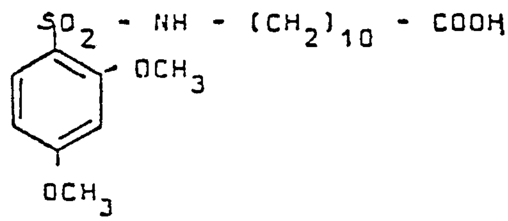

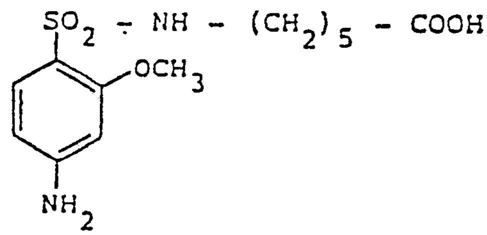

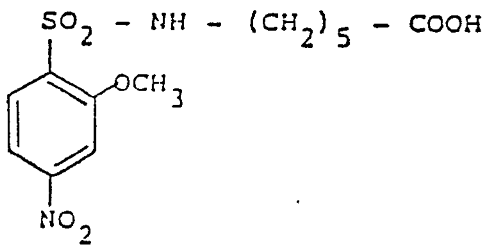

26. Medicament according to claim 13, characterized by the fact that it comprises as active substance a compound of formula :

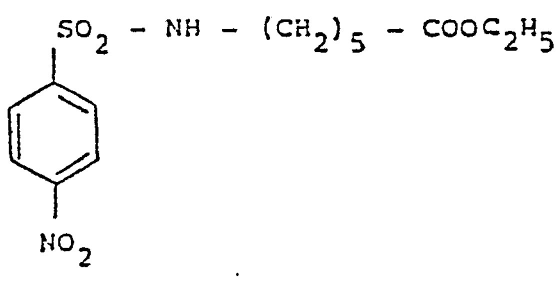

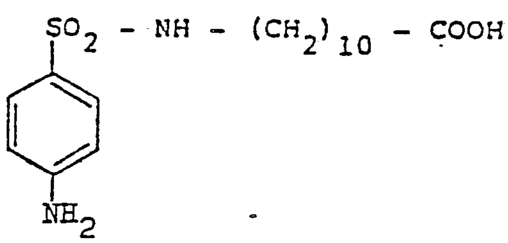

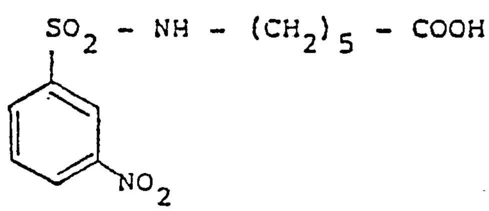

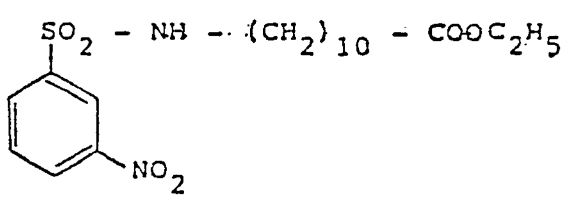

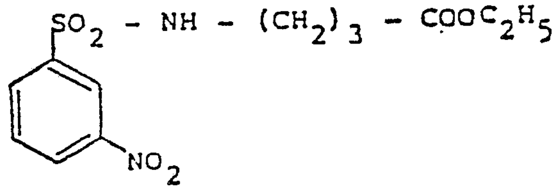

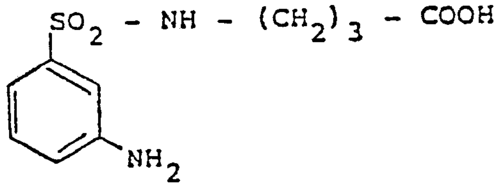

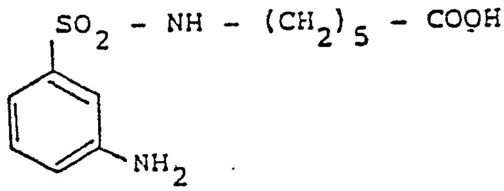

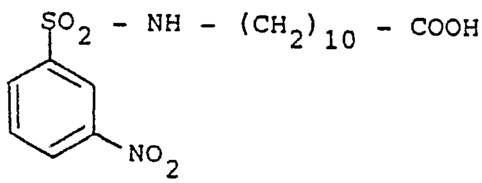

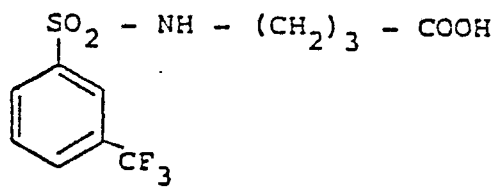

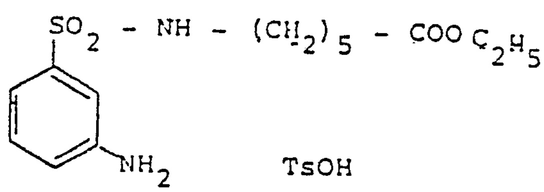

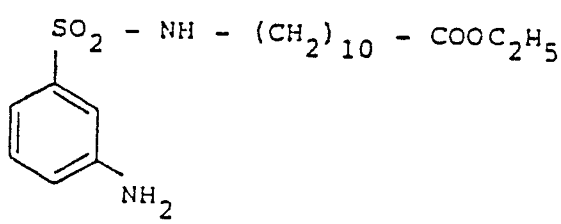

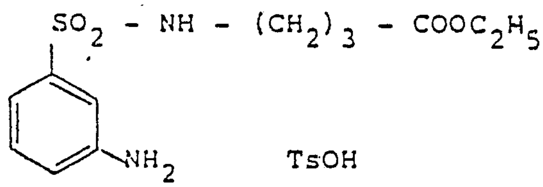

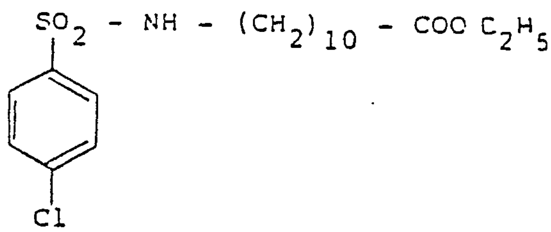

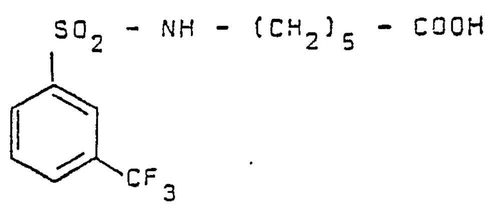

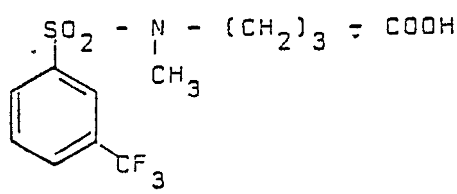

27. Medicament according to claim 13, characterized by the fact that it comprises as active substance a compound of formula :

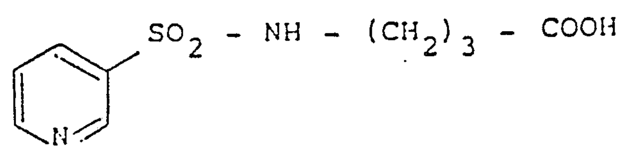

28. Medicament according to claim 13, characterized by the fact that it comprises as active substance a compound of formula :

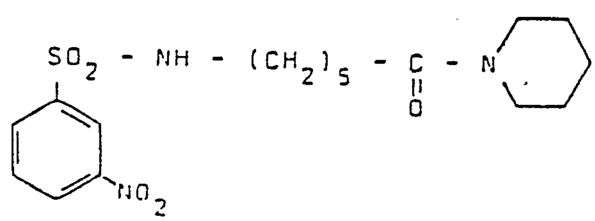

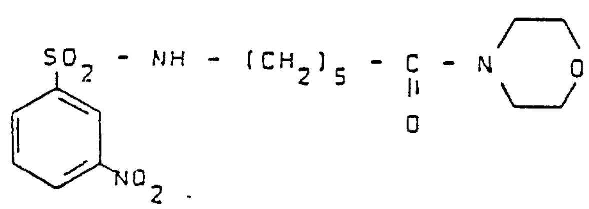

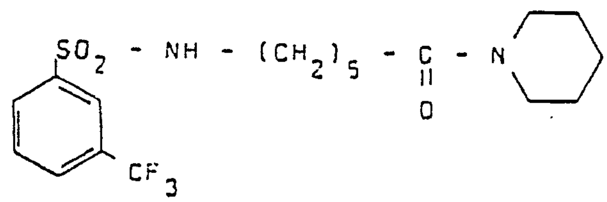

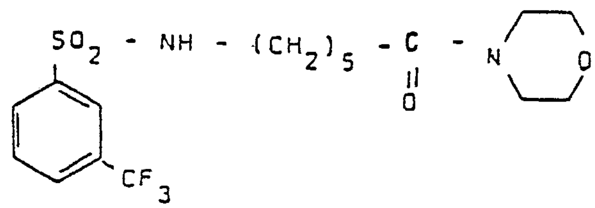

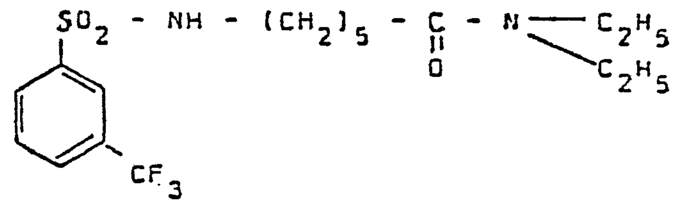

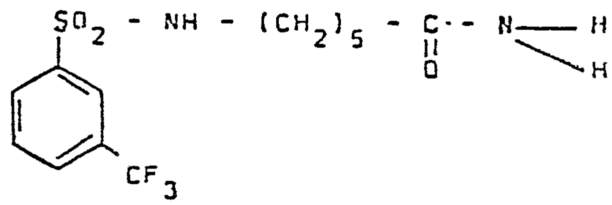

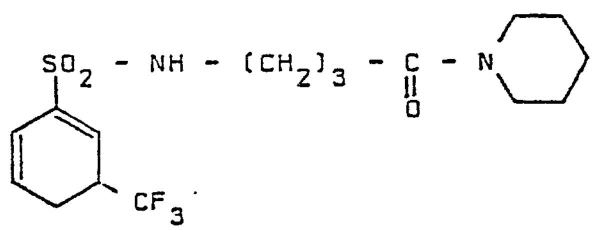

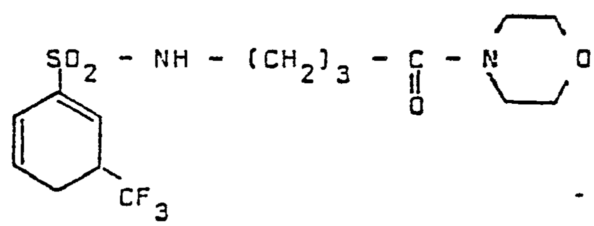

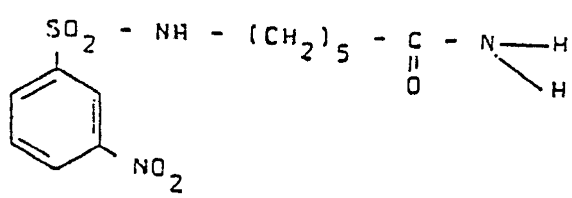

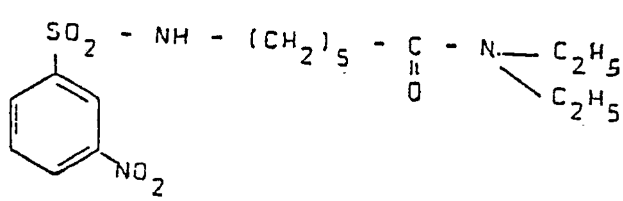

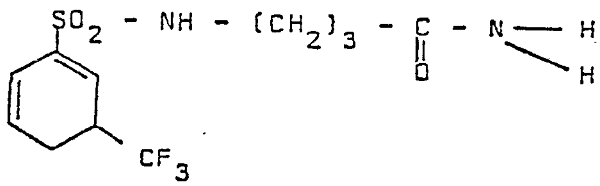

29. Pharmaceutically acceptable salts, particularly tosylate and hydrochloride, of compounds according to any of claims 1 to 28.

30. Medicament according to any of claims 12 to 29, for oral administration, characterized by the fact that they contain about 500 mg, preferably from about 50 to 250 mg of active substance.

31. Medicament according to any of claims 12 to 29, for topical administration, characterized by the fact it contains from 1 to 20 % by weight of active substance.

**Claims** (for the Contracting State AT)

1. Process for preparing compounds useful for the preparation of medicaments of formula :

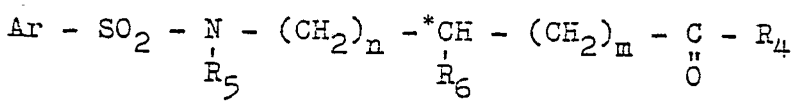

as well as the optical isomers of the compounds of formula (I), in which

Ar represents one of the following rings :

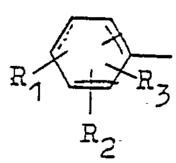
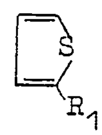
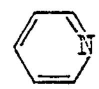

the substituents $R_1$, $R_2$, $R_3$ are identical or different and represent a hydrogen atom, a halogen atom, the $NO_2$ radical, the $NH_2$ radical, $CF_3$, an alkyl group having from 1 to 6 carbon atoms, preferably from 1 to 4 carbon atoms, an alkoxy group having from 1 to 3 carbon atoms, preferably from 1 to 4 carbon atoms, an acid group, an ester group having from 2 to 7 carbon atoms and preferably from 2 to 5 carbon atoms ;

the sum $n + m + 1$ is comprised from 3 to 11, being preferably equal to 3, 5 or 10, this sum being also liable to be equal to 1 or 2, when Ar contains a benzenic ring of which one at least of the substituents $R_1$, $R_2$ and $R_3$ is a $CF_3$ group ;

$R_5$ and $R_6$ represent independently of one another a hydrogen atom, an alkyl radical having from 1 to 6 carbon atoms, preferably from 1 to 4 carbon atoms, an aralkyl radical having from 7 to 9 carbon atoms ;

$R_4$ represents :

— either a hydroxy group,

— or an $OR_7$ radical in which $R_7$ is an alkyl group having from 1 to 6 carbon atoms, and preferably from 1 to 4 carbon atoms,

— or a

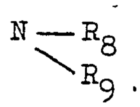

in which $R_8$ and $R_9$, identical or different, represent a hydrogen atom, an alkyl group having from 1 to 6 carbon atoms, and preferably from 1 to 4 carbon atoms, or conjointly form with nitrogen a nitrogenous heterocyclic group of 5 to 6 links, particularly a piperidino, morpholino, pyrrolidino, pyrrol or pyrrolino group ; characterized by the fact that :

when $R_4$ represents $OR_7$, $R_7$ being an alkyl group of 1 to 6 carbon atoms :

— either a halide, particularly an acid chloride of formula :

$$ArSO_2Cl$$

is reacted on to an aminoester of formula :

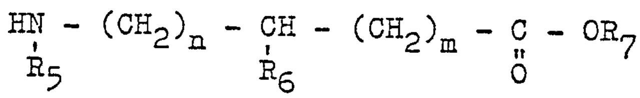

or its hydrochloride of formula :

$$\underset{R_5}{HN} - (CH_2)_n - \underset{R_6}{CH} - (CH_2)_m - \underset{O}{\overset{}{C}} - OR_7\ ,\ HCl$$

to obtain the compound of formula :

$$Ar - SO_2 - \underset{R_5}{N} - (CH_2)_n - \underset{R_6}{CH} - (CH_2)_m - \underset{O}{C} - OR_7$$

— or a halide, particularly an acid chloride of formula :

$$ArSO_2Cl$$

is reacted on to an aminoacid of formula :

$$\underset{R_5}{HN} - (CH_2)_n - \underset{R_6}{CH} - (CH_2)_m - \underset{O}{C} - OH$$

then the acid thus obtained is esterified by reacting it on to an appropriate alcohol of formula $R_7OH$ to obtain the compound of formula :

$$Ar - SO_2 - \underset{R_5}{N} - (CH_2)_n - \underset{R_6}{CH} - (CH_2)_m - \underset{O}{C} - OR_7$$

or an acid of formula :

$$ArSO_2H$$

is reacted on to an aminoester of formula :

$$\underset{R_5}{HN} - (CH_2)_n - \underset{R_6}{CH} - (CH_2)_m - \underset{O}{C} - OR_7$$

to obtain the compound of formula :

$$Ar - SO_2 - \underset{R_5}{N} - (CH_2)_n - \underset{R_6}{CH} - (CH_2)_m - \underset{O}{C} - OR_7$$

when $R_4$ represents the hydroxy radical :
— either an acid chloride :

$$ArSO_2Cl$$

is reacted on to an aminoacid of formula :

$$Ar - SO_2 - \underset{R_5}{N} - (CH_2)_n - \underset{R_6}{CH} - (CH_2)_m - COOH$$

— or in a first step, an ester of the acid which is desired is prepared by reacting a halide, particularly an acid chlorid of formula :

$$ArSO_2Cl$$

on to a compound of formula :

$$\underset{R_5}{HN} - (CH_2)_n - \underset{R_6}{CH} - (CH_2)_m - COOR_7$$

to obtain the compound of formula:

$$Ar - SO_2 - \underset{R_5}{N} - (CH_2)_n - \underset{R_6}{CH} - (CH_2)_m - COOR_7$$

then in a second step, the ester which is obtained is hydrolyzed into its corresponding acid of formula:

$$Ar - SO_2 - \underset{R_5}{N} - (CH_2)_n - \underset{R_6}{CH} - (CH_2)_m - COOH$$

when $R_4$ represents OH or an alkoxy radical of 1 to 6 carbon atoms, the compound of formula:

$$Ar - SO_2 - NH - (CH_2)_n - \underset{R_6}{CH} - (CH_2)_m - COOR_7$$

is prepared as mentioned above and it is reacted on to an alkyl halide, such as an alkyl iodide to obtain the compound of formula:

$$Ar - SO_2 - \underset{R_5}{N} - (CH_2)_n - \underset{R_6}{CH} - (CH_2)_m - \underset{O}{\overset{\parallel}{C}} - R_4$$

when $R_4$ represents

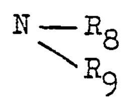

the compound of formula:

$$Ar - SO_2 - \underset{R_5}{N} - (CH_2)_n - \underset{R_6}{CH} - (CH_2)_m - \underset{O}{\overset{\parallel}{C}} - OH$$

is prepared as mentioned above and it is reacted on to a thionyl chloride $SO_2Cl$ to obtain the compound of formula:

$$Ar - SO_2 - \underset{R_5}{N} - (CH_2)_n - \underset{R_6}{CH} - (CH_2)_m - \underset{O}{\overset{\parallel}{C}} - Cl$$

then the amine of formula:

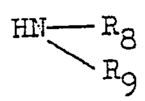

is reacted on to the acid chloride mentioned above to obtain the compound of formula:

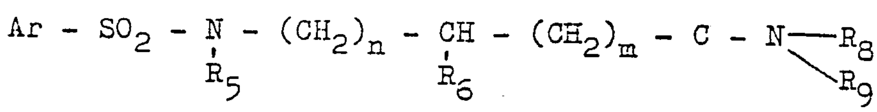

2. Process for preparing compounds useful for the preparation of medicaments according to claim 1, characterized by th fact that one at least of the $R_5$ or $R_6$ radicals represents hydrogen.

3. Process for preparing compounds useful for the preparation of medicaments according to claim 1 or 2, characterized by the fact that the substituted benzene ring has the formula:

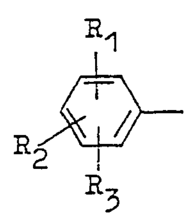

in which $R_1$, $R_2$, $R_3$ are identical or different and represent a hydrogen atom or are simultaneously different from hydrogen and are selected in the group constituted by a halogen atom, the $NO_2$ radical, the $NH_2$ radical, $CF_3$, an alkyl group from 1 to 6 carbon atoms, and preferably from 1 to 4 carbon atoms, an alkoxy group having from 1 to 6 carbon atoms and preferably 1 to 4 carbon atoms, an acid group, an ester group of 2 to 7 carbon atoms, and preferably from 2 to 5 carbon atoms.

4. Process for preparing compounds useful for the preparation of medicaments according to claim 3, characterized by the fact that $R_1$, $R_2$, $R_3$ represent simultaneously a hydrogen atom.

5. Process for preparing compounds useful for the preparation of medicaments according to claim 3, characterized by the fact that the compounds have the formula :

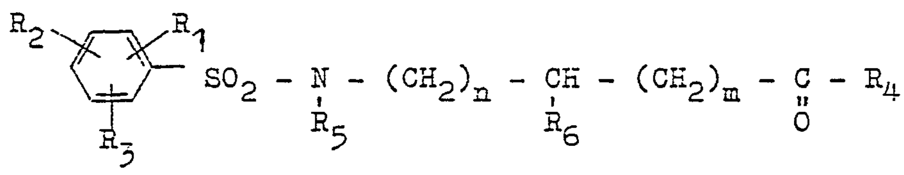

(III)

in which $R_1$, $R_2$ and $R_3$ are simultaneously different from hydrogen.

6. Process for preparing compounds useful for the preparation of medicaments according to claim 5, characqterized by the fact that the benzene ring is trisubstituted by the radicals chosen in the group comprising $NO_2$, $NH_2$, $OCH_3$, $CH_3$, a halogen atom and particularly chlorine.

7. Process for preparing compounds useful for the preparation of medicaments according to claims 1 to 3, characterized by the fact that they have the formula (III) in which :

$R_1$ represents a hydrogen atom ;

$R_2$ and $R_3$ represent a halogen atom, the $NO_2$ radical, the $NH_2$ radical, an alkyl group from 1 to 6 carbon atoms and preferably 1 to 4 carbon atoms, an alkoxy group of 1 to 6 carbon atoms and preferably 1 to 4 carbon atoms, an acid group, an ester group having from 2 to 7 carbon atoms and preferably 2 to 5 carbon atoms.

8. Process for preparing compounds useful for the preparation of medicaments according to claim 7, characterized by the fact that the benzene ring is substituted by radicals chosen in the group constituted by $NO_2$, $NH_2$, $OCH_3$ or a halogen, particularly chlorine.

9. Process for preparing compounds useful for the preparation of medicaments according to claims 1 to 3, characterized by the fact that they have the formula (III) in which :

$R_1$ and $R_2$ simultaneously represent a hydrogen atom ;

$R_3$ represents a halogen atom, the $NO_2$ radical, the $NH_2$ radical, an alkyl group having from 1 to 6 carbon atoms, an alkoxy group having from 1 to 6 carbon atoms, an acid group, an ester group having from 2 to 7 carbon atoms and preferably from 2 to 5 carbon atoms.

10. Process for preparing compounds useful for the preparation of medicaments according to claim 1, characterized by the fact that Ar represents a cycle of formula :

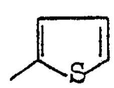

in which the carbon in the alpha position of $SO_2$ can be possibly substituted by a halogen atom, particularly chlorine or by a methyl radical.

11. Process for preparing compounds useful for the preparation of medicaments according to claim 1, characterized by the fact that Ar represents the cycle of formula :

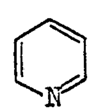

this cycle being liable to be substituted by $CF_3$.

12. Process for preparing compounds useful for the preparation of medicaments according to claim 1, characterized by the fact that they comprise, as active substance, one of the compounds of formula :

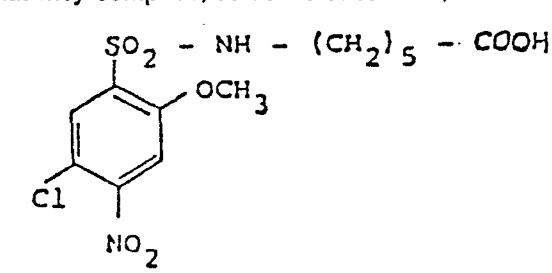

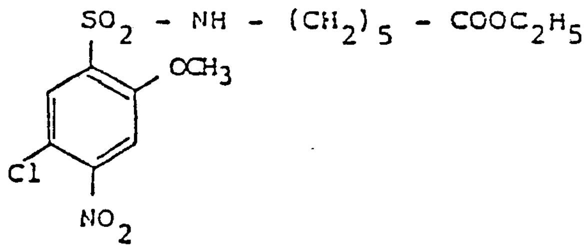

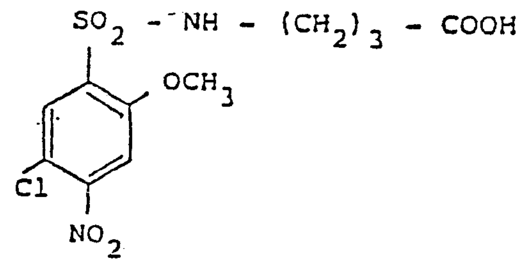

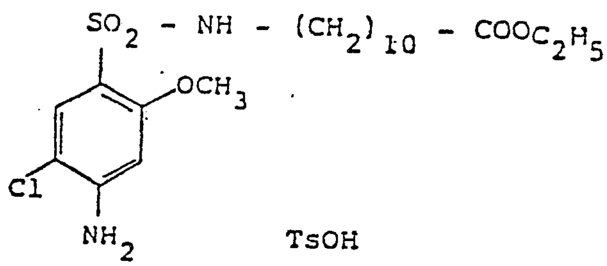

TsOH

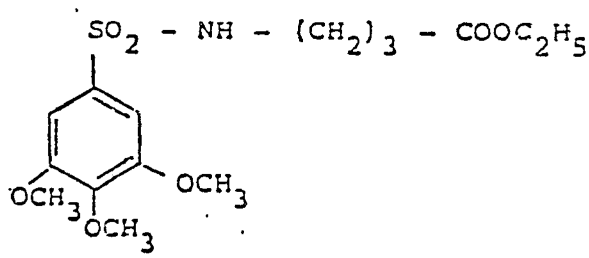

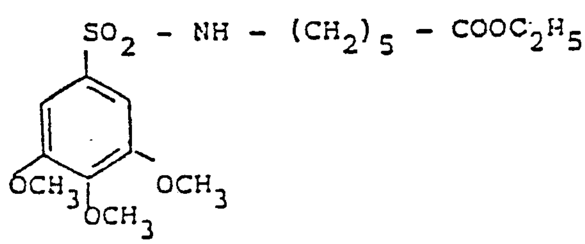

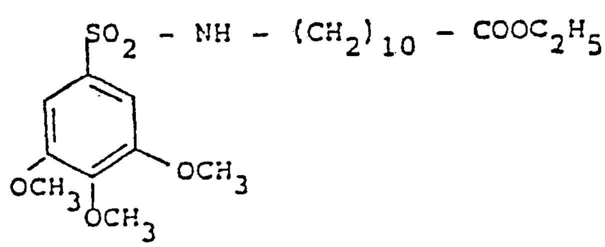

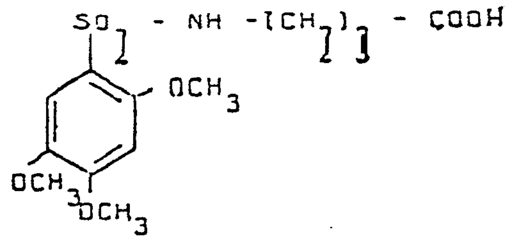

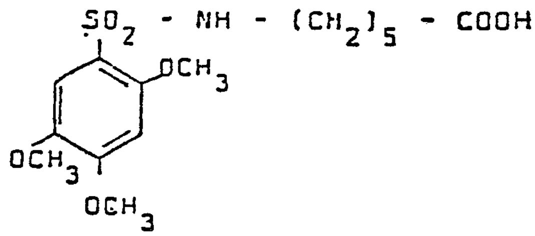

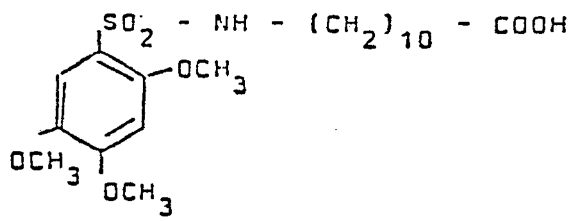

13. Process for preparing compounds useful for the preparation of medicaments according to claim 1, characterized by the fact that they comprise, as active substance, one of the compounds of formula :

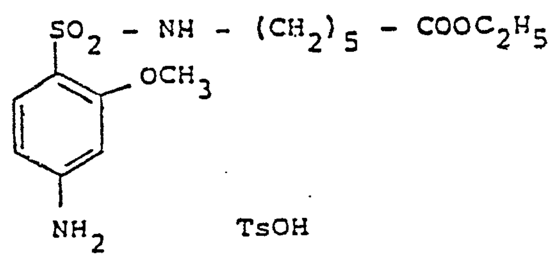

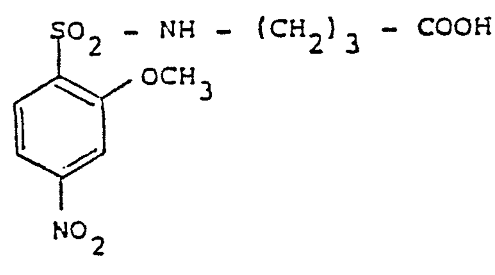

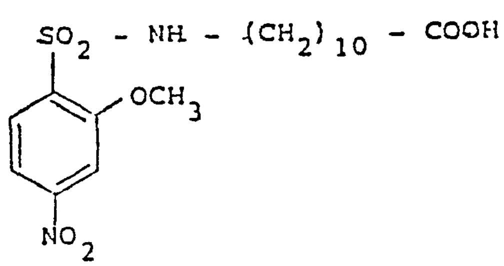

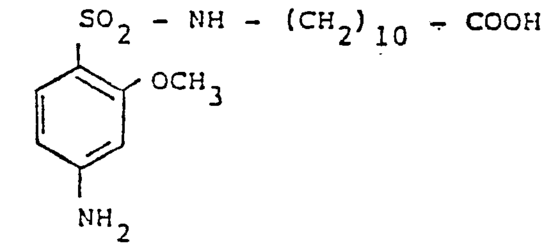

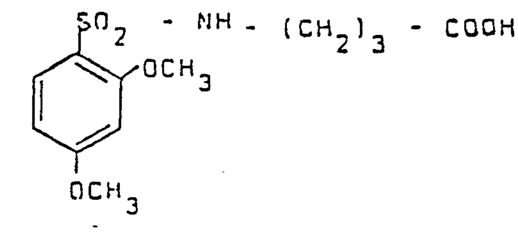

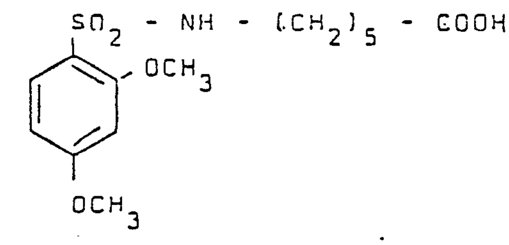

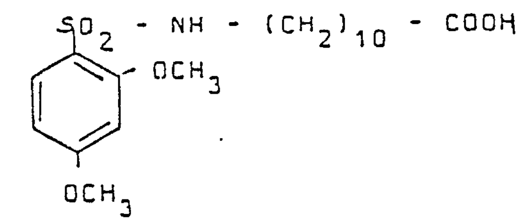

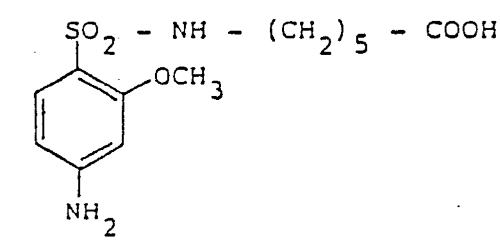

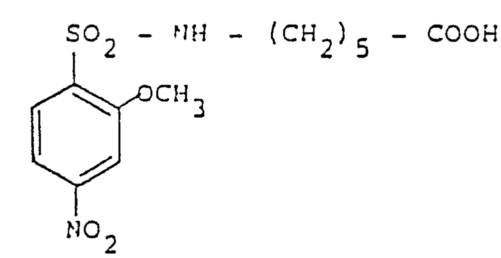

14. Process for preparing compounds useful for the preparation of medicaments according to claim 1, characterized by the fact that they comprise, as active substance, one of the compounds of formula :

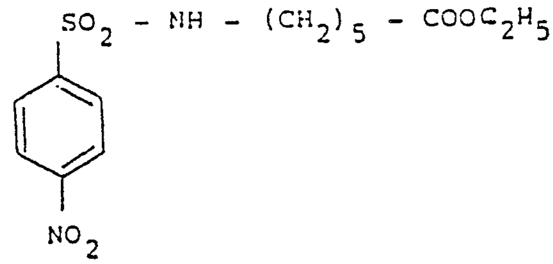

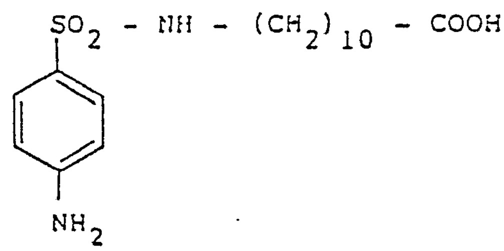

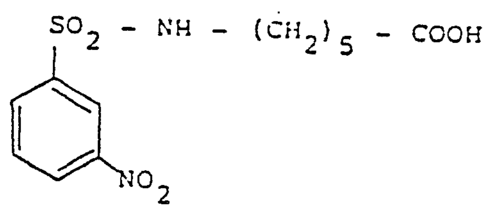

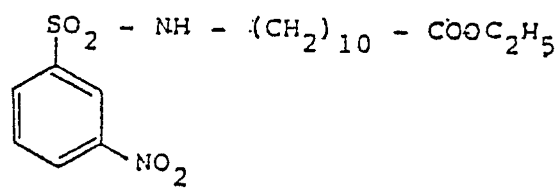

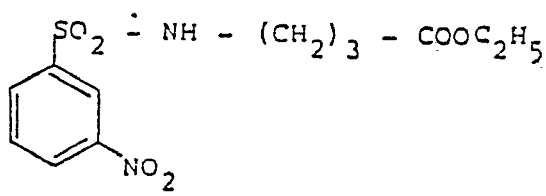

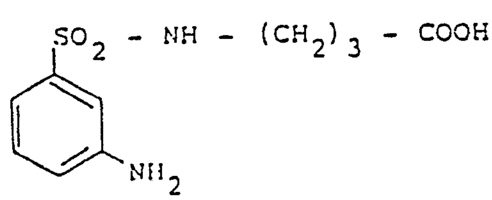

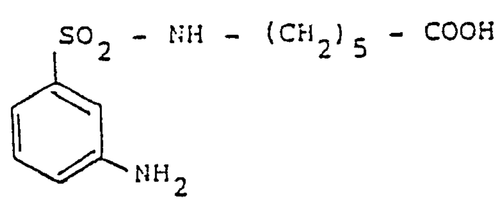

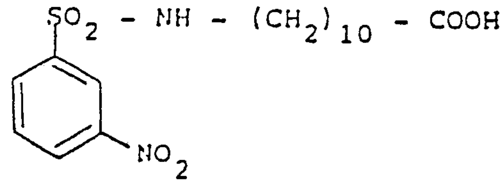

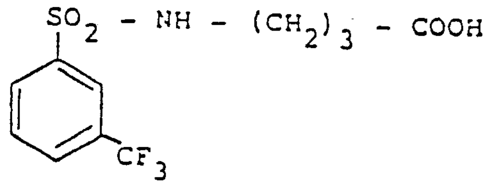

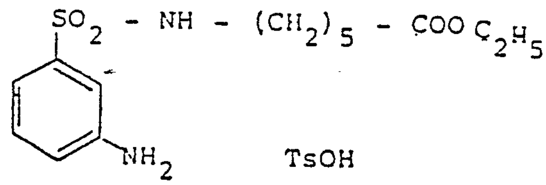

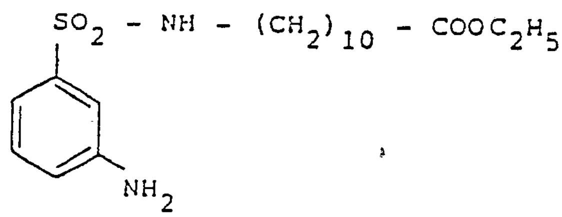

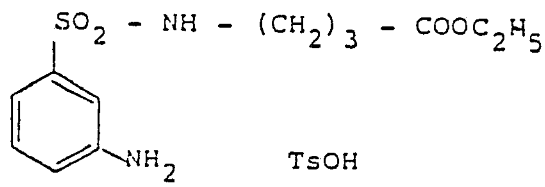

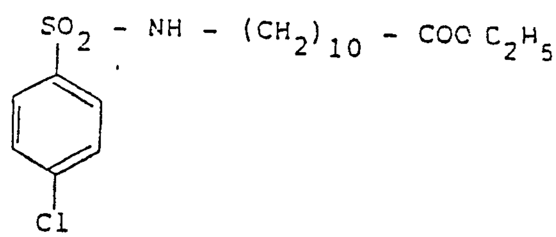

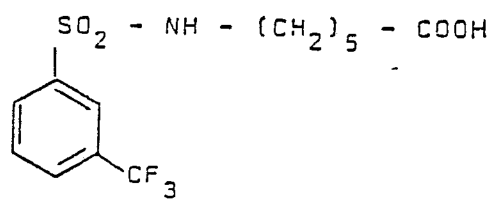

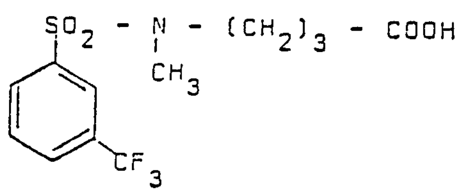

15. Process for preparing compounds useful for the preparation of medicaments according to claim 1, characterized by the fact that they comprise, as active substance, one of the compounds of formula :

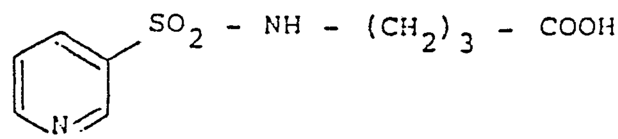

16. Process for preparing compounds useful for the preparation of medicaments according to claim 1, characterized by the fact that they comprise, as active substance, one of the compounds of formula :

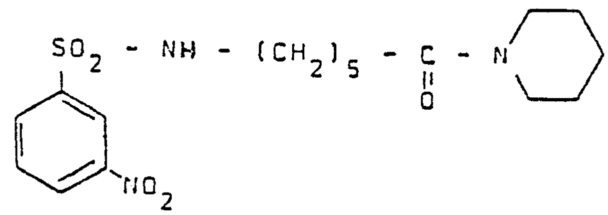

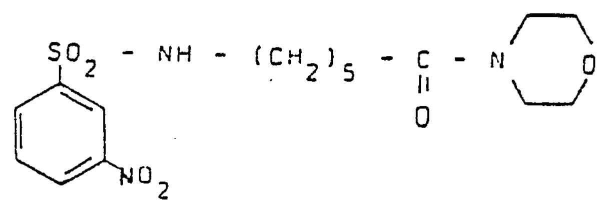

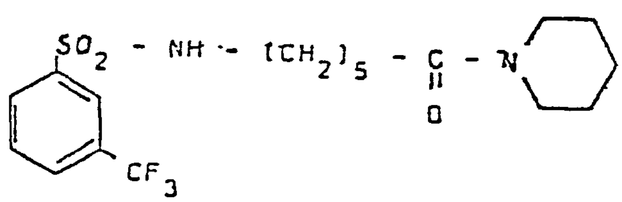

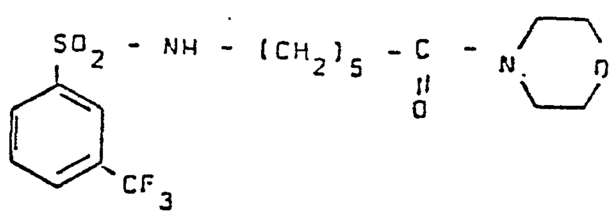

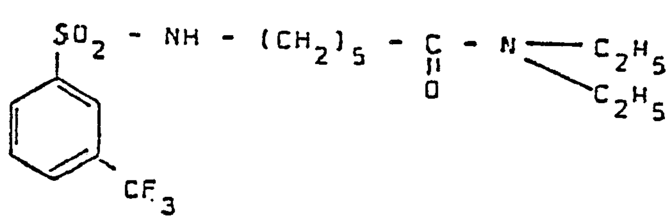

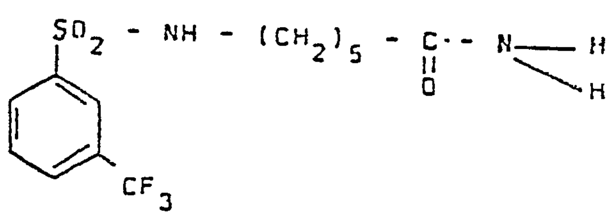

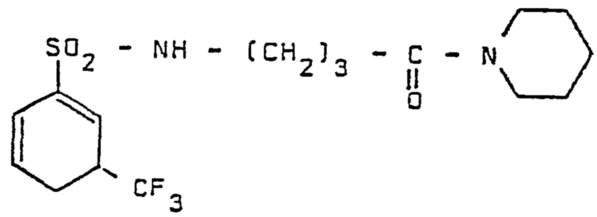

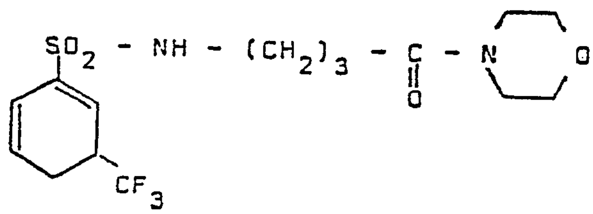

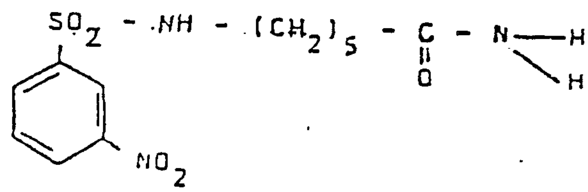

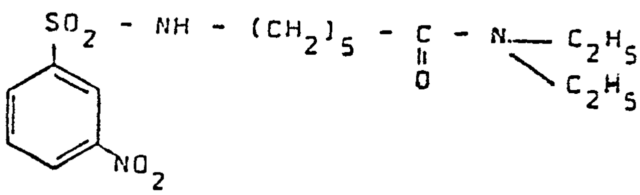

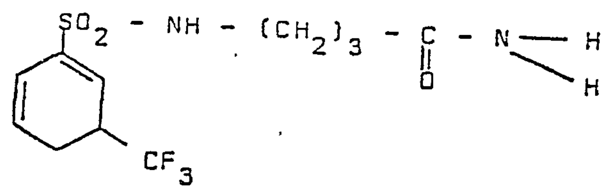

17. Process for preparing a pharmaceutical composition, characterized by the fact that any of compounds obtained according to the process of any of claims 1 to 16, is associated with a pharmaceutically acceptable carrier, under conditions enabling the preparation of said pharmaceutical composition.

**Patentansprüche** (für die Vertragsstaaten : BE, CH, DE, GB, IT, LI, LU, NL, SE)

1. Neue, für die Herstellung von Arzneimitteln brauchbare Verbindungen sowie ihre entsprechenden optischen Isomeren, dadurch gekennzeichnet, daß sie der Formel :

$$Ar - SO_2 - \underset{R_5}{N} - (CH_2)_n - \underset{R_6}{CH} - (CH_2)_m - \underset{O}{\overset{}{C}} - R_4$$

entsprechen, in welcher

Ar einen der folgenden cyclischen Reste bedeutet :

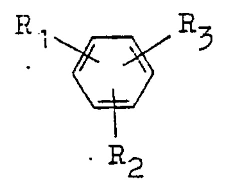 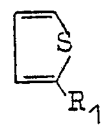 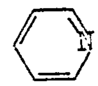

$R_1$, $R_2$ und $R_3$ gleich oder verschieden sind und ein Wasserstoffatom, ein Halogenatom, den $NO_2$-Rest, den $NH_2$-Rest, den $CF_3$-Rest, eine Alkylgruppe mit 1 bis 6 und vorzugsweise 1 bis 4 Kohlenstoffatomen, eine Alkoxygruppe mit 1 bis 6 und vorzugsweise 1 bis 4 Kohlenstoffatomen, eine Säuregruppe, eine Estergruppe mit 2 bis 7 und vorzugsweise 2 bis 5 Kohlenstoffatomen bedeuten ;

die Summe $n + m + 1$ 3 bis 11 ausmacht und vorzugsweise gleich 3, 5 oder 10 ist, wobei diese Summe auch 1 oder 2 sein kann, falls Ar einen Benzolkern enthält, von dem wenigstens einer der Substituenten $R_1$, $R_2$ und $R_3$ eine $CF_3$ Gruppe ist ;

$R_5$ und $R_6$ unabhängig voneinander ein Wasserstoffatom, einen Alkylrest mit 1 bis 6 Kohlenstoffatomen, vorzugsweise 1 bis 4 Kohlenstoffatomen, oder einen Aralkylrest mit 7 bis 9 Kohlenstoffatomen bedeuten ;

$R_4$ entweder eine Hydroxygruppe

— oder einen $OR_7$-Rest, in welchem $R_7$ eine Alkylgruppe mit 1 bis 6 Kohlenstoffatomen, und vorzugsweise 1 bis 4 Kohlenstoffatomen, ist,

— oder einen

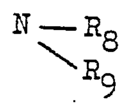

Rest bedeutet, in welchem $R_8$ und $R_9$, die gleich oder verschieden sind, ein Wasserstoffatom, eine Alkylgruppe mit 1 bis 6 Kohlenstoffatomen, und vorzugsweise 1 bis 4 Kohlenstoffatomen, bedeuten, oder zusammen mit dem Stickstoff einen stickstoffhältigen, 5- oder 6-gliedrigen Heterocyclus, insbesondere eine Piperidino-, Morpholino-, Pyrrolidino-, Pyrrol- oder Pyrrolingruppe, mit der Maßgabe bilden, daß, falls Ar einen Benzolkern enthält und $R_4$ eine Hydroxygruppe oder den Rest $OR_7$ bedeutet, wenigstens einer der drei Substituenten $R_1$, $R_2$ oder $R_3$ den $CF_3$-Rest bedeutet.

2. Verbindungen nach Anspruch 1, dadurch gekennzeichnet, daß wenigstens einer der Reste $R_5$ oder $R_6$ Wasserstoff bedeutet.

3. Verbindungen nach einem der Ansprüche 1 oder 2, der Formel :

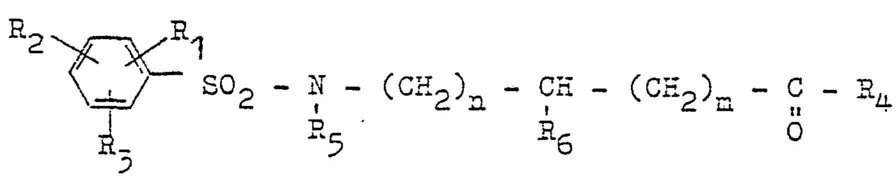

in welcher :

wenigstens einer der Substituenten $R_1$, $R_2$ und $R_3$ einen $CF_3$-Rest bedeutet ;

$R_4$ die Hydroxygruppe ;

— den $OR_7$-Rest, in welchem $R_7$ eine Alkylgruppe mit 1 bis 6 Kohlenstoffatomen, vorzugsweise 1 bis 4 Kohlenstoffatomen ist ; oder

— den Rest

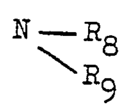

bedeutet, in welchem $R_8$ und $R_9$, die gleich oder verschieden sind, ein Wasserstoffatom, eine Alkylgruppe mit 1 bis 6 Kohlenstoffatomen, und vorzugsweise 1 bis 4 Kohlenstoffatomen, bedeuten, oder zusammen mit dem Stickstoff einen stickstoffhältigen, 5- oder 6-gliedrigen Heterocyclus, insbesondere eine Piperidino-, Morpholino-, Pyrrolidino-, Pyrrol- oder Pyrrolingruppe, bilden ; und

$n + m + 1$ 1 bis 11 ausmacht und vorzugsweise 3, 5 oder 10 ist.

4. Verbindungen nach einem der Ansprüche 1 bis 3, dadurch gekennzeichnet, daß sie der Formel :

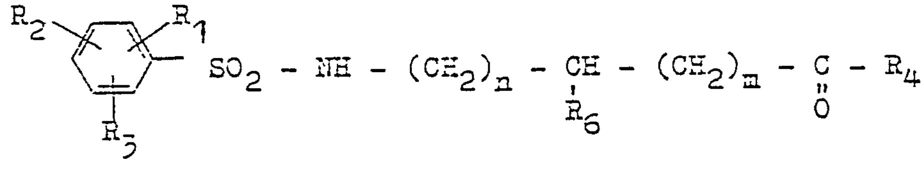

entsprechen, in welcher wenigstens einer der Substituenten $R_1$, $R_2$ und $R_3$ $CF_3$ bedeutet ;

$R_4$ die Hydroxygruppe,

— den $OR_7$-Rest, in welchem $R_7$ eine Alkylgruppe mit 1 bis 6 Kohlenstoffatomen, vorzugsweise 1 bis 4 Kohlenstoffatomen, ist ; oder

— den Rest

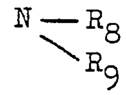

bedeutet, in welchem $R_8$ und $R_9$, die gleich oder verschieden sind, ein Wasserstoffatom, eine Alkylgruppe mit 1 bis 6 Kohlenstoffatomen, vorzugsweise 1 bis 4 Kohlenstoffatomen, bedeuten, oder zusammen mit dem Stickstoff einen stickstoffhältigen, 5- oder 6-gliedrigen Heterocyclus, insbesondere eine Piperidino-, Morpholino-, Pyrrolidino-, Pyrrol- oder Pyrrolingruppe, bilden ; und

$n + m + 1$ 1 bis 11 ausmacht und vorzugsweise 3, 5 oder 10 ist.

5. Verbindungen nach einem der Ansprüche 1 bis 4, dadurch gekennzeichnet, daß sie der Formel :

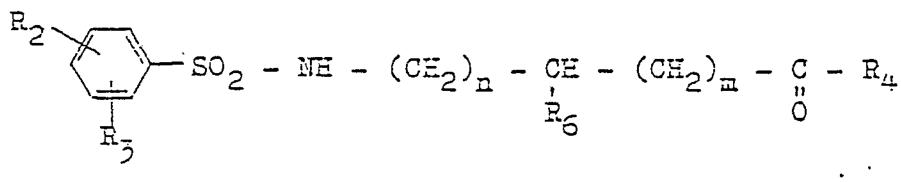

entsprechen, in welcher wenigstens einer der Substituenten $R_2$ oder $R_3$ den $CF_3$-Rest bedeutet ;

$R_4$ die Hydroxygruppe,

— den $OR_7$-Rest, in welchem $R_7$ eine Alkylgruppe mit 1 bis 6 Kohlenstoffatomen, und vorzugsweise 1 bis 4 Kohlenstoffatomen, ist ; oder

— den Rest

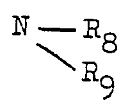

bedeutet, in welchem $R_8$ und $R_9$ gleich oder verschieden sind und ein Wasserstoffatom, eine Alkylgruppe mit 1 bis 6 Kohlenstoffatomen, vorzugsweise 1 bis 4 Kohlenstoffatomen, bedeuten, oder zusammen mit dem Stickstoff einen stickstoffhältigen, 5- oder 6-gliedrigen Heterocyclus, insbesondere eine Piperidino-, Morpholino-, Pyrrolidino-, Pyrrol- oder Pyrrolingruppe, bilden ; und

$n + m + 1$ 1 bis 11 ausmacht und vorzugsweise 3, 5 oder 10 ist.

6. Verbindungen nach einem der Ansprüche 1 bis 5, dadurch gekennzeichnet, daß sie der Formel :

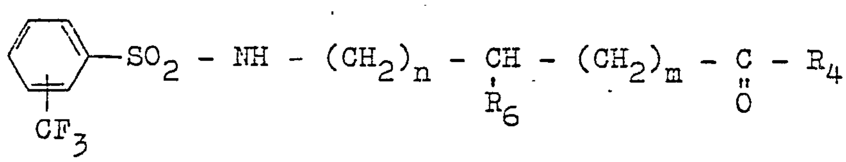

entsprechen, in welcher :

$R_4$ die Hydroxygruppe,

— den $OR_7$-Rest, in welchem $R_7$ eine Alkylgruppe mit 1 bis 6 Kohlenstoffatomen, vorzugsweise 1 bis 4 Kohlenstoffatomen, ist, oder

— den Rest

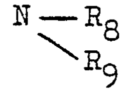

bedeutet, in welchem $R_8$ und $R_9$ gleich oder verschieden sind und ein Wasserstoffatom, eine Alkylgruppe mit 1 bis 6 Kohlenstoffatomen, und vorzugsweise 1 bis 4 Kohlenstoffatomen, bedeuten, oder zusammen mit dem Stickstoff einen stickstoffhältigen, 5- oder 6-gliedrigen Heterocyclus, insbesondere eine piperidino-, Morpholino-, Pyrrolidino-, Pyrrol- oder Pyrrolingruppe, bilden ; und

$n + m + 1$ 1 bis 11 ausmacht und vorzugsweise 3, 5 oder 10 ist.

7. Verbindungen nach Anspruch 1, dadurch gekennzeichnet, daß Ar den cyclischen Rest der Formel :

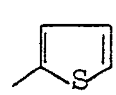

bedeutet, in welchem der in α-Stellung zur $SO_2$-Gruppe befindliche Kohlenstoff gegebenenfalls durch ein Halogenatom, insbesondere durch Chlor, oder durch einen Methylrest substituiert sein kann.

8. Verbindungen nach Anspruch 1, dadurch gekennzeichnet, daß Ar den cyclischen Rest der Formel :

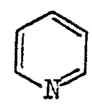

bedeutet, wobei dieser cyclische Rest gegebenenfalls durch $CF_3$ substituiert sein kann.

9. Verbindungen nach Anspruch 1, dadurch gekennzeichnet, daß sie der Formel :

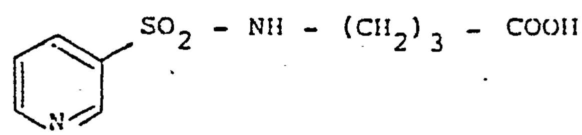

entsprechen.

10. Verbindungen nach Anspruch 1, dadurch gekennzeichnet, daß sie der Formel :

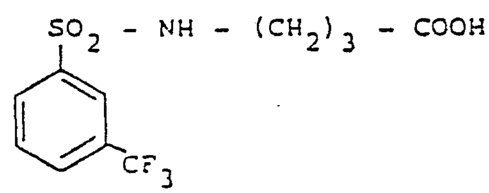

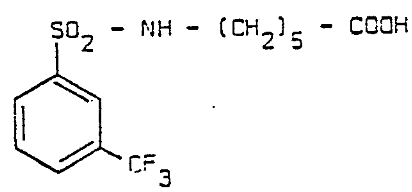

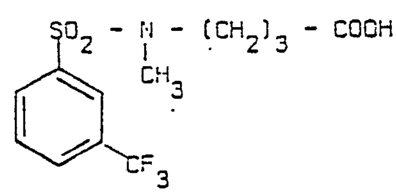

entsprechen.

11. Verbindungen nach Anspruch 1, dadurch gekennzeichnet, daß sie der Formel :

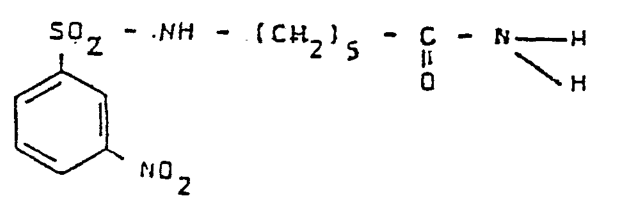

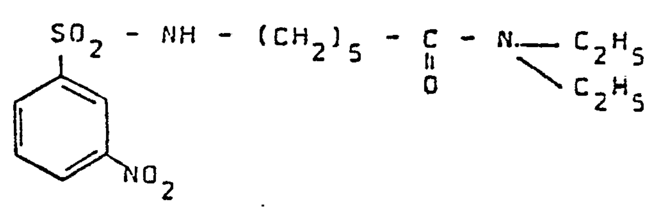

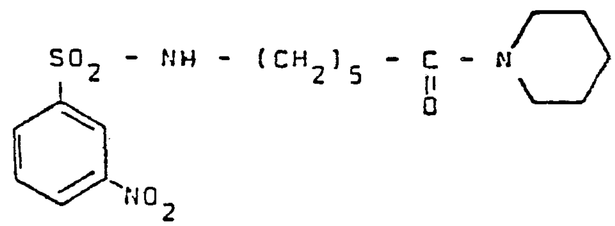

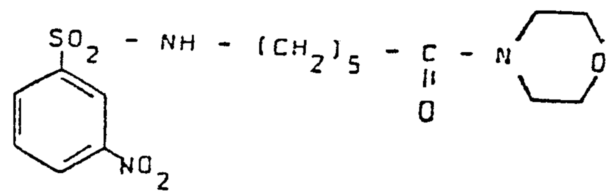

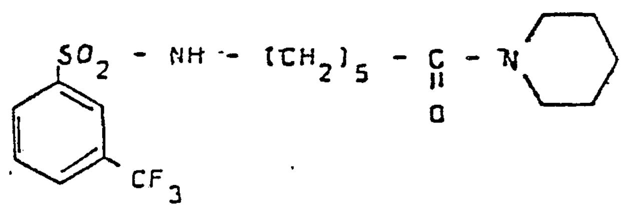

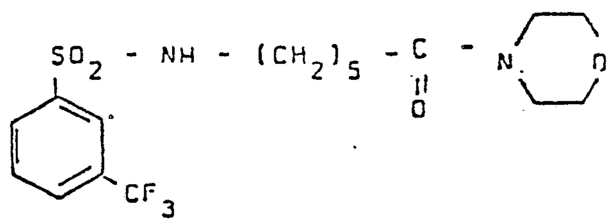

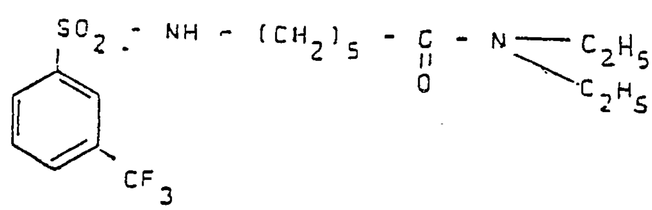

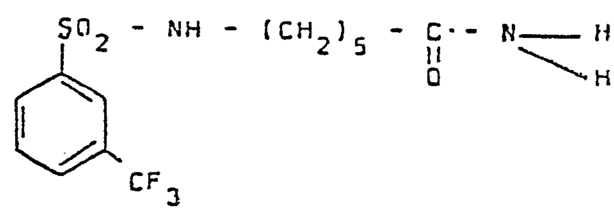

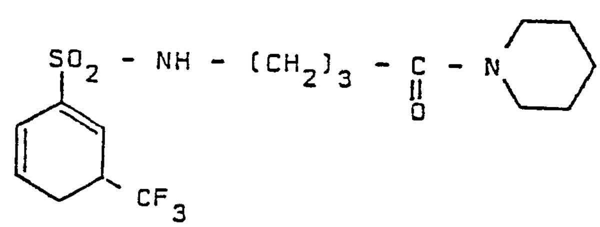

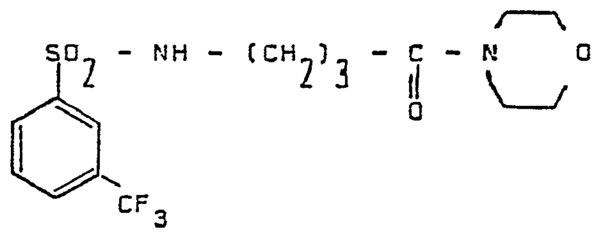

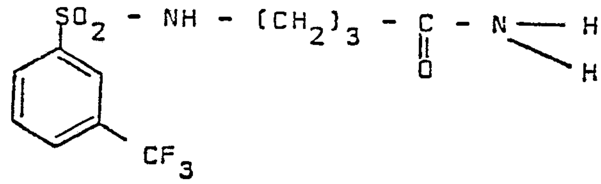

entsprechen.

12. Arzneimittel, dadurch gekennzeichnet, daß sie als Wirksubstanz wenigstens eine Verbindung gemäß einem der Ansprüche 1 bis 11 enthalten.

13. Arzneimittel, das normolipidämische Eigenschaften aufweist, dadurch gekennzeichnet, daß es als Wirksubstanz wenigstens eine Verbindung (oder ihr entsprechendes optisches Isomere) vom Arylsulfonamidtypus enthält, die der Formel :

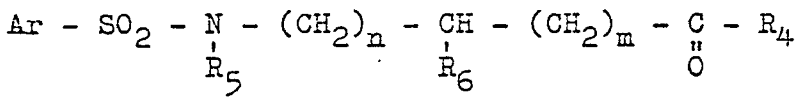 (I)

entspricht, in welcher :

Ar einen der folgenden cyclischen Reste bedeutet :

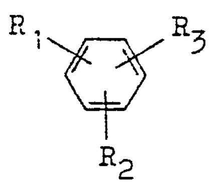 , 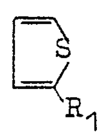 , 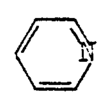

wobei die Substituenten $R_1$, $R_2$ und $R_3$ gleich oder verschieden sind und ein Wasserstoffatom, ein Halogenatom, den $NO_2$-Rest, den $NH_2$-Rest, den $CF_3$-Rest, eine Alkylgruppe mit 1 bis 6 Kohlenstoffatomen, vorzugsweise 1 bis 4 Kohlenstoffatomen, eine Alkoxygruppe mit 1 bis 6 Kohlenstoffatomen, und vorzugsweise 1 bis 4 Kohlenstoffatomen, eine Säuregruppe, eine Estergruppe mit 2 bis 7 Kohlenstoffatomen, und vorzugsweise 2 bis 5 Kohlenstoffatomen, bedeuten ;

die Summe n + m + 1 3 bis 11 ausmacht und vorzugsweise gleich 3, 5 oder 10 ist, wobei diese Summe auch 1 oder 2 sein kann, falls Ar einen Benzolkern enthält, von dem wenigstens einer der Substituenten $R_1$, $R_2$ und $R_3$ eine $CF_3$-Gruppe ist ;

$R_5$ und $R_6$ unabhängig voneinander ein Wasserstoffatom, einen Alkylrest mit 1 bis 6 Kohlenstofatomen, vorzugsweise 1 bis 4 Kohlenstoffatomen, oder einen Aralkylrest mit 7 bis 9 Kohlenstoffatomen bedeuten ;

$R_4$ die Hydroxygruppe,

— den $OR_7$-Rest, in welchem $R_7$ eine Alkylgruppe mit 1 bis 6 Kohlenstoffatomen, und vorzugsweise 1 bis 4 Kohlenstoffatomen, ist,

— den

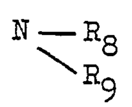

Rest bedeutet, in welchem

$R_8$ und $R_9$, die gleich oder verschieden sind, ein Wasserstoffatom, eine Alkylgruppe mit 1 bis 6 Kohlenstoffatomen, und vorzugsweise 1 bis 4 Kohlenstoffatomen, bedeuten, oder zusammen mit dem Stickstoff einen stickstoffhältigen, 5- oder 6-gliedrigen Heterocyclus, insbesondere eine Piperidino-, Morpholino-, Pyrrolidino, Pyrrol- oder Pyrrolingruppe, bilden.

14. Arzneimittel nach Anspruch 13, dadurch gekennzeichnet, daß wenigstens einer der Reste $R_5$ oder $R_6$ Wasserstoff bedeutet.

15. Arzneimittel nach einem der Ansprüche 13 oder 14, dadurch gekennzeichnet, daß der substituierte Benzolkern die Formel :

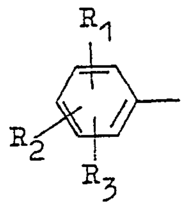

hat, in welcher $R_1$, $R_2$ und $R_3$ gleichzeitig ein Wasserstoffatom bedeuten, oder gleichzeitig von Wasserstoff verschieden sind und aus der Gruppe ausgewählt sind, die aus einem Halogenatom, dem $NO_2$-Rest, dem $NH_2$-Rest, dem $CF_3$-Rest, einer Alkylgruppe mit 1 bis 6 Kohlenstoffatomen, und vorzugsweise 1 bis 4 Kohlenstoffatomen, einer Alkoxygruppe mit 1 bis 6 Kohlenstoffatomen, und vorzugsweise 1 bis 4 Kohlenstoffatomen, einer Säuregruppe, einer Estergruppe mit 2 bis 7 Kohlenstoffatomen, und vorzugsweise 2 bis 5 Kohlenstoffatomen, besteht.

16. Arzneimittel nach Anspruch 15, dadurch gekennzeichnet, daß $R_1$, $R_2$ und $R_3$ gleichzeitig ein Wasserstoffatom bedeuten.

17. Arzneimittel nach Anspruch 15, dadurch gekennzeichnet, daß es der folgenden Formel (III) entspricht :

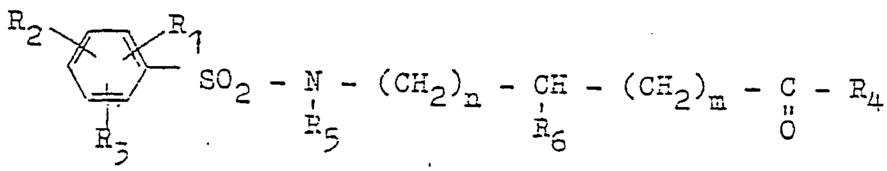

(III)

in welcher $R_1$, $R_2$ und $R_3$ gleichzeitig von Wasserstoff verschieden sind.

18. Arzneimittel nach Anspruch 17, dadurch gekennzeichnet, daß der Benzolkern durch Reste trisubstituiert ist, die aus der $NO_2$, $NH_2$, $OCH_3$, $CH_3$, ein Halogenatom, und insbesondere Chlor, umfassenden Gruppe ausgewählt sind.

19. Arzneimittel nach einem der Ansprüche 13 bis 15, dadurch gekennzeichnet, daß es der Formel (III) entspricht, in welcher :

$R_1$ ein Wasserstoffatom bedeutet und

$R_2$ und $R_3$ ein Halogenatom, den $NO_2$-Rest, den $NO_2$-Rest, eine Alkylgruppe mit 1 bis 6 Kohlenstoffatomen, und vorzugsweise mit 1 bis 4 Kohlenstoffatomen, eine Alkoxygruppe mit 1 bis 6 Kohlenstoffatomen, und vorzugsweise 1 bis 4 Kohlenstoffatomen, eine Säuregruppe, eine Estergruppe mit 2 bis 7 Kohlenstoffatomen, und vorzugsweise 2 bis 5 Kohlenstoffatomen, bedeuten.

20. Arzneimittel nach Anspruch 19, dadurch gekennzeichnet, daß der Benzolkern durch Reste substituiert ist, die aus der aus $NO_2$, $NH_2$, $OCH_3$ oder aus einem Halogen, insbesondere Chlor, bestehenden Gruppe ausgewählt sind.

21. Arzneimittel nach einem der Ansprüche 13 bis 15, dadurch gekennzeichnet, daß es der allgemeinen Formel (III) entspricht, in welcher :

$R_1$ und $R_2$ gleichzeitig ein Wasserstoffatom bedeuten ; und

$R_3$ ein Halogenatom, den $NO_2$-Rest, den $NH_2$-Rest, eine Alkylgruppe mit 1 bis 6 Kohlenstoffatomen, und vorzugsweise mit 1 bis 4 Kohlenstoffatomen, eine Alkoxygruppe mit 1 bis 6 Kohlenstoffatomen, und vorzugsweise 1 bis Kohlenstoffatomen, eine Säuregruppe, eine Estergruppe mit 2 bis 7 Kohlenstoffatomen, und vorzugsweise 2 bis 5 Kohlenstoffatomen, bedeutet.

22. Arzneimittel nach Anspruch 13, dadurch gekennzeichnet, daß Ar den cyclischen Rest der Formel :

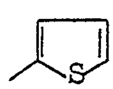

bedeutet, in welchem der Kohlenstoff in α-Stellung zur $SO_2$-Gruppe gegebenenfalls durch ein Halogenatom, insbesondere durch Chlor, oder durch einen Methylrest, substituiert sein kann.

23. Arzneimittel nach Anspruch 13, dadurch gekennzeichnet, daß Ar den cyclischen Rest der Formel :

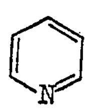

bedeutet, wobei dieser cyclische Rest gegebenenfalls durch $CF_3$ substituiert sein kann.

24. Arzneimittel nach Anspruch 13, dadurch gekennzeichnet, daß es als Wirksubstanz eine der Verbindungen der Formel :

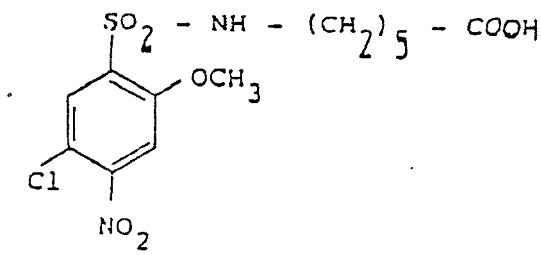

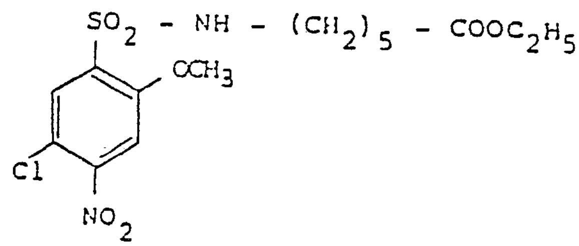

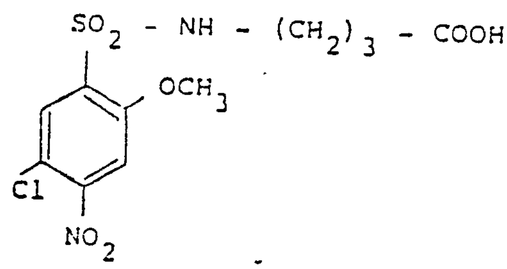

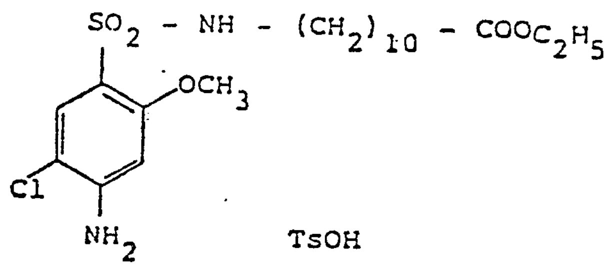

TsOH

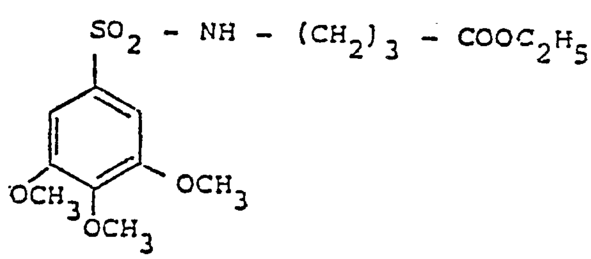

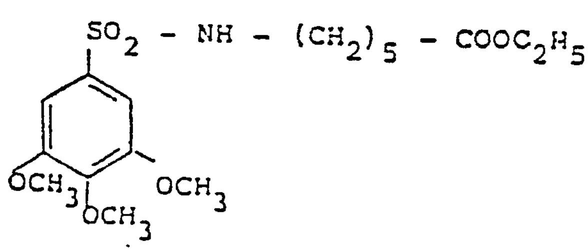

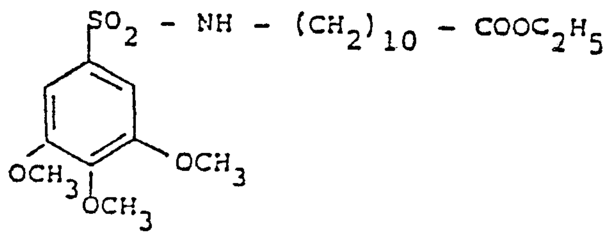

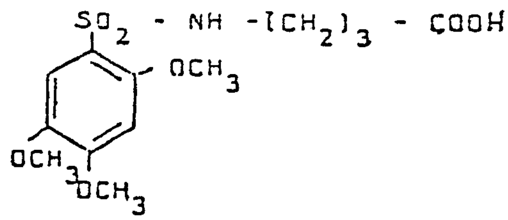

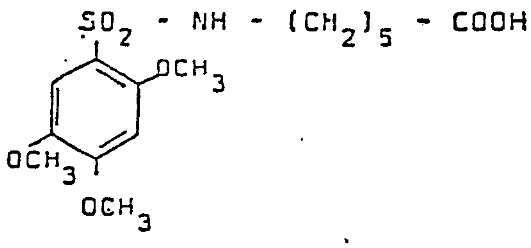

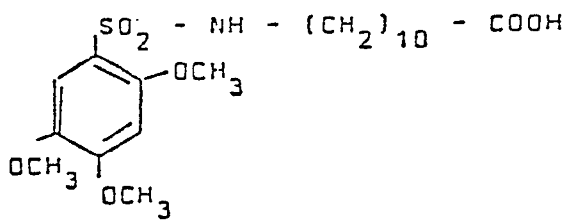

enthält.

25. Arzneimittel nach Anspruch 13, dadurch gekennzeichnet, daß es als Wirksubstanz eine der Verbindungen der Formel :

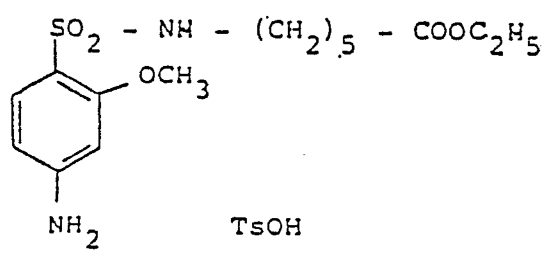

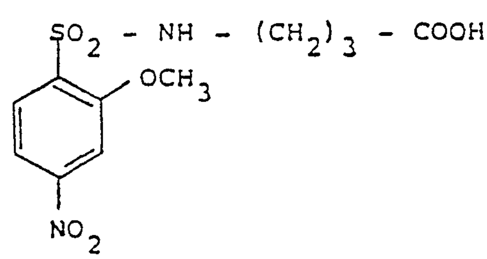

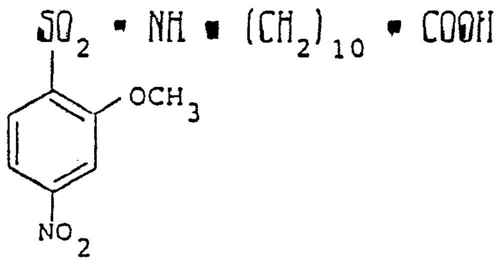

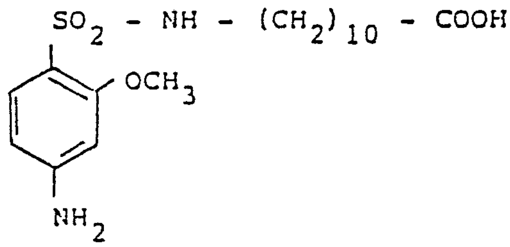

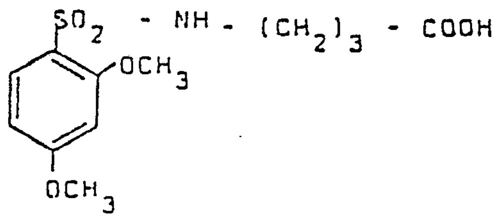

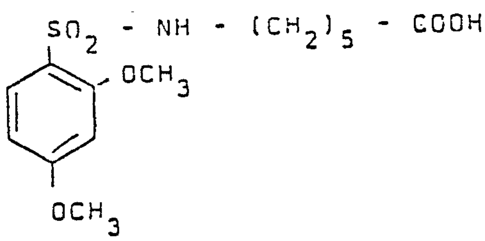

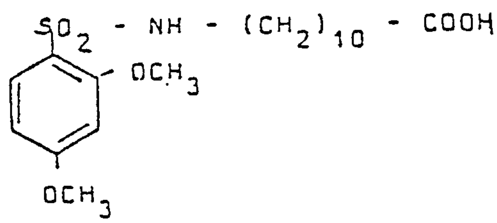

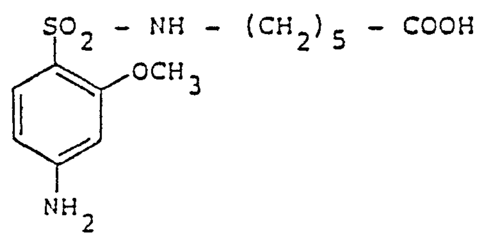

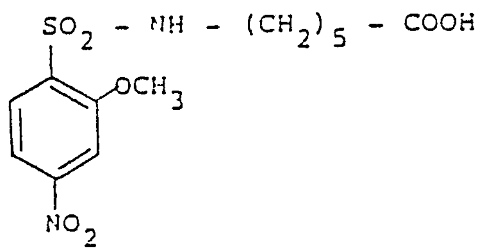

enthält.

26. Arzneimittel nach Anspruch 13, dadurch gekennzeichnet, daß es als Wirksubstanz eine der Verbindungen der Formel :

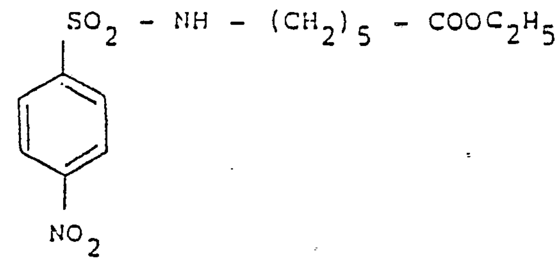

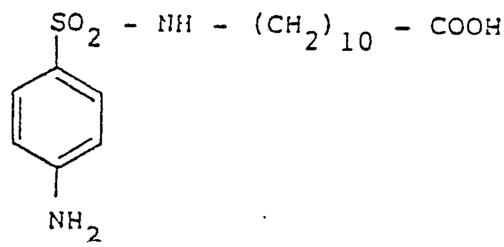

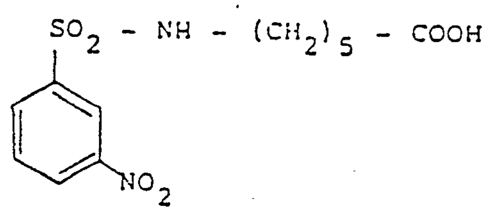

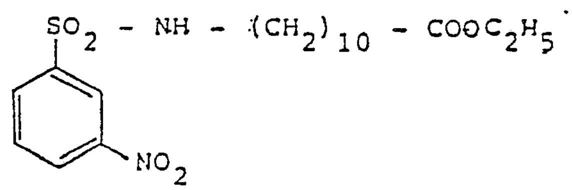

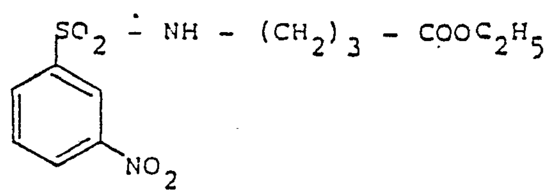

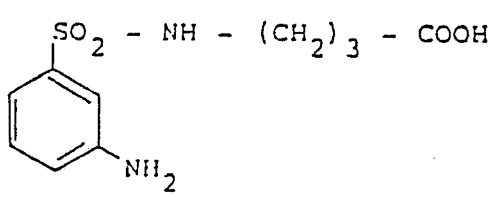

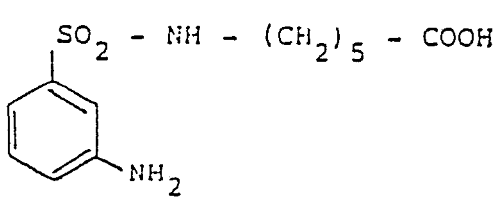

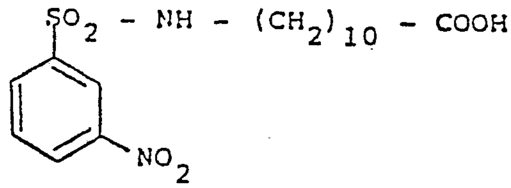

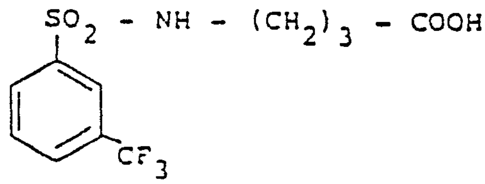

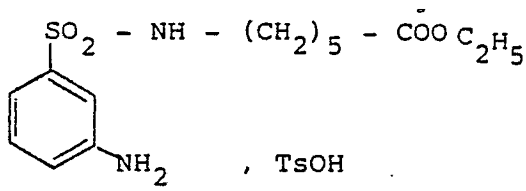

, TsOH

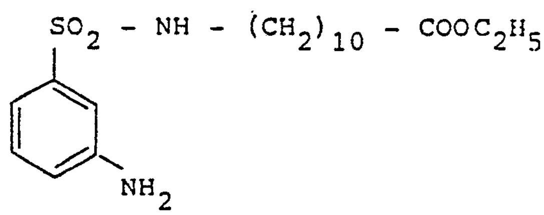

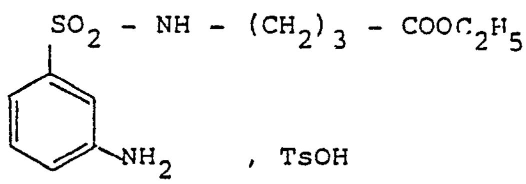

, TsOH

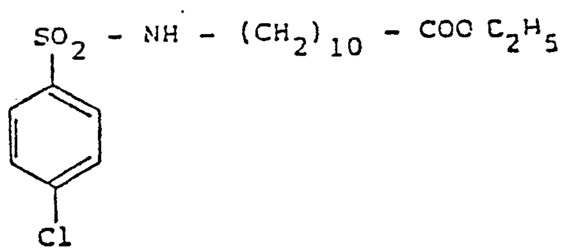

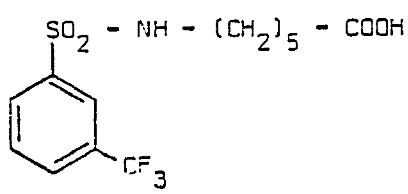

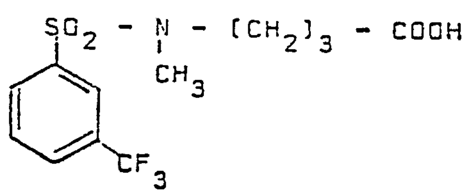

enthält.

27. Arzneimittel nach Anspruch 13, dadurch gekennzeichnet, daß es als Wirksubstanz eine Verbindung der Formel :

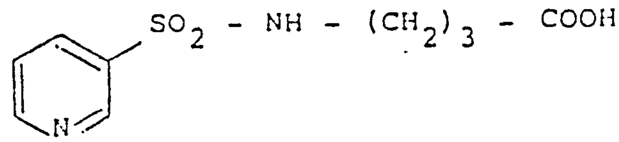

enthält.

28. Arzneimittel nach Anspruch 13, dadurch gekennzeichnet, daß es als Wirksubstanz eine der Verbindungen der Formel :

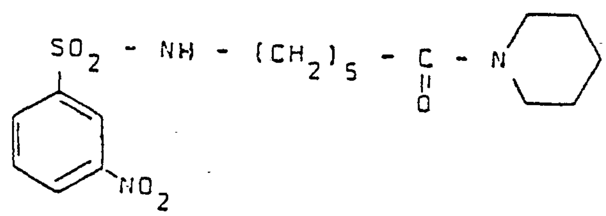

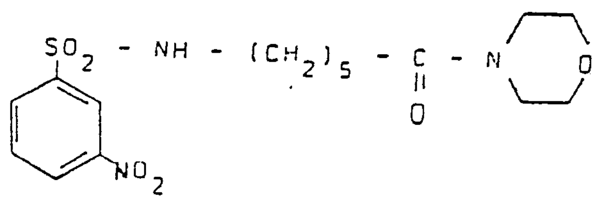

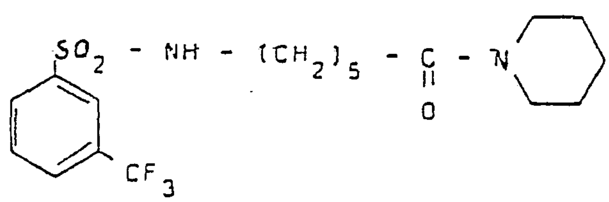

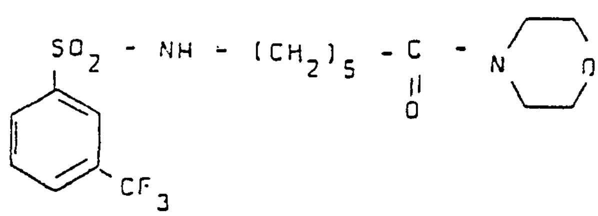

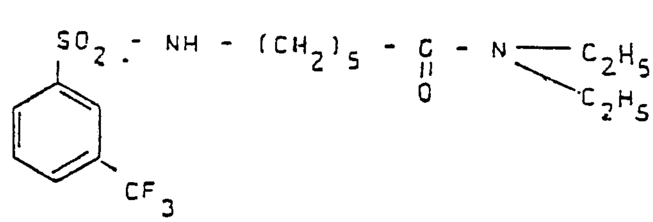

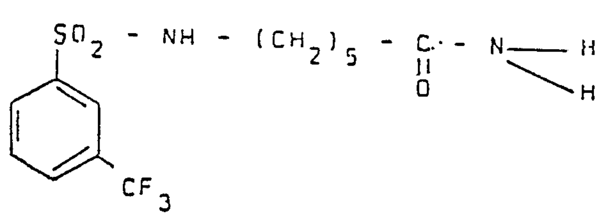

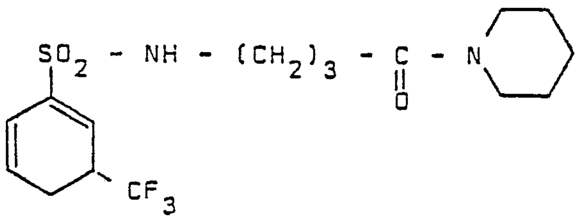

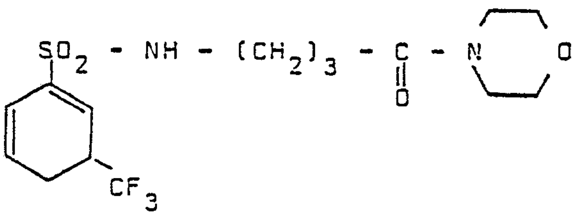

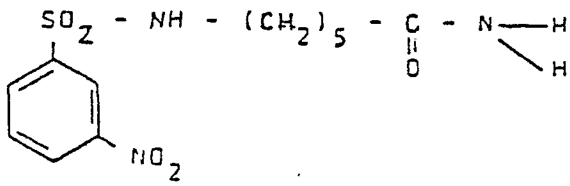

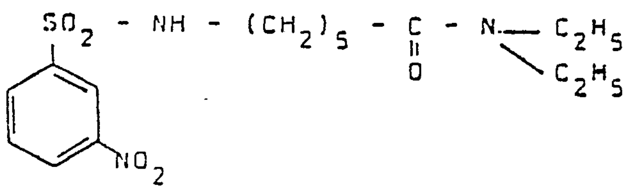

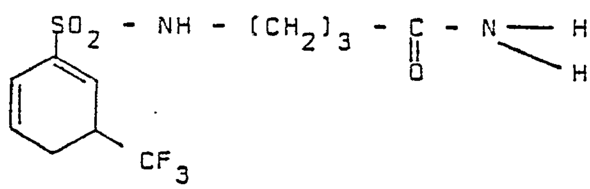

enthält.

29. Pharmazeutisch annehmbare Salze, insbesondere Tosylat und Hydrochlorid, von Verbindungen und Arzneimitteln nach einem der Ansprüche 1 bis 28.

30. Arzneimittel nach einem der Ansprüche 12 bis 29, zur Verabreichung auf oralem Wege, dadurch gekennzeichnet, daß es etwa 10 mg bis etwa 500 mg, vorzugsweise etwa 50 bis 250 mg, an Wirksubstanz enthält.

31. Arzneimittel nach einem der Ansprüche 12 bis 29, zur Verabreichung auf topischem Wege, dadurch gekennzeichnet, daß die Zubereitung 1 bis 20 Gew.-% an Wirksubstanz enthält.

**Patentansprüche** (für den Vertragsstaat AT)

1. Verfahren zur Herstellung von für die Herstellung von Arzneimitteln brauchbaren Verbindungen der Formel :

$$Ar - SO_2 - \underset{R_5}{\underset{|}{N}} - (CH_2)_n - \underset{R_6}{\underset{|}{CH}} - (CH_2)_m - \underset{O}{\underset{\|}{C}} - R_4 \qquad (I)$$

sowie der optischen Isomeren der Verbindungen der Formel I, in welcher :

Ar einen der folgenden cyclischen Reste bedeutet :

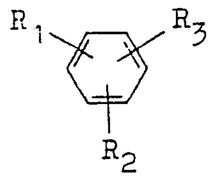

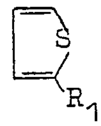

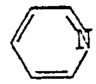

die Substituenten $R_1$, $R_2$ und $R_3$ gleich oder verschieden sind und ein Wasserstoffatom, ein halogenatom, den $NO_2$-Rest, den $NH_2$-Rest, den $CF_3$-Rest, eine Alkylgruppe mit 1 bis 6 Kohlenstoffatomen, vorzugsweise 1 bis 4 Kohlenstoffatomen, eine Alkoxygruppe mit 1 bis 6 Kohlenstoffatomen, und vorzugsweise 1 bis 4 Kohlenstoffatomen, eine Säuregruppe, eine Ester gruppe mit 2 bis 7 Kohlenstoffatomen, und vorzugsweise 2 bis 5 Kohlenstoffatomen, bedeuten ;

die Summe n + m + 1 3 bis 11 ausmacht und vorzugsweise gleich 3, 5 oder 10 ist, wobei diese Summe auch 1 oder 2 sein kann, falls Ar einen Benzolkern enthält, von dem wenigstens einer der Substituenten $R_1$, $R_2$ und $R_3$ eine $CF_3$-Gruppe ist ;

$R_5$ und $R_6$ unabhängig voneinander ein Wasserstoffatom, einen Alkylrest mit 1 bis 6 Kohlenstoffatomen, vorzugsweise 1 bis 4 Kohlenstoffatomen, oder einen Aralkylrest mit 7 bis 9 Kohlenstoffatomen bedeuten ;

$R_4$ die Hydroxygruppe,

— den $OR_7$-Rest, in welchem $R_7$ eine Alkylgruppe mit 1 bis 6 Kohlenstoffatomen, und vorzugsweise 1 bis 4 Kohlenstoffatomen, ist,

— oder den

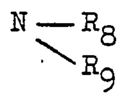

Rest bedeutet, in welchem $R_8$ und $R_9$, die gleich oder verschieden sind, ein Wasserstoffatom, eine Alkylgruppe mit 1 bis 6 Kohlenstoffatomen, und vorzugsweise 1 bis 4 Kohlenstoffatomen, bedeuten, oder zusammen mit dem Stickstoff einen stickstoffhältigen, 5- oder 6-gliedrigen Heterocyclus, insbesondere eine Piperidino-, Morpholino-, Pyrrolidino-, Pyrrol- oder Pyrrolingruppe, bilden, dadurch gekennzeichnet, daß man :

falls $R_4$ den $OR_7$-Rest bedeutet, wobei $R_7$ eine Alkylgruppe mit 1 bis 6 Kohlenstoffatomen ist,

— entweder ein Säurehalogenid, insbesondere ein Säurechlorid der Formel :

$$ArSO_2Cl$$

mit einem Aminoester der Formel :

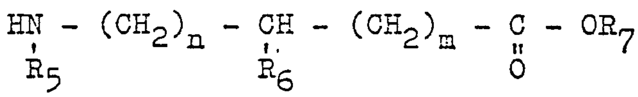

oder seinem Hydrochlorid der Formel :

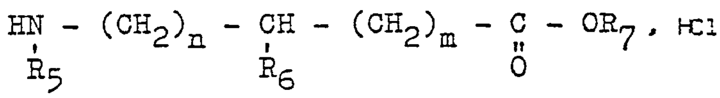

zur Gewinnung der Verbindung der Formel :

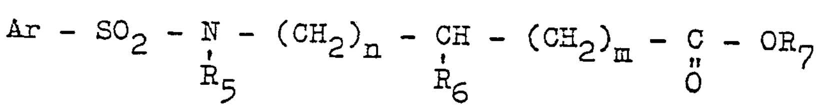

umsetzt

— oder ein Säurehalogenid, insbesondere ein Säurechlorid der Formel :

$$ArSO_2Cl$$

mit einer Aminosäure der Formel :

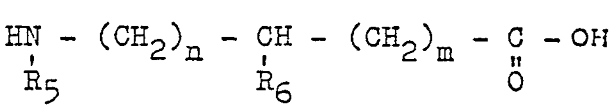

umsetzt und dann die so erhaltene Säure verestert, indem man sie mit einem entsprechenden Alkohol der Formel : $R_7OH$ zur Gewinnung der Verbindung der Formel :

$$Ar-SO_2-\underset{R_5}{N}-(CH_2)_n-\underset{R_6}{CH}-(CH_2)_m-\underset{O}{\overset{}{C}}-OR_7$$

umsetzt,

— oder eine Säure der Formel :

$$ArSO_2H$$

mit einem Aminoester der Formel :

$$\underset{R_5}{HN}-(CH_2)_n-\underset{R_6}{CH}-(CH_2)_m-\underset{O}{C}-OR_7$$

zur Gewinnung der Verbindung der Formel :

$$Ar-SO_2-\underset{R_5}{N}-(CH_2)_n-\underset{R_6}{CH}-(CH_2)_m-\underset{O}{C}-OR_7$$

umsetzt,

falls $R_4$ den Hydroxyrest bedeutet,

— entweder ein Säurechlorid :

$$ArSO_2Cl$$

mit einer Aminosäure der Formel :

$$\underset{R_5}{HN}-(CH_2)_n-\underset{R_6}{CH}-(CH_2)_m-COOH$$

zur Gewinnung der Verbindung der Formel :

$$Ar-SO_2-\underset{R_5}{N}-(CH_2)_n-\underset{R_6}{CH}-(CH_2)_m-COOH$$

umsetzt,

— oder in einer ersten Stufe einen Ester der Säure, die man erhalten möchte, herstellt, indem man ein Säurehalogenid, insbesondere ein Säurechlorid der Formel :

$$ArSO_2Cl$$

mit einer Verbindung der Formel :

$$\underset{R_5}{HN}-(CH_2)_n-\underset{R_6}{CH}-(CH_2)_m-COOR_7$$

zur Gewinnung der Verbindung der Formel :

$$Ar-SO_2-\underset{R_5}{N}-(CH_2)_n-\underset{R_6}{CH}-(CH_2)_m-COOR_7$$

umsetzt, und dann in einer zweiten Stufe den erhaltenen Ester zu seiner entsprechenden Säure der Formel :

$$Ar-SO_2-\underset{R_5}{N}-(CH_2)_n-\underset{R_6}{CH}-(CH_2)_m-COOH$$

hydrolysiert,

falls $R_4$ OH oder einen Alkoxyrest mit 1 bis 6 Kohlenstoffatomen bedeutet, wie oben angegeben die Verbindung der Formel :

$$Ar - SO_2 - NH - (CH_2)_n - \underset{\substack{|\\R_6}}{CH} - (CH_2)_m - COR_4$$

herstellt und sie mit einem Alkylhalogenid, wie einem Alkyliodid $R_5I$, zur Gewinnung der Verbindung der Formel :

$$Ar - SO_2 - \underset{\substack{|\\R_5}}{N} - (CH_2)_n - \underset{\substack{|\\R_6}}{CH} - (CH_2)_m - \underset{\substack{\|\\O}}{C} - R_4$$

umsetzt,

— oder falls $R_4$ :

$$N<^{R_8}_{R_9}$$

bedeutet, die Verbindung der Formel :

$$Ar - SO_2 - \underset{\substack{|\\R_5}}{N} - (CH_2)_n - \underset{\substack{|\\R_6}}{CH} - (CH_2)_m - \underset{\substack{\|\\O}}{C} - OH$$

wie oben angegeben herstellt und diese mit Thionylchlorid $SO_2Cl$ zur Gewinnung der Verbindung der Formel :

$$Ar - SO_2 - \underset{\substack{|\\R_5}}{N} - (CH_2)_n - \underset{\substack{|\\R_6}}{CH} - (CH_2)_m - \underset{\substack{\|\\O}}{C} - Cl$$

umsetzt und dann das Amin der Formel :

$$HN<^{R_8}_{R_9}$$

mit dem oben erwähnten Säurechlorid zur Gewinnung der Verbindung der Formel :

$$Ar - SO_2 - \underset{\substack{|\\R_5}}{N} - (CH_2)_n - \underset{\substack{|\\R_6}}{CH} - (CH_2)_m - \underset{\substack{\|\\O}}{C} - N<^{R_8}_{R_9}$$

zur Umsetzung bringt.

2. Verfahren zur Herstellung von für die Herstellung von Arzneimitteln brauchbaren Verbindungen nach Anspruch 1, dadurch gekennzeichnet, daß wenigstens einer der Reste $R_5$ oder $R_6$ Wasserstoff bedeutet.

3. Verfahren zur Herstellung von für die Herstellung von Arzneimitteln brauchbaren Verbindungen nach Anspruch 1 oder 2, dadurch gekennzeichnet, daß der substituierte Benzolkern die Formel :

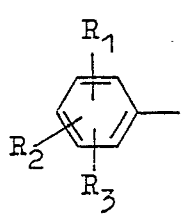

hat, in welcher $R_1$, $R_2$ und $R_3$ gleich oder verschieden sind und ein Wasserstoffatom bedeuten, oder gleichzeitig von Wasserstoff verschieden sind und aus der Gruppe ausgewählt sind, die aus einem Halogenatom, dem $NO_2$-Rest, dem $NH_2$-Rest, dem $CF_3$-Rest, einer Alkylgruppe mit 1 bis 6 Kohlenstoffatomen, und vorzugsweise 1 bis 4 Kohlenstoffatomen, einer Alkoxygruppe mit 1 bis 6 Kohlenstoffatomen, und vorzugsweise 1 bis 4 Kohlenstoffatomen, einer Säuregruppe, einer Estergruppe mit 2 bis 7 Kohlenstoffatomen, und vorzugsweise 2 bis 5 Kohlenstoffatomen, besteht.

4. Verfahren zur Herstellung von für die Herstellung von Arzneimitteln brauchbaren Verbindungen nach Anspruch 3, dadurch gekennzeichnet, daß $R_1$, $R_2$ und $R_3$ gleichzeitig ein Wasserstoffatom bedeuten.

5. Verfahren zur Herstellung von für die Herstellung von Arzneimitteln brauchbaren Verbindungen nach Anspruch 3, dadurch gekennzeichnet, daß sie der folgenden Formel entsprechen :

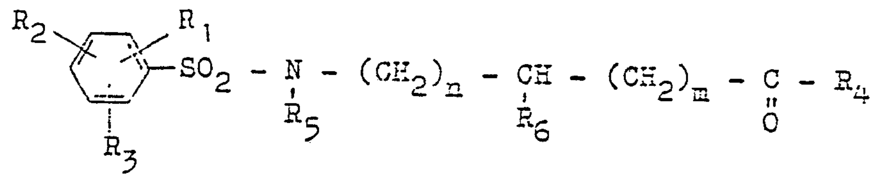

(III)

in welcher $R_1$, $R_2$ und $R_3$ gleichzeitig von Wasserstoff verschieden sind.

6. Verfahren zur Herstellung von für die Herstellung von Arzneimitteln brauchbaren Verbindungen nach Anspruch 5, dadurch gekennzeichnet, daß der Benzolkern durch Reste trisubstituiert ist, die aus der $NO_2$, $NH_2$, $OCH_3$, $CH_3$, ein Halogenatom, und insbesondere Chlor, umfassenden Gruppe ausgewählt sind.

7. Verfahren zur Herstellung von für die Herstellung von Arzneimitteln brauchbaren Verbindungen nach den Ansprüchen 1 bis 3, dadurch gekennzeichnet, daß sie der Formel (III) entsprechen, in welcher

$R_1$ ein Wasserstoffatom bedeutet und

$R_2$ und $R_3$ ein Halogenatom, den $NO_2$-Rest, eine Alkylgruppe mit 1 bis 6 Kohlenstoffatomen, und vorzugsweise mit 1 bis 4 Kohlenstoffatomen, eine Alkoxygruppe mit 1 bis 6 Kohlenstoffatome, und vorzugsweise 1 bis 4 Kohlenstoffatomen, eine Säuregruppe, eine Estergruppe mit 2 bis 7 Kohlenstoffatomen, und vorzugsweise 2 bis 5 Kohlenstoffatomen, bedeuten.

8. Verfahren zur Herstellung von für die herstellung von Arzneimitteln brauchbaren Verbindungen nach Anspruch 7, dadurch gekennzeichnet, daß der Benzolkern durch Reste substituiert ist, die aus der aus $NO_2$, $NH_2$, $OCH_3$ oder aus einem Halogen, insbesondere Chlor, bestehenden Gruppe ausgewählt sind.

9. Verfahren zur Herstellung von für die Herstellung von Arzneimitteln brauchbaren Verbindungen nach den Ansprüchen 1 bis 3, dadurch gekennzeichnet, daß sie der allgemeinen Formel (III) entsprechen, in welcher

$R_1$ und $R_2$ gleichzeitig ein Wasserstoffatom bedeuten ; und

$R_3$ ein Halogenatom, den $NO_2$-Rest, den $NH_2$-Rest, eine Alkylgruppe mit 1 bis 6 Kohlenstoffatomen, und vorzugsweise mit 1 bis 4 Kohlenstoffatomen, eine Alkoxygruppe mit 1 bis 6 Kohlenstoffatomen, und vorzugsweise 1 bis 4 Kohlenstoffatomen, eine Säuregruppe, eine Estergruppe mit 2 bis 7 Kohlenstoffatomen, und vorzugsweise 2 bis 5 Kohlenstoffatomen, bedeutet.

10. Verfahren zur Herstellung von für die Herstellung von Arzneimitteln brauchbaren Verbindungen nach Anspruch 1, dadurch gekennzeichnet, daß Ar den cyclischen Rest der Formel :

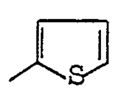

bedeutet, in welchem der Kohlenstoff in α-Stellung zur $SO_2$-Gruppe gegebenenfalls durch ein Halogenatom, insbesondere durch Chlor, oder durch einen Methylrest, substituiert sein kann.

11. Verfahren zur Herstellung von für die Herstellung von Arzneimitteln brauchbaren Verbindungen nach Anspruch 1, dadurch gekennzeichnet, daß Ar den cyclischen Rest der Formel :

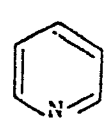

bedeutet, wobei dieser cyclische Rest gegebenenfalls durch $CF_3$ substituiert sein kann.

12. Verfahren zur Herstellung von für die Herstellung von Arzneimitteln brauchbaren Verbindungen nach Anspruch 1, dadurch gekennzeichnet, daß sie als Wirksubstanz eine der Verbindungen der Formel :

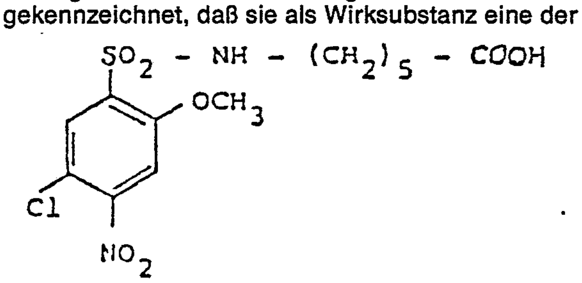

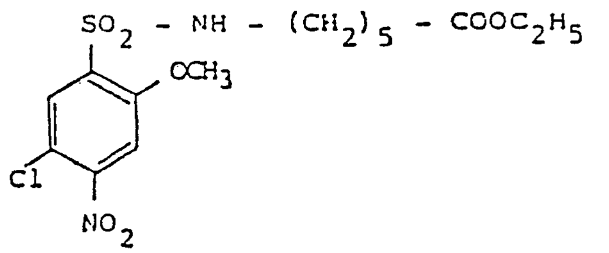

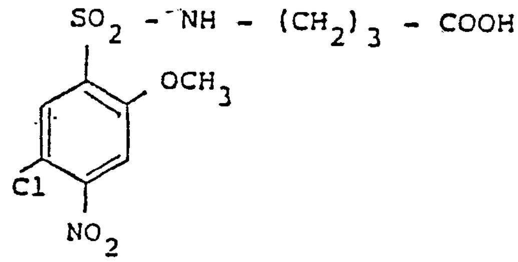

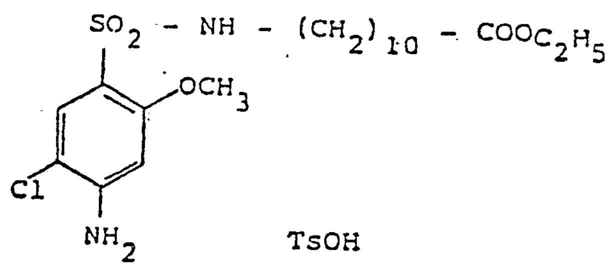

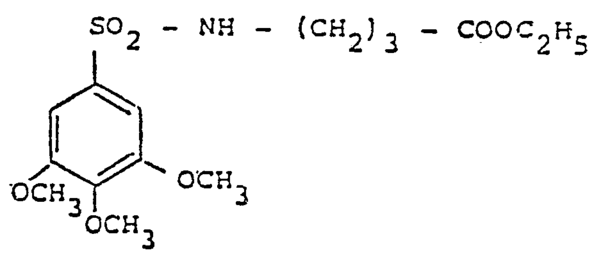

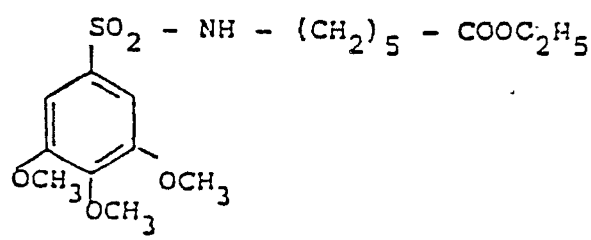

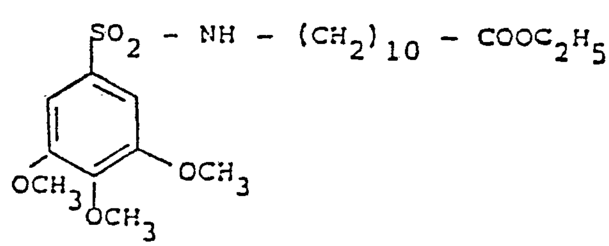

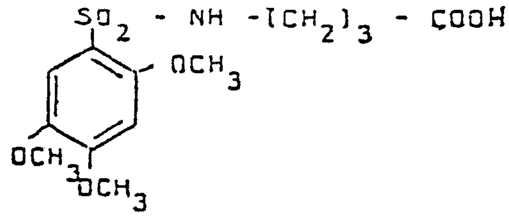

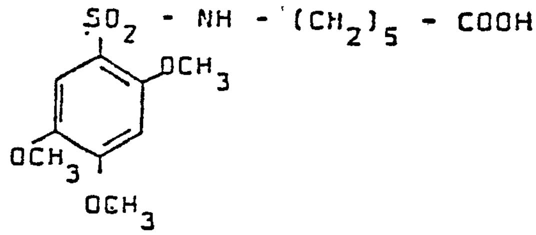

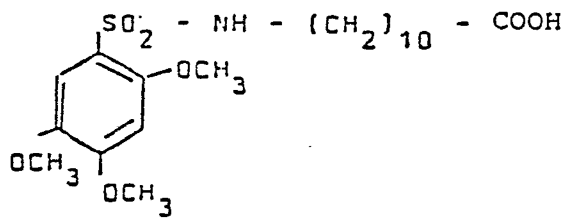

enthalten.

13. Verfahren zur Herstellung von für die Herstellung von Arzneimitteln brauchbaren Verbindungen nach Anspruch 1, dadurch gekennzeichnet, daß sie als Wirksubstanz eine der Verbindungen der Formel :

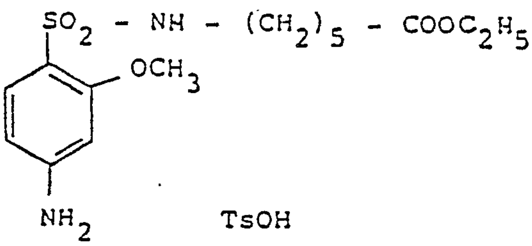

TsOH

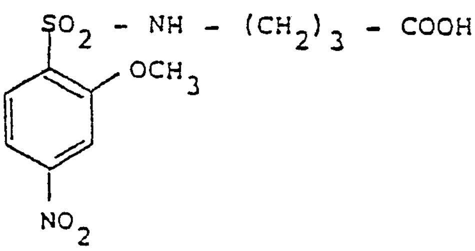

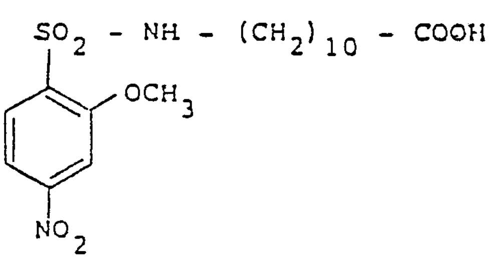

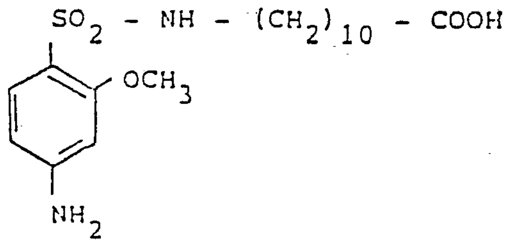

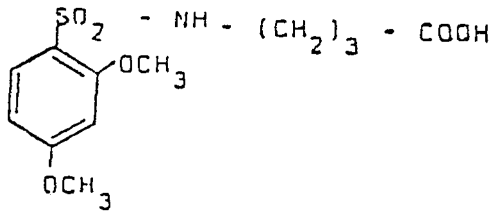

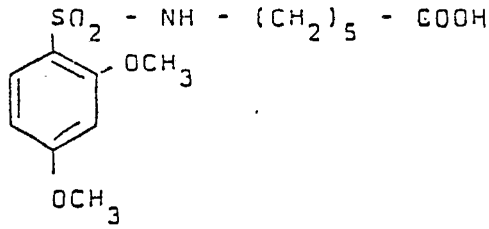

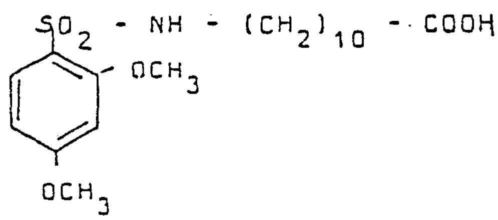

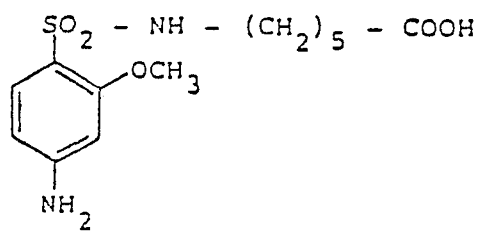

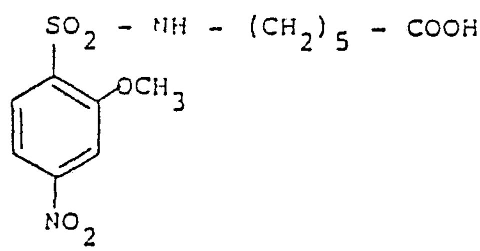

enthalten.

14. Verfahren zur Herstellung von für die Herstellung von Arzneimitteln brauchbaren Verbindungen nach Anspruch 1, dadurch gekennzeichnet, daß sie als Wirksubstanz eine der Verbindungen der Formel :

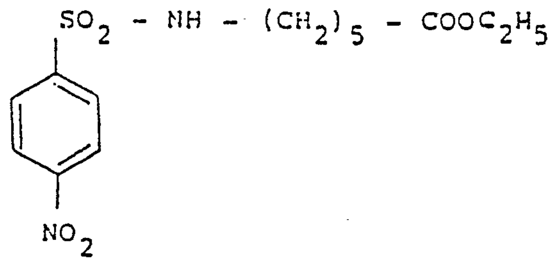

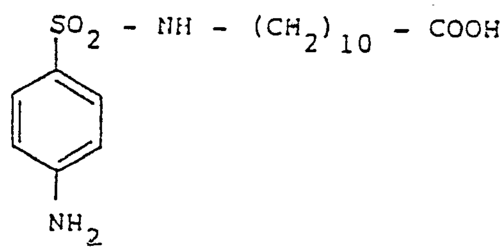

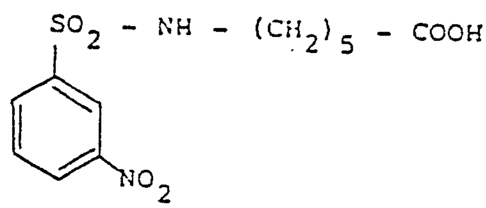

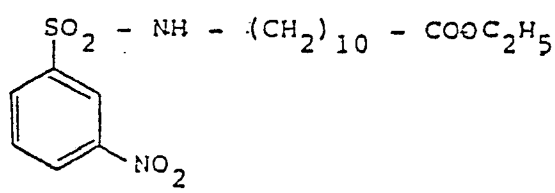

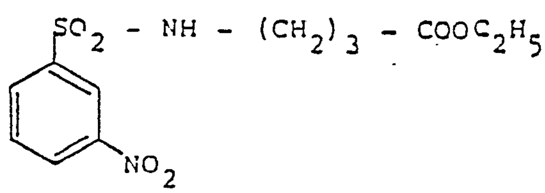

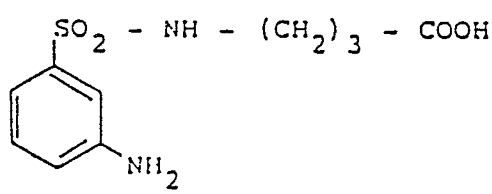

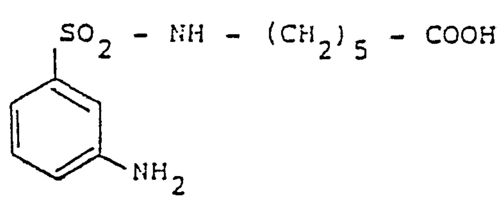

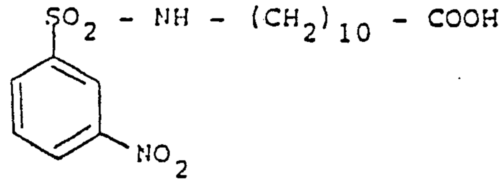

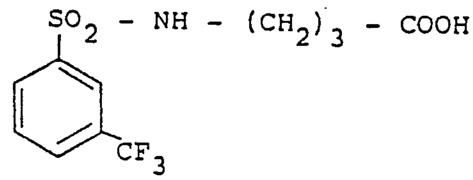

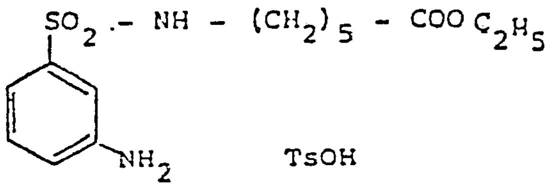

TsOH

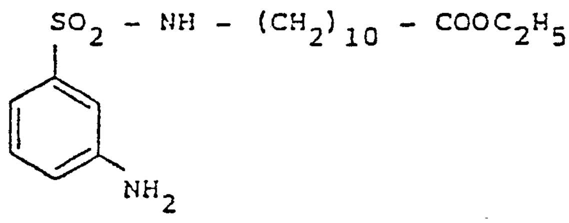

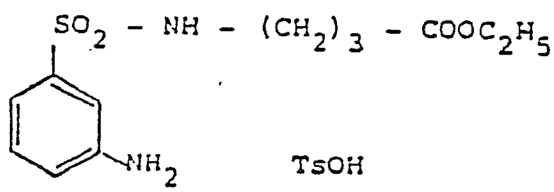

TsOH

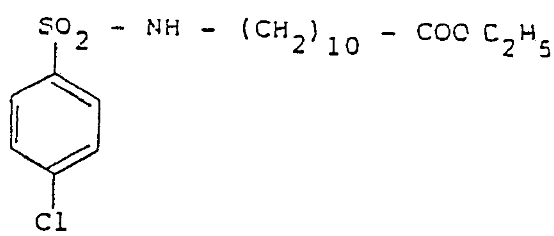

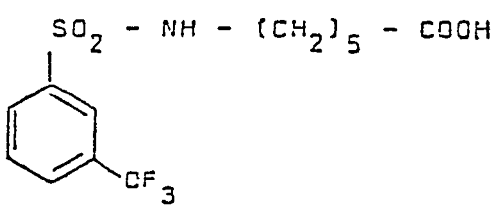

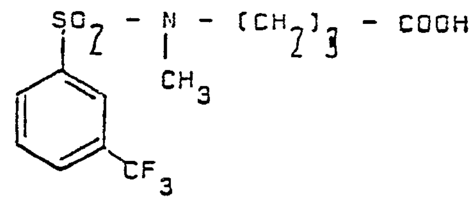

enthalten.

15. Verfahren zur Herstellung von für die Herstellung von Arzneimitteln brauchbaren Verbindungen nach Anspruch 1, dadurch gekennzeichnet, daß sie als Wirksubstanz eine Verbindung der Formel :

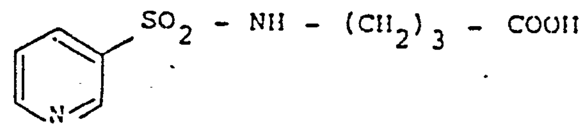

enthalten.

16. Verfahren zur Herstellung von für die Herstellung von Arzneimitteln brauchbaren Verbindungen nach Anspruch 1, dadurch gekennzeichnet, daß sie als Wirksubstanz eine der Verbindungen der Formel :

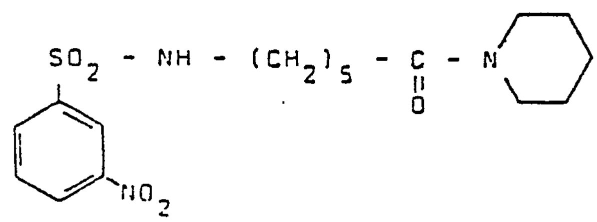

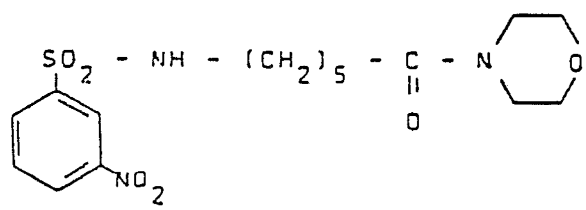

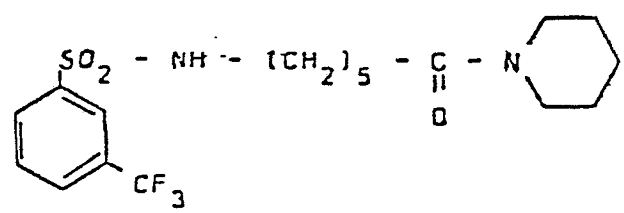

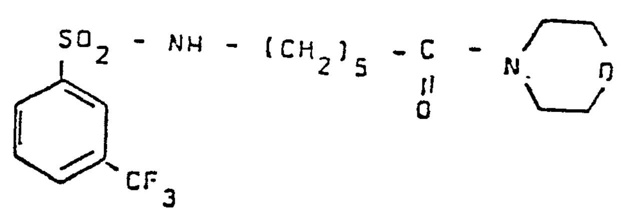

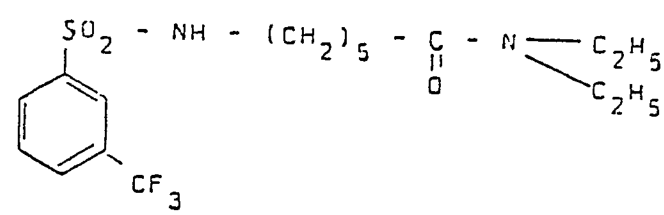

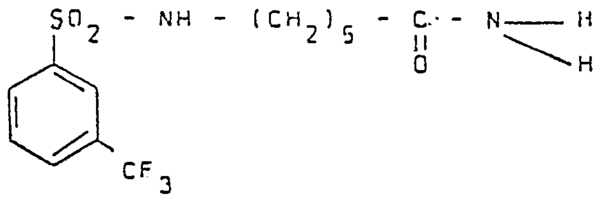

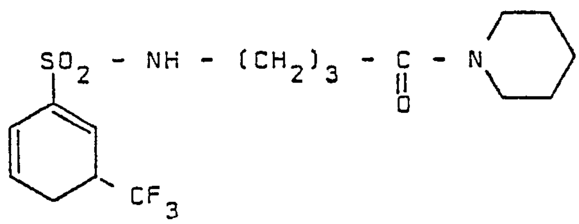

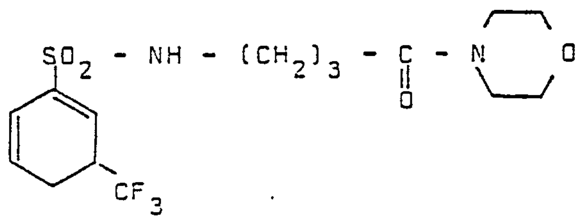

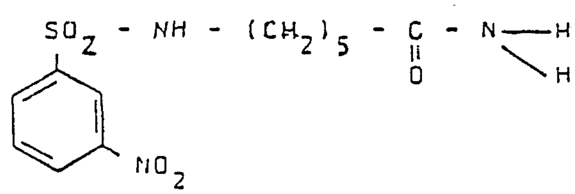

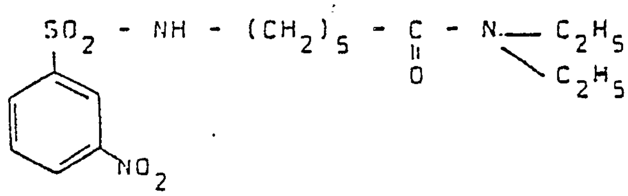

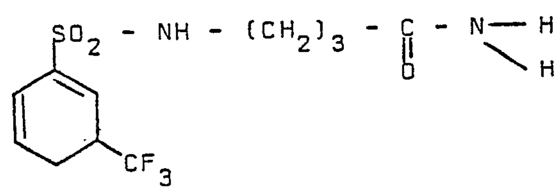

enthalten.

17. Verfahren zur Herstellung einer pharmazeutischen Zusammensetzung, dadurch gekennzeichnet, daß man irgendeine der nach dem Verfahren gemäß irgendeinem der Ansprüche 1 bis 16 erhaltenen Verbindungen mit einem pharmazeutisch annehmbaren Träger unter Bedingungen, die die Herstellung der gennanten pharmazeutischen Zusammensetzung gestatten, vereinigt.